# EUROPEAN PATENT SPECIFICATION

(11) **EP 3 183 578 B1**
(45) Date of publication and mention of the grant of the patent: **03.06.2020**
(21) Application number: 15757396.5
(22) Date of filing: 21.08.2015
(51) Int. Cl.: A61N 5/10, G01N 33/574, C12Q 1/68

(54) **METHODS FOR THE EARLY DETECTION OF COLORECTAL CANCER**
VERFAHREN ZUR FRÜHERKENNUNG VON KOLOREKTALKARZINOM
MÉTHODES DE DÉTECTION PRÉCOCE DU CANCER COLORECTAL

(30) Priority: 22.08.2014 US 201462040874 P
(43) Date of publication of application: 28.06.2017
(73) Proprietor: Abbott Laboratories, Abbott Park, IL 60064 (US); Hvidovre Hospital, 2650 Hvidovre (DK); University of Copenhagen, 1165 Copenhagen (DK)
(72) Inventor: DAVIS, Gerard J., Wauconda, Illinois 60084 (US); GAWEL, Susan H., LaGrange Park, Illinois 60526 (US); YANG, Xiaoqing, Buffalo Grove, Illinois 60089 (US); NIELSEN, Hans Jørgen, DK-2800 Lyngby (DK); CHRISTENSEN, Ib Jarle, DK-3400 Hillerød (DK)
(74) Representative: Modiano, Micaela Nadia
(86) International application number: PCT/US2015/046301
(87) International publication number: WO 2016/029117

(56) References cited:
- EP-A1- 2 071 337
- EP-A2- 1 439 393
- WO-A1-2006/015616
- WO-A1-2006/066915
- WO-A1-2007/071367
- WO-A1-2010/054789
- WO-A1-2010/127782
- WO-A1-2012/115885
- WO-A2-2009/074276
- US-A1- 2006 154 245
- US-A1- 2012 021 929
- CLEMENS GIESSEN ET AL: "Evaluation of preoperative serum markers for individual patient prognosis in stage I-III rectal cancer", TUMOR BIOLOGY, vol. 35, no. 10, 17 July 2014 (2014-07-17) , pages 10237-10248, XP055225960, CH ISSN: 1010-4283, DOI: 10.1007/s13277-014-2338-6
- HELEN SWEDE ET AL: "Baseline serum C-reactive protein and death from colorectal cancer in the NHANES III cohort", INTERNATIONAL JOURNAL OF CANCER, vol. 134, no. 8, 15 April 2014 (2014-04-15), pages 1862-1870, XP055226470, US ISSN: 0020-7136, DOI: 10.1002/ijc.28504
- KONSTANTINOS K. TSILIDIS ET AL: "C-reactive protein and colorectal cancer risk: A systematic review of prospective studies", INTERNATIONAL JOURNAL OF CANCER, vol. 123, no. 5, 1 September 2008 (2008-09-01), pages 1133-1140, XP055226465, US ISSN: 0020-7136, DOI: 10.1002/ijc.23606

## Description

### CROSS-REFERENCE TO RELATED APPLICATIONS

This application claims the benefit of U.S. Provisional Patent Application No. 62/040,874, filed August 22, 2014.

### FIELD ON THE INVENTION

The present invention relates to methods and immunoassay platforms for determining or aiding in determining a prognosis, diagnosis, or risk identification of high risk adenomas and colorectal cancer in a patient by detecting carcinoembryonic antigen (CEA), cytokeratin 19 fragment (CYFRA), c-reactive protein (CRP) and ferritin biomarkers in the patient. The carcinoembryonic antigen (CEA), cytokeratin 19 fragment (CYFRA), c-reactive protein (CRP) and ferritin biomarkers can be used to identify, diagnose, or aid in identifying or diagnosing a patient with colorectal cancer (CRC), to identify or aid in identifying whether a patient is suffering from CRC or high risk adenomas.

### BACKGROUND

Colorectal cancer (CRC) is the third most frequent cancer disease in Denmark counting 4.200 new cases annually. Despite that 80% of the patients will undergo intended curative resection, half of these patients will be diagnosed recurrent or metastatic disease within the next 5 years. The World Health Organization reports over 1 million cases of CRC worldwide. Developed countries account for about two-thirds of the cases. 55-60% of CRCs are diagnosed at an advanced stage, which results in high mortality and increased health care costs. CRC is very treatable if diagnosed early. Compared to 5-years survival rates at the individual stages where curative operation is possible - stage I: 90%, stage II: 70%, stage III: 35 - 40%-these facts indicate, that even in spite of absence of clinical and/or laboratory findings at the time of operation, a large proportion of patients must have occult disseminated disease at the time of surgical intervention. The prognosis for patients with verified disseminated disease - stage IV - is discouraging and longtime survival is rarely achieved. Approximately 50% of the patients diagnosed with CRC will not survive the next 5 years. Therefore, it is urgent to improve the overall treatment strategy for patients with CRC.

The stage-dependent distribution of patients with CRC is: stage I: 10%, stage II: 38%, stage III: 33% and stage IV: 19%. The survival rate would be significantly improved if more patients could be identified at the early stages of the disease (stage I or II). Within these patient groups surgery as the only intervention would cure a far greater proportion of the total patient population. Until now it has been possible to increase the quota of patients with early stages of the disease and hereby the total survival rate just by using "fecal-occult-blood" testing (FOBT) with subsequent endoscopy. FOBT has a high specificity (85%), but a less favourable sensitivity (40-65%). The compliance rates, though, are quite limited. Recently, two Danish feasibility screening studies with a mailed invitation to submit a FOBT have shown compliance less than 50%. This means that the clinical sensitivity is restricted to 20-33% as sensitivity of the test must be multiplied by compliance.

Biomarker analysis is potentially one element of such a diagnostic strategy, but the discovery and validation of tumor specific markers has been difficult. Accordingly, there remains a great need for more accurate and precise markers for the detection and screening of colorectal cancer in patients.
WO 2006/066915 discloses the use of the CYFRA 21-1 assay in the assessment of colorectal cancer. It also relates to a method for assessing colorectal cancer in vitro using a liquid sample, derived from an individual by measuring CYFRA 21-1 in said sample. CLEMENS GIESSEN ET AL, "Evaluation of preoperative serum markers for individual patient prognosis in stage I-III rectal cancer", TUMOR BIOLOGY, CH, (20140717), vol. 35, no. 10, pages 10237 - 10248. disclose CEA as prognostic factor for cancer-specific survival and disease-free in patients with rectal cancer who underwent surgery for curative intent survival.
WO 2007/071367 discloses the use of a marker combination comprising osteopontin and carcinoembryonic antigen in the assessment of colorectal cancer. US 2012/021929 discloses a method aiding in the assessment of the presence of cancer using the soluble DPPIV/seprase protein complex (DPPIV/seprase) as a universal marker of different cancer types.
WO 2009/074276 discloses a method for assessing colorectal cancer (CRC) in vitro comprising measuring in a sample the concentration and/or activity of a seprase polypeptide and/or fragments thereof and of either anti-p53 and/or osteopontin and/or Ferritin, of optionally one or more other marker of CRC, and using the combined measurement result in the assessment of CRC.
EP 2071337 (A1) discloses method aiding in the assessment of colon cancer or colorectal cancer using the human fibroblast activation protein (FAP/seprase) as a marker and the use of measurement of seprase in the early detection of cancer or in the surveillance of patients who undergo surgery.
WO 2010/127782 discloses a method aiding in the assessment of cancer using "soluble DPPIV/Seprase protein complex" (= DPPIV/Seprase) as a universal marker of different cancer types, and the use of measurement of DPPIV/Seprase in the early detection or diagnosis of cancer or in the surveillance of patients who undergo surgery.
WO 2010/054789 discloses the use of the proapoptotic caspase adaptor protein (= PACAP) as a universal marker of colon cancer and the use of measurement of PACAP in the early detection of cancer or in the surveillance of patients who undergo surgery.
US 2006/154245 discloses a method for screening and/or detecting and/or monitoring a cancer in an individual, the method comprising determining a first parameter represented by the concentration of TIMP-1 in at least one excreta, e.g. saliva, from the individual.
EP1439393 discloses a method for detecting the presence of colorectal cancer in an individual, wherein colorectal cancer is detected by detecting the presence of Reg1 alpha or TIMP1 nucleic acid or amino acid molecules in a clinical sample obtained from the patient, wherein Reg1 alpha or TIMP1 expression is indicative of the presence of colorectal cancer.

### SUMMARY OF INVENTION

The present invention is directed to a method of determining whether a subject is suffering from or at risk of suffering from high risk adenomas or colorectal cancer (CRC) and use of a kit for performing the method, as defined in the claims. The present invention provides a method of determining whether a subject is suffering from, or at risk of suffering from, high risk adenomas or colorectal cancer (CRC), the method comprising the steps of:
(a)providing a biological sample previously obtained from a subject;
(b) determining the levels of carcinoembryonic antigen (CEA), Cytokeratin 19 Fragment (CYFRA). C-reactive protein (CRP), and ferritin in the biological sample from the subject;
(c) comparing the levels of CEA, CYFRA, CRP, and ferritin in the biological sample to reference levels of CEA, CYFRA, CRP, and ferritin;
   (i) providing a CEA score for the subject wherein the subject gets a score of 1 if the level of CEA in the biological sample is greater than the reference level of CEA and a score of 0 if the level of CEA in the biological sample is equal or less than the reference level of CEA;
   (ii) providing a CYFRA score for the subject wherein the subject gets a score of 1 if the level of CYFRA in the biological sample is greater than the reference level of CYFRA and a score of 0 if the level of CYFRA in the biological sample is equal or less than the reference level of CYFRA;
   (iii) providing a CRP score for the subject wherein the subject gets a score of 1 if the level of CRP in the biological sample is greater than the reference level of CRP and a score of 0 if the level of CRP in the biological sample is equal or less than the reference level of CRP; and
   (iv) providing a ferritin score for the subject wherein the subject gets a score of 1 if the level of ferritin in the biological sample is less than the reference level of ferritin and a score of 0 if the level of ferritin in the biological sample is equal or greater than the reference level of ferritin;
(d) adding the scores from step (c) to generate a total score; and
(e) providing a diagnosis of a subject as suffering from, or at risk of suffering from, high risk adenomas or CRC if the total score is greater than a reference score, or a diagnosis of a subject as not suffering from, or not at risk of suffering from, high risk adenomas or CRC if the total score is equal to or less than the reference score.

The present invention also provides the use of a kit for performing the method of the invention, the kit comprising:
(a) a reagent capable of specifically binding to CEA. a reagent capable of specifically binding to CYFRA, a reagent capable of specifically binding to CRP, and a reagent capable ofspecifically binding to ferritin, to quantify the levels of CEA, CYFRA, CRP, and ferritin in the biological sample of a subject;
(b) a reference standard indicating reference levels of CEA, CYFRA, CRP. and ferritin; and optionally
(c) at least one additional reagent capable of specifically binding at least one additional biomarker selected from Methyl Septin 9 (mS9), Galectin-3 (Gal3). Glycated hemoglobin (HbA1c), complement component 3 (C3a), Cathepsin X (Cat X), soluble urokinase receptor (suPAR(I)), Plasminogen activator inhibitor-1 (PAI-1), Enolase 2 (ENO2), or combinations thereof, and
(d) a reference standard indicating a reference level of the at least one additional biomarker of Methyl Septin 9 (mS9), Galectin-3 (Gal3), Glycated hemoglobin (HbA1c), complement component 3 (C3a), Cathepsin X (Cat X), soluble urokinase receptor (suPAR(I)), Plasminogen activator inhibitor-1 (PAI-1), Enolase 2 (ENO2), or combinations thereof.

The method further comprises: determining the age of the subject in step (b); comparing the age of the subject to a reference age in step (c); and (v) providing an age score for the subject wherein the subject gets a score of 1 if the age of the subject is greater than or equal to the reference age and a score of 0 if the age of the subject is less than the reference age; wherein the subject is male or female; wherein: (a) in a male subject at least 54 years old, the reference level of CEA is at least 6.5 ng/mL, the reference level of CYFRA is at least 1.98 ng/mL, the reference level of CRP is at least 1.8 mg/mL, and the reference level of ferritin is less than 109 ng/mL; and
(b) in a female subject at least 63 years old, the reference level of CEA is at least 4.8 ng/ mL, the reference level of CYFRA is at least 1.72 ng/mL, the reference level of CRP is at least 5.5 mg/mL, and the reference level of ferritin is less than 38 ng/mL. The method, wherein the reference age for a male subject is 54, and the reference age for a female subject is 63. The method wherein the reference score is 0, 1,2, 3, 4, or 5. The method further comprising determining the levels of CEA,CYFRA, CRP, and ferritin comprises an immunological method with at least one antibody capable of specifically binding to CEA, CYFRA, CRP, ferritin, or combinations thereof. The method, wherein the immunological method comprises: (a) measuring the levels of CEA by: (i) contacting the test sample with at least one capture antibody, wherein the capture antibody binds to an epitope on CEA or a fragment of CEA to form a capture antibody-CEA antigen complex; (ii) contacting the capture antibody-CEA antigen complex with at least one detection antibody comprising a delectable label, wherein the detection antibody binds to an epitope on CEA that is not bound by the capture antibody and forms a capture antibody-CEA antigen-detection antibody complex; and (iii) determining the CEA levels in the test sample based on the signal generated by the detectable label in the capture antibody-CEA antigen-detection antibody complex formed in (a)(ii); (b) measuring the levels of CYFRA by: (i) contacting the lest sample with at least one capture antibody, wherein the capture antibody binds to an epitope on CYI'RA or a fragment of CYI'RA to form a capture antibody-CYIRA antigen complex; (ii) contacting the capture antibody-CYFRA antigen complex with at least one detection antibody comprising a detectable label, wherein the detection antibody binds to un epitope on CYI'RA that is not hound by the capture antibody and forms a capture antibody-CYFRA antigen-detection antibody complex; and (iii) determining the CYI'RA levels in the test sample based on the signal generated by the detectable label in the capture antibody-CYFRA antigen-detection antibody complex formed in (b)(ii); (c) measuring the levels of CRP by: (i) contacting the lest sample with at least one capture antibody, wherein the capture antibody binds to an epitope on CRP or a fragment of CRP to form a capture antibody-CRP antigen complex; (ii) contacting the capture antibody-CRP antigen complex with at least one detection antibody comprising a detectable label, wherein the detection antibody binds to an epitope on CRP that is not bound by the capture antibody and forms a capture antibody-CRP antigen-detection antibody complex; and (iii) determining the CRP levels in the test sample based on the signal generated by the detectable label in the capture antibody-CRP antigen-detection antibody complex formed in (c)(ii); and (d) measuring the levels of ferritin by: (i) contacting the test sample with at least one capture antibody, wherein the capture antibody binds to an epitope on ferritin or a fragment of ferritin to form a capture antibody-ferritin antigen complex; (ii) contacting the capture antibody-ferritin antigen complex with at least one detection antibody comprising a detectable label, wherein the detection antibody binds to an epitope on ferritin that is not bound by the capture antibody and forms a capture antibody-ferritin antigen-detection antibody complex; and (iii) determining the ferritin levels in the test sample based on the signal generated by the detectable label in the capture antibody-ferritin antigen-detection antibody complex formed in (d)(ii). The method further comprising determining the level of at least one additional biomarker of C'RC' in the biological sample selected from: Methyl Septin 9 (mS9), Galectin-3 (Gal3). Glycated hemoglobin (HbA1c), complement component 3(C3a), Cathepsin X (Cal X), soluble urokinase receptor (suPAR(I)), Plasminogen activator inhibitor-1 (PA1-I), Enolase 2 (LN02), or combinations thereof, and comparing the level of the at least one additional biomarker of CRC to a reference level for the at least one additional biomarker of CRC. The method of any one of the preceding claims, wherein the biological sample is a tissue sample, whole blood, plasma, serum, urine, bronchoalveolar lavage fluid, or a cell culture suspension or fraction thereof. The method, wherein the antibody is selected from a polyclonal antibody, a monoclonal antibody, a human antibody, a disulfide linked FV, an affinity matured antibody, a scFv, a chimeric antibody, a single domain antibody, a CDR-grafted antibody, antibody, a humanized antibody, a multispecific antibody, a lab. a dual-variable-domain (DVD) immunoglobulin, a Fab', a bispecific antibody, a F(ab*)2, and a Fv.

Disclosed herein is also a method of determining whether a subject is suffering or at risk of suffering from colorectal cancer (CRC). The method comprises the steps of: (a) obtaining a biological sample from a subject; (b) determining the levels of carcinoembryonic antigen (CEA), Cytokeratin 19 Fragment (CYFRA), C-reactive protein (CRP), carbohydrate antigen 19-9 (CA19-9), and ferritin in the biological sample from the subject; (c) comparing the levels of CEA, CYFRA, CRP, CA19-9, and ferritin, in the biological sample to reference levels of CEA, CYFRA, CRP, CA19-9, and ferritin; and (d) providing a diagnosis of a subject suffering from or at risk of suffering from CRC if the levels of CEA, CYFRA, CRP, and CA19-9 in the biological sample are greater than the reference levels of CEA, CYFRA, CRP, and CA19-9 and the levels of ferritin in the biological sample is less than the reference level of ferritin, or a diagnosis of a subject as not suffering from or not at risk of suffering from if the levels of CEA, CYFRA, and CA19-9 in the biological sample are equal to or less than the reference levels of CEA, CYFRA, and CA19-9 and the level of ferritin in the biological sample is equal to or greater than the reference level of ferritin. The reference levels of CEA, CYFRA, CRP, CA19-9, and ferritin may be the CEA, CYFRA, CRP, CA19-9, and ferritin cutoff values determined by adaptive index model methodology from biological samples of a reference group. The reference group may be selected from the group consisting of a control group and a cancer group. The subject may be male. The CEA, CYFRA, CRP, CA19-9, and ferritin reference level may be higher than or equal to 4.5 ng/mL, 4.6 ng/mL, 4.7 ng/mL, 4.8 ng/mL, 4.9 ng/mL, 5.0 ng/mL, 5.1 ng/mL, 5.2 ng/mL, 5.3 ng/mL, 5.4 ng/mL, 5.5 ng/mL, 5.6 ng/mL, 5.7 ng/mL, 5.8 ng/mL, 5.9 ng/mL, 6.0 ng/mL, 6.1 ng/mL, 6.2 ng/mL, 6.3 ng/mL, 6.4 ng/mL, 6.5 ng/mL, 6.6 ng/mL, 6.7 ng/mL, 6.8 ng/mL, 6.9 ng/mL, 7.0 ng/mL, 7.1 ng/mL, 7.2 ng/mL, 7.3 ng/mL, 7.4 ng/mL, 7.5 ng/mL, 7.6 ng/mL, 7.7 ng/mL, 7.8 ng/mL, 7.9 ng/mL, or 8.0 ng/mL in serum for CEA in combination with levels higher than or equal to 1.50 ng/mL, 1.60 ng/mL, 1.70 ng/mL, 1.80 ng/mL, 1.90 ng/mL, 2.00 ng/mL, 2.01 ng/mL, 2.02 ng/mL, 2.03 ng/mL, 2.04 ng/mL, 2.05 ng/mL, 2.06 ng/mL, 2.07 ng/mL, 2.08 ng/mL, 2.09 ng/mL, 2.10 ng/mL, 2.11 ng/mL, 2.12 ng/mL, 2.13 ng/mL, 2.14 ng/mL, 2.15 ng/mL, 2.16 ng/mL, 2.17 ng/mL, 2.18 ng/mL, 2.19 ng/mL, 2.20 ng/mL, 2.21 ng/mL, 2.22 ng/mL, 2.23 ng/mL, 2.24 ng/mL, 2.25 ng/mL, 2.50 ng/mL, or 3.00 ng/mL in serum for CYFRA, levels higher than or equal to 17.0 mg/mL, 17.1 mg/mL, 17.2 mg/mL, 17.3 mg/mL, 17.4 mg/mL, 17.5 mg/mL, 17.6 mg/mL, 17.7 mg/mL, 17.8 mg/mL, 17.9 mg/mL, 18.0 mg/mL, 18.1 mg/mL, 18.2 mg/mL, 18.3 mg/mL, 18.4 mg/mL, 18.5 mg/mL, 18.6 mg/mL, 18.7 mg/mL, 18.8 mg/mL, 18.9 mg/mL, 19.0 mg/mL, 19.5 mg/mL, or 20.0 mg/mL in serum for CRP, higher than or equal to 23.0 U/mL, 23.1 U/mL, 23.2 U/mL, 23.3 U/mL, 23.4 U/mL, 23.5 U/mL, 23.6 U/mL, 23.7 U/mL, 23.8 U/mL, 23.9 U/mL, 24.0 U/mL, 24.1 U/mL, 24.2 U/mL, 24.3 U/mL, 24.4 U/mL, 24.5 U/mL, 24.6 U/mL, 24.7 U/mL, 24.8 U/mL, 24.9 U/mL, 25.0 U/mL, 25.1 U/mL, 25.2 U/mL, 25.3 U/mL, 25.4 U/mL, 25.5 U/mL, 25.6 U/mL, 25.7 U/mL, 25.8 U/mL, 25.9 U/mL, 26.0 U/mL, 26.1 U/mL, 26.2 U/mL, 26.3 U/mL, 26.4 U/mL, 26.5 U/mL, 26.6 U/mL, 26.7 U/mL, 26.8 U/mL, 26.9 U/mL, 27.0 U/mL, 27.1 U/mL, 27.2 U/mL, 27.3 U/mL, 27.4 U/mL, 27.5 U/mL, 27.6 U/mL, 27.7 U/mL, 27.8 U/mL, 27.9 U/mL, 28.0 U/mL, 28.5 U/mL, or 29.0 U/mL in serum for CA19-9, and lower than or equal to 105.0 ng/mL, 104.0 ng/mL, 103.0 ng/mL, 102.0 ng/mL, 101.0 ng/mL, 100.0 ng/mL, 99.0 ng/mL, 98.0 ng/mL, 97.0 ng/mL, 96.0 ng/mL, 95.0 ng/mL, 94.0 ng/mL, 93.0 ng/mL, 92.0 ng/mL, 91.0 ng/mL, 90.0 ng/mL, 89.0 ng/mL, 88.0 ng/mL, 87.0 ng/mL, 86.0 ng/mL, or 85.0 ng/mL in serum for ferritin. The reference level of CEA may be at least about 6.9 ng/mL, the reference level of CYFRA may be at least about 2.17 ng/mL, the reference level of CRP may be at least about 18.3 mg/mL, the reference level of CA19-9 may be at least about 26.8 U/mL, and the reference level of ferritin may be at least about 97.0 ng/mL. The subject may be female. The CEA, CYFRA, CRP, CA19-9, and ferritin reference level may be higher than or equal to 4.5 ng/mL, 4.6 ng/mL, 4.7 ng/mL, 4.8 ng/mL, 4.9 ng/mL, 5.0 ng/mL, 5.1 ng/mL, 5.2 ng/mL, 5.3 ng/mL, 5.4 ng/mL, 5.5 ng/mL, 5.6 ng/mL, 5.7 ng/mL, 5.8 ng/mL, 5.9 ng/mL, 6.0 ng/mL, 6.1 ng/mL, 6.2 ng/mL, 6.3 ng/mL, 6.4 ng/mL, 6.5 ng/mL, 6.6 ng/mL, 6.7 ng/mL, 6.8 ng/mL, 6.9 ng/mL, 7.0 ng/mL, 7.1 ng/mL, 7.2 ng/mL, 7.3 ng/mL, 7.4 ng/mL, 7.5 ng/mL, 7.6 ng/mL, 7.7 ng/mL, 7.8 ng/mL, 7.9 ng/mL, or 8.0 ng/mL in serum for CEA in combination with levels higher than or equal to 1.50 ng/mL, 1.60 ng/mL, 1.70 ng/mL, 1.80 ng/mL, 1.90 ng/mL, 2.00 ng/mL, 2.01 ng/mL, 2.02 ng/mL, 2.03 ng/mL, 2.04 ng/mL, 2.05 ng/mL, 2.06 ng/mL, 2.07 ng/mL, 2.08 ng/mL, 2.09 ng/mL, 2.10 ng/mL, 2.11 ng/mL, 2.12 ng/mL, 2.13 ng/mL, 2.14 ng/mL, 2.15 ng/mL, 2.16 ng/mL, 2.17 ng/mL, 2.18 ng/mL, 2.19 ng/mL, 2.20 ng/mL, 2.21 ng/mL, 2.22 ng/mL, 2.23 ng/mL, 2.24 ng/mL, 2.25 ng/mL, 2.50 ng/mL, or 3.00 ng/mL in serum for CYFRA, levels higher than or equal to 6.5 mg/mL, 6.6 mg/mL, 6.7 mg/mL, 6.8 mg/mL, 6.9 mg/mL, 7.0 mg/mL, 7.1 mg/mL, 7.2 mg/mL, 7.3 mg/mL, 7.4 mg/mL, 7.5 mg/mL, 7.6 mg/mL, 7.7 mg/mL, 7.8 mg/mL, 7.9 mg/mL, 8.0 mg/mL, 8.1 mg/mL, 8.2 mg/mL, 8.3 mg/mL, 8.4 mg/mL, 8.5 mg/mL, 8.6 mg/mL, 8.7 mg/mL, 8.8 mg/mL, 8.9 mg/mL, 9.0 mg/mL, 9.5 mg/mL, or 10.0 mg/mL in serum for CRP, higher than or equal to 23.0 U/mL, 23.1 U/mL, 23.2 U/mL, 23.3 U/mL, 23.4 U/mL, 23.5 U/mL, 23.6 U/mL, 23.7 U/mL, 23.8 U/mL, 23.9 U/mL, 24.0 U/mL, 24.1 U/mL, 24.2 U/mL, 24.3 U/mL, 24.4 U/mL, 24.5 U/mL, 24.6 U/mL, 24.7 U/mL, 24.8 U/mL, 24.9 U/mL, 25.0 U/mL, 25.1 U/mL, 25.2 U/mL, 25.3 U/mL, 25.4 U/mL, 25.5 U/mL, 25.6 U/mL, 25.7 U/mL, 25.8 U/mL, 25.9 U/mL, 26.0 U/mL, 26.1 U/mL, 26.2 U/mL, 26.3 U/mL, 26.4 U/mL, 26.5 U/mL, 26.6 U/mL, 26.7 U/mL, 26.8 U/mL, 26.9 U/mL, 27.0 U/mL, 27.1 U/mL, 27.2 U/mL, 27.3 U/mL, 27.4 U/mL, 27.5 U/mL, 27.6 U/mL, 27.7 U/mL, 27.8 U/mL, 27.9 U/mL, 28.0 U/mL, 28.5 U/mL, or 29.0 U/mL in serum for CA19-9, and lower than or equal to 45 ng/mL, 44 ng/mL, 43 ng/mL, 42 ng/mL, 41 ng/mL, 40 ng/mL, 39 ng/mL, 38 ng/mL, 36 ng/mL, 35 ng/mL, 34 ng/mL, 33 ng/mL, 32 ng/mL, 31 ng/mL, 30 ng/mL, or 25 ng/mL in serum for ferritin. The reference level of CEA may be at least about 5.9 ng/mL, the reference level of CYFRA may be at least about 2.01 ng/mL, the reference level of CRP may be at least about 7.8 mg/mL, the reference level of CA19-9 may be at least about 24.0 U/mL, and the reference level of ferritin may be at least about 36 ng/mL. The method may further comprise administering a CRC structural screening regimen, a CRC treatment regimen, or a CRC monitoring regimen to the subject diagnosed as suffering from or at risk of suffering from CRC. The CRC treatment regimen may comprise administering at least one of surgery, radiotherapeutic therapy, radiotherapeutic treatments, chemotherapy, targeted therapy, or combinations thereof, to the subject. The CRC structural screening regimen may be a colonoscopy or sigmoidoscopy. The colonoscopy may confirm the diagnosis of a subject. The CRC monitoring regimen may comprise determining CEA, CYFRA, CRP, CA19-9, and ferritin levels at periodic intervals. Determining the levels of CEA, CYFRA, CRP, CA19-9, and ferritin may comprise an immunological method with molecules specifically binding to CEA, CYFRA, CRP, CA19-9, and ferritin. The molecules specifically binding to CEA, CYFRA, CRP, CA19-9, and ferritin may comprise at least one antibody capable of specifically binding CEA, CYFRA, CRP, CA19-9, and ferritin. Determining the level of CEA, CYFRA, CRP, CA19-9, and ferritin may involve the step of contacting the biological sample with at least one antibody selected from the group consisting of: an antibody that specifically binds to CEA, an antibody that specifically binds to CYFRA, an antibody that specifically binds to CRP, an antibody that specifically binds to CA19-9, an antibody that specifically binds to ferritin, and combinations thereof. Determining the level of CEA, CYFRA, CRP, CA19-9, and ferritin may involve the step of assaying the biological sample for CEA, CYFRA, CRP, CA19-9, and ferritin by an immunoassay that employs at least one capture antibody and at least one antibody labeled with a detectable label, which generates a signal, and comprises comparing a signal generated by the detectable label as a direct or indirect indication of the amount of CEA, CYFRA, CRP, CA19-9, and ferritin in the biological sample, wherein the capture antibody and the antibody labeled with a detectable label comprise: (a) at least one capture antibody that specifically binds to CEA and at least one antibody labeled with a detectable label; (b) at least one capture antibody that specifically binds to CYFRA and at least one antibody labeled with a detectable label; (c) at least one capture antibody that specifically binds to CRP and at least one antibody labeled with a detectable label; (d) at least one capture antibody that specifically binds to CA19-9 and at least one antibody labeled with a detectable label; and (e) at least one capture antibody that specifically binds to ferritin and at least one antibody labeled with a detectable label. The immunological method may comprise: (a) measuring the levels of CEA by: (i) contacting the test sample with at least one capture antibody, wherein the capture antibody binds to an epitope on CEA or a fragment of CEA to form a capture antibody-CEA antigen complex; (ii) contacting the capture antibody-CEA antigen complex with at least one detection antibody comprising a detectable label, wherein the detection antibody binds to an epitope on CEA that is not bound by the capture antibody and forms a capture antibody-CEA antigen-detection antibody complex; and (iii) determining the CEA levels in the test sample based on the signal generated by the detectable label in the capture antibody-CEA antigen-detection antibody complex formed in (a)(ii); (b) measuring the levels of CYFRA by: (i) contacting the test sample with at least one capture antibody, wherein the capture antibody binds to an epitope on CYFRA or a fragment of CYFRA to form a capture antibody-CYFRA antigen complex; (ii) contacting the capture antibody-CYFRA antigen complex with at least one detection antibody comprising a detectable label, wherein the detection antibody binds to an epitope on CYFRA that is not bound by the capture antibody and forms a capture antibody-CYFRA antigen-detection antibody complex; and (iii) determining the CYFRA levels in the test sample based on the signal generated by the detectable label in the capture antibody-CYFRA antigen-detection antibody complex formed in (b)(ii); (c) measuring the levels of CRP by: (i) contacting the test sample with at least one capture antibody, wherein the capture antibody binds to an epitope on CRP or a fragment of CRP to form a capture antibody-CRP antigen complex; (ii) contacting the capture antibody-CRP antigen complex with at least one detection antibody comprising a detectable label, wherein the detection antibody binds to an epitope on CRP that is not bound by the capture antibody and forms a capture antibody-CRP antigen-detection antibody complex; and (iii) determining the CRP levels in the test sample based on the signal generated by the detectable label in the capture antibody-CRP antigen-detection antibody complex formed in (c)(ii); (d) measuring the levels of CA19-9 by: (i) contacting the test sample with at least one capture antibody, wherein the capture antibody binds to an epitope on CA19-9 or a fragment of CA19-9 to form a capture antibody-CA19-9 antigen complex; (ii) contacting the capture antibody-CA19-9 antigen complex with at least one detection antibody comprising a detectable label, wherein the detection antibody binds to an epitope on CA19-9 that is not bound by the capture antibody and forms a capture antibody-CRP antigen-detection antibody complex; and (iii) determining the CA19-9 levels in the test sample based on the signal generated by the detectable label in the capture antibody-CA19-9 antigen-detection antibody complex formed in (d)(ii); and (e) measuring the levels of ferritin by: (i) contacting the test sample with at least one capture antibody, wherein the capture antibody binds to an epitope on ferritin or a fragment of ferritin to form a capture antibody-ferritin antigen complex; (ii) contacting the capture antibody-ferritin antigen complex with at least one detection antibody comprising a detectable label, wherein the detection antibody binds to an epitope on ferritin that is not bound by the capture antibody and forms a capture antibody-ferritin antigen-detection antibody complex; and (iii) determining the ferritin levels in the test sample based on the signal generated by the detectable label in the capture antibody-ferritin antigen-detection antibody complex formed in (e)(ii). The antibody may be selected from the group consisting of: a polyclonal antibody, a monoclonal antibody, a human antibody, an immunoglobulin molecule, a disulfide linked Fv, a monoclonal antibody, an affinity matured antibody, a scFv, a chimeric antibody, a single domain antibody, a CDR-grafted antibody, a diabody, a humanized antibody, a multispecific antibody, a Fab, a dual specific antibody, a DVD, a Fab', a bispecific antibody, a F(ab')2, and a Fv. The method may further comprise determining the level of at least one additional biomarker of CRC in the biological sample selected from the group consisting of: Methyl Septin 9 (mS9), Galectin-3 (Gal3), Glycated hemoglobin (HbA1c), C3a, Cathepsin X (Cat X), soluble urokinase receptor (suPAR(I)), Plasminogen activator inhibitor-1 (PAI-1), Enolase 2 (ENO2), and combinations thereof, and comparing the level of the at least one additional biomarker of CRC to a reference level for the at least one additional biomarker of CRC cancer. The subject may be a human. The biological sample of a subject may be selected from a tissue sample, bodily fluid, whole blood, plasma, serum, urine, bronchoalveolar lavage fluid, and a cell culture suspension or fraction thereof. The biological sample of a subject may be blood plasma or blood serum. Disclosed herein is also a kit for performing said method. The kit comprising: (a) a reagent capable of specifically binding to CEA, a reagent capable of specifically binding to CYFRA, a reagent capable of specifically binding to CRP, a reagent capable of specifically binding to CA19-9, and a reagent capable of specifically binding to ferritin to quantify the levels of CEA, CYFRA, CRP, CA19-9, and ferritin in the biological sample of a subject; and (b) a reference standard indicating reference levels of CEA, CYFRA, CRP, CA19-9, and ferritin. The kit may further comprise at least one additional reagent capable of specifically binding at least one additional biomarker of Methyl Septin 9 (mS9), Galectin-3 (Gal3), Glycated hemoglobin (HbA1c), C3a, Cathepsin X (Cat X), soluble urokinase receptor (suPAR(I)), Plasminogen activator inhibitor-1 (PAI-1), Enolase 2 (ENO2), or combinations thereof, in the biological sample to quantify the concentration of the at least one additional biomarker in the biological sample, and a reference standard indicating a reference level of the at least one additional biomarker of Methyl Septin 9 (mS9), Galectin-3 (Gal3), Glycated hemoglobin (HbA1c), C3a, Cathepsin X (Cat X), soluble urokinase receptor (suPAR(I)), Plasminogen activator inhibitor-1 (PAI-1), Enolase 2 (ENO2), or combinations thereof, in the biological sample.

Disclosed herein is also a method of determining whether a subject is suffering from or at risk of suffering from colorectal cancer (CRC). The method comprises the steps of: (a) obtaining a biological sample from a subject; (b) determining the levels of carcinoembryonic antigen (CEA), Cytokeratin 19 Fragment (CYFRA), C-reactive protein (CRP), carbohydrate antigen 19-9 (CA19-9), and ferritin in the biological sample from the subject; (c) comparing the levels of CEA, CYFRA, CRP, CA19-9, and ferritin in the biological sample to reference levels of CEA, CYFRA, CRP, CA19-9, and ferritin; (d) providing a CEA score for the subject wherein the subject gets a score of 1 if the level of CEA in the biological sample is greater than the reference level of CEA and a score of 0 if the level of CEA in the biological sample is equal or less than the reference level of CEA; (e) providing a CYFRA score for the subject wherein the subject gets a score of 1 if the level of CYFRA in the biological sample is greater than the reference level of CYFRA and a score of 0 if the level of CYFRA in the biological sample is equal or less than the reference level of CYFRA; (f) providing a CRP score for the subject wherein the subject gets a score of 1 if the level of CRP in the biological sample is greater than the reference level of CRP and a score of 0 if the level of CRP in the biological sample is equal or less than the reference level of CRP; (g) providing a CA19-9 score for the subject wherein the subject gets a score of 1 if the level of CA19-9 in the biological sample is greater than the reference level of CA19-9 and a score of 0 if the level of CA19-9 in the biological sample is equal or less than the reference level of CA19-9; (h) providing a ferritin score for the subject wherein the subject gets a score of 1 if the level of ferritin in the biological sample is less than the reference level of ferritin and a score of 0 if the level of ferritin in the biological sample is equal or greater than the reference level of ferritin; (i) adding the CEA score, CYFRA score, CRP score, CA19-9 score, and ferritin score to generate a total score; and (j) providing a diagnosis of a subject as suffering from or at risk of suffering from CRC if the total score is greater than a reference score, or a diagnosis of a subject as not suffering from or not at risk of suffering from CRC if the total score is equal to or less than the reference score. The reference levels of CEA, CYFRA, CRP, CA19-9, and ferritin may be the CEA, CYFRA, CRP, CA19-9, and ferritin cutoff values determined by adaptive index model methodology from biological samples of a reference group. The reference group may be selected from the group consisting of a control group and a cancer group. The subject may be male. The CEA, CYFRA, CRP, CA19-9, and ferritin reference level may be higher than or equal to 4.5 ng/mL, 4.6 ng/mL, 4.7 ng/mL, 4.8 ng/mL, 4.9 ng/mL, 5.0 ng/mL, 5.1 ng/mL, 5.2 ng/mL, 5.3 ng/mL, 5.4 ng/mL, 5.5 ng/mL, 5.6 ng/mL, 5.7 ng/mL, 5.8 ng/mL, 5.9 ng/mL, 6.0 ng/mL, 6.1 ng/mL, 6.2 ng/mL, 6.3 ng/mL, 6.4 ng/mL, 6.5 ng/mL, 6.6 ng/mL, 6.7 ng/mL, 6.8 ng/mL, 6.9 ng/mL, 7.0 ng/mL, 7.1 ng/mL, 7.2 ng/mL, 7.3 ng/mL, 7.4 ng/mL, 7.5 ng/mL, 7.6 ng/mL, 7.7 ng/mL, 7.8 ng/mL, 7.9 ng/mL, or 8.0 ng/mL in serum for CEA in combination with levels higher than or equal to 1.50 ng/mL, 1.60 ng/mL, 1.70 ng/mL, 1.80 ng/mL, 1.90 ng/mL, 2.00 ng/mL, 2.01 ng/mL, 2.02 ng/mL, 2.03 ng/mL, 2.04 ng/mL, 2.05 ng/mL, 2.06 ng/mL, 2.07 ng/mL, 2.08 ng/mL, 2.09 ng/mL, 2.10 ng/mL, 2.11 ng/mL, 2.12 ng/mL, 2.13 ng/mL, 2.14 ng/mL, 2.15 ng/mL, 2.16 ng/mL, 2.17 ng/mL, 2.18 ng/mL, 2.19 ng/mL, 2.20 ng/mL, 2.21 ng/mL, 2.22 ng/mL, 2.23 ng/mL, 2.24 ng/mL, 2.25 ng/mL, 2.50 ng/mL, or 3.00 ng/mL in serum for CYFRA, levels higher than or equal to 17.0 mg/mL, 17.1 mg/mL, 17.2 mg/mL, 17.3 mg/mL, 17.4 mg/mL, 17.5 mg/mL, 17.6 mg/mL, 17.7 mg/mL, 17.8 mg/mL, 17.9 mg/mL, 18.0 mg/mL, 18.1 mg/mL, 18.2 mg/mL, 18.3 mg/mL, 18.4 mg/mL, 18.5 mg/mL, 18.6 mg/mL, 18.7 mg/mL, 18.8 mg/mL, 18.9 mg/mL, 19.0 mg/mL, 19.5 mg/mL, or 20.0 mg/mL in serum for CRP, higher than or equal to 23.0 U/mL, 23.1 U/mL, 23.2 U/mL, 23.3 U/mL, 23.4 U/mL, 23.5 U/mL, 23.6 U/mL, 23.7 U/mL, 23.8 U/mL, 23.9 U/mL, 24.0 U/mL, 24.1 U/mL, 24.2 U/mL, 24.3 U/mL, 24.4 U/mL, 24.5 U/mL, 24.6 U/mL, 24.7 U/mL, 24.8 U/mL, 24.9 U/mL, 25.0 U/mL, 25.1 U/mL, 25.2 U/mL, 25.3 U/mL, 25.4 U/mL, 25.5 U/mL, 25.6 U/mL, 25.7 U/mL, 25.8 U/mL, 25.9 U/mL, 26.0 U/mL, 26.1 U/mL, 26.2 U/mL, 26.3 U/mL, 26.4 U/mL, 26.5 U/mL, 26.6 U/mL, 26.7 U/mL, 26.8 U/mL, 26.9 U/mL, 27.0 U/mL, 27.1 U/mL, 27.2 U/mL, 27.3 U/mL, 27.4 U/mL, 27.5 U/mL, 27.6 U/mL, 27.7 U/mL, 27.8 U/mL, 27.9 U/mL, 28.0 U/mL, 28.5 U/mL, or 29.0 U/mL in serum for CA19-9, and lower than or equal to 105.0 ng/mL, 104.0 ng/mL, 103.0 ng/mL, 102.0 ng/mL, 101.0 ng/mL, 100.0 ng/mL, 99.0 ng/mL, 98.0 ng/mL, 97.0 ng/mL, 96.0 ng/mL, 95.0 ng/mL, 94.0 ng/mL, 93.0 ng/mL, 92.0 ng/mL, 91.0 ng/mL, 90.0 ng/mL, 89.0 ng/mL, 88.0 ng/mL, 87.0 ng/mL, 86.0 ng/mL, or 85.0 ng/mL in serum for ferritin. The reference level of CEA may be at least about 6.9 ng/mL, the reference level of CYFRA may be at least about 2.17 ng/mL, the reference level of CRP may be at least about 18.3 mg/mL, the reference level of CA19-9 may be at least about 26.8 U/mL, and the reference level of ferritin may be at least about 97.0 ng/mL. The subject may be female. The CEA, CYFRA, CRP, CA19-9, and ferritin reference level may be higher than or equal to 4.5 ng/mL, 4.6 ng/mL, 4.7 ng/mL, 4.8 ng/mL, 4.9 ng/mL, 5.0 ng/mL, 5.1 ng/mL, 5.2 ng/mL, 5.3 ng/mL, 5.4 ng/mL, 5.5 ng/mL, 5.6 ng/mL, 5.7 ng/mL, 5.8 ng/mL, 5.9 ng/mL, 6.0 ng/mL, 6.1 ng/mL, 6.2 ng/mL, 6.3 ng/mL, 6.4 ng/mL, 6.5 ng/mL, 6.6 ng/mL, 6.7 ng/mL, 6.8 ng/mL, 6.9 ng/mL, 7.0 ng/mL, 7.1 ng/mL, 7.2 ng/mL, 7.3 ng/mL, 7.4 ng/mL, 7.5 ng/mL, 7.6 ng/mL, 7.7 ng/mL, 7.8 ng/mL, 7.9 ng/mL, or 8.0 ng/mL in serum for CEA in combination with levels higher than or equal to 1.50 ng/mL, 1.60 ng/mL, 1.70 ng/mL, 1.80 ng/mL, 1.90 ng/mL, 2.00 ng/mL, 2.01 ng/mL, 2.02 ng/mL, 2.03 ng/mL, 2.04 ng/mL, 2.05 ng/mL, 2.06 ng/mL, 2.07 ng/mL, 2.08 ng/mL, 2.09 ng/mL, 2.10 ng/mL, 2.11 ng/mL, 2.12 ng/mL, 2.13 ng/mL, 2.14 ng/mL, 2.15 ng/mL, 2.16 ng/mL, 2.17 ng/mL, 2.18 ng/mL, 2.19 ng/mL, 2.20 ng/mL, 2.21 ng/mL, 2.22 ng/mL, 2.23 ng/mL, 2.24 ng/mL, 2.25 ng/mL, 2.50 ng/mL, or 3.00 ng/mL in serum for CYFRA, levels higher than or equal to 6.5 mg/mL, 6.6 mg/mL, 6.7 mg/mL, 6.8 mg/mL, 6.9 mg/mL, 7.0 mg/mL, 7.1 mg/mL, 7.2 mg/mL, 7.3 mg/mL, 7.4 mg/mL, 7.5 mg/mL, 7.6 mg/mL, 7.7 mg/mL, 7.8 mg/mL, 7.9 mg/mL, 8.0 mg/mL, 8.1 mg/mL, 8.2 mg/mL, 8.3 mg/mL, 8.4 mg/mL, 8.5 mg/mL, 8.6 mg/mL, 8.7 mg/mL, 8.8 mg/mL, 8.9 mg/mL, 9.0 mg/mL, 9.5 mg/mL, or 10.0 mg/mL in serum for CRP, higher than or equal to 23.0 U/mL, 23.1 U/mL, 23.2 U/mL, 23.3 U/mL, 23.4 U/mL, 23.5 U/mL, 23.6 U/mL, 23.7 U/mL, 23.8 U/mL, 23.9 U/mL, 24.0 U/mL, 24.1 U/mL, 24.2 U/mL, 24.3 U/mL, 24.4 U/mL, 24.5 U/mL, 24.6 U/mL, 24.7 U/mL, 24.8 U/mL, 24.9 U/mL, 25.0 U/mL, 25.1 U/mL, 25.2 U/mL, 25.3 U/mL, 25.4 U/mL, 25.5 U/mL, 25.6 U/mL, 25.7 U/mL, 25.8 U/mL, 25.9 U/mL, 26.0 U/mL, 26.1 U/mL, 26.2 U/mL, 26.3 U/mL, 26.4 U/mL, 26.5 U/mL, 26.6 U/mL, 26.7 U/mL, 26.8 U/mL, 26.9 U/mL, 27.0 U/mL, 27.1 U/mL, 27.2 U/mL, 27.3 U/mL, 27.4 U/mL, 27.5 U/mL, 27.6 U/mL, 27.7 U/mL, 27.8 U/mL, 27.9 U/mL, 28.0 U/mL, 28.5 U/mL, or 29.0 U/mL in serum for CA19-9, and lower than or equal to 45 ng/mL, 44 ng/mL, 43 ng/mL, 42 ng/mL, 41 ng/mL, 40 ng/mL, 39 ng/mL, 38 ng/mL, 36 ng/mL, 35 ng/mL, 34 ng/mL, 33 ng/mL, 32 ng/mL, 31 ng/mL, 30 ng/mL, or 25 ng/mL in serum for ferritin. The reference level of CEA may be at least about 5.9 ng/mL, the reference level of CYFRA may be at least about 2.01 ng/mL, the reference level of CRP may be at least about 7.8 mg/mL, the reference level of CA19-9 may be at least about 24.0 U/mL, and the reference level of ferritin may be at least about 36 ng/mL. The reference score may be 0, 1, 2, 3, 4, or 5. If the subject is male, the reference score may be 1. If the subject is male and the total score is greater than 1, the subject may be diagnosed as suffering from or at risk of suffering from CRC. If the subject is female, the reference score may be 1 or 2. If the subject is female and the total score is greater than 1, the subject may be diagnosed as suffering from or at risk of suffering from CRC. If the subject is female and the total score is greater than 2, the subject may be diagnosed as suffering from or at risk of suffering from CRC. The method may further comprise administering a CRC structural screening regimen, a CRC treatment regimen, or a CRC monitoring regimen to the subject diagnosed as suffering from or at risk of suffering from CRC. The CRC treatment regimen may comprise administering at least one of surgery, radiotherapeutic therapy, radiotherapeutic treatments, chemotherapy, targeted therapy, or combinations thereof, to the subject. The CRC structural screening regimen may be a colonoscopy or sigmoidoscopy. The colonoscopy may confirm the diagnosis of a subject. The CRC monitoring regimen may comprise determining CEA, CYFRA, CRP, CA19-9, and ferritin levels at periodic intervals. Determining the levels of CEA, CYFRA, CRP, CA19-9, and ferritin may comprise an immunological method with molecules specifically binding to CEA, CYFRA, CRP, CA19-9, and ferritin. The molecules specifically binding to CEA, CYFRA, CRP, CA19-9, and ferritin may comprise at least one antibody capable of specifically binding CEA, CYFRA, CRP, CA19-9, and ferritin. Determining the level of CEA, CYFRA, CRP, CA19-9, and ferritin may involve the step of contacting the biological sample with at least one antibody selected from the group consisting of: an antibody that specifically binds to CEA, an antibody that specifically binds to CYFRA, an antibody that specifically binds to CRP, an antibody that specifically binds to CA19-9, an antibody that specifically binds to ferritin, and combinations thereof. Determining the level of CEA, CYFRA, CRP, CA19-9, and ferritin may involve the step of assaying the biological sample for CEA, CYFRA, CRP, CA19-9, and ferritin by an immunoassay that employs at least one capture antibody and at least one antibody labeled with a detectable label, which generates a signal, and comprises comparing a signal generated by the detectable label as a direct or indirect indication of the amount of CEA, CYFRA, CRP, CA19-9, and ferritin in the biological sample, wherein the capture antibody and the antibody labeled with a detectable label comprise: (a) at least one capture antibody that specifically binds to CEA and at least one antibody labeled with a detectable label; (b) at least one capture antibody that specifically binds to CYFRA and at least one antibody labeled with a detectable label; (c) at least one capture antibody that specifically binds to CRP and at least one antibody labeled with a detectable label; (d) at least one capture antibody that specifically binds to CA19-9 and at least one antibody labeled with a detectable label; and (e) at least one capture antibody that specifically binds to ferritin and at least one antibody labeled with a detectable label. The immunological method may comprise: (a) measuring the levels of CEA by: (i) contacting the test sample with at least one capture antibody, wherein the capture antibody binds to an epitope on CEA or a fragment of CEA to form a capture antibody-CEA antigen complex; (ii) contacting the capture antibody-CEA antigen complex with at least one detection antibody comprising a detectable label, wherein the detection antibody binds to an epitope on CEA that is not bound by the capture antibody and forms a capture antibody-CEA antigen-detection antibody complex; and (iii) determining the CEA levels in the test sample based on the signal generated by the detectable label in the capture antibody-CEA antigen-detection antibody complex formed in (a)(ii); (b) measuring the levels of CYFRA by: (i) contacting the test sample with at least one capture antibody, wherein the capture antibody binds to an epitope on CYFRA or a fragment of CYFRA to form a capture antibody-CYFRA antigen complex; (ii) contacting the capture antibody-CYFRA antigen complex with at least one detection antibody comprising a detectable label, wherein the detection antibody binds to an epitope on CYFRA that is not bound by the capture antibody and forms a capture antibody-CYFRA antigen-detection antibody complex; and (iii) determining the CYFRA levels in the test sample based on the signal generated by the detectable label in the capture antibody-CYFRA antigen-detection antibody complex formed in (b)(ii); (c) measuring the levels of CRP by: (i) contacting the test sample with at least one capture antibody, wherein the capture antibody binds to an epitope on CRP or a fragment of CRP to form a capture antibody-CRP antigen complex; (ii) contacting the capture antibody-CRP antigen complex with at least one detection antibody comprising a detectable label, wherein the detection antibody binds to an epitope on CRP that is not bound by the capture antibody and forms a capture antibody-CRP antigen-detection antibody complex; and (iii) determining the CRP levels in the test sample based on the signal generated by the detectable label in the capture antibody-CRP antigen-detection antibody complex formed in (c)(ii); (d) measuring the levels of CA19-9 by: (i) contacting the test sample with at least one capture antibody, wherein the capture antibody binds to an epitope on CA19-9 or a fragment of CA19-9 to form a capture antibody-CA19-9 antigen complex; (ii) contacting the capture antibody-CA19-9 antigen complex with at least one detection antibody comprising a detectable label, wherein the detection antibody binds to an epitope on CA19-9 that is not bound by the capture antibody and forms a capture antibody-CRP antigen-detection antibody complex; and (iii) determining the CA19-9 levels in the test sample based on the signal generated by the detectable label in the capture antibody-CA19-9 antigen-detection antibody complex formed in (d)(ii); and (e) measuring the levels of ferritin by: (i) contacting the test sample with at least one capture antibody, wherein the capture antibody binds to an epitope on ferritin or a fragment of ferritin to form a capture antibody-ferritin antigen complex; (ii) contacting the capture antibody-ferritin antigen complex with at least one detection antibody comprising a detectable label, wherein the detection antibody binds to an epitope on ferritin that is not bound by the capture antibody and forms a capture antibody-ferritin antigen-detection antibody complex; and (iii) determining the ferritin levels in the test sample based on the signal generated by the detectable label in the capture antibody-ferritin antigen-detection antibody complex formed in (e)(ii). The antibody may be selected from the group consisting of: a polyclonal antibody, a monoclonal antibody, a human antibody, an immunoglobulin molecule, a disulfide linked Fv, a monoclonal antibody, an affinity matured antibody, a scFv, a chimeric antibody, a single domain antibody, a CDR-grafted antibody, a diabody, a humanized antibody, a multispecific antibody, a Fab, a dual specific antibody, a DVD, a Fab', a bispecific antibody, a F(ab')2, and a Fv. The method may further comprise determining the level of at least one additional biomarker of CRC in the biological sample selected from the group consisting of: Methyl Septin 9 (mS9), Galectin-3 (Gal3), Glycated hemoglobin (HbA1c), C3a, Cathepsin X (Cat X), soluble urokinase receptor (suPAR(I)), Plasminogen activator inhibitor-1 (PAI-1), Enolase 2 (ENO2), and combinations thereof, and comparing the level of the at least one additional biomarker of CRC to a reference level for the at least one additional biomarker of CRC cancer. The subject may be a human. The biological sample of a subject may be selected from a tissue sample, bodily fluid, whole blood, plasma, serum, urine, bronchoalveolar lavage fluid, and a cell culture suspension or fraction thereof. The biological sample of a subject may be blood plasma or blood serum. Disclosed herein is also a kit for performing said method. The kit comprising: (a) a reagent capable of specifically binding to CEA, a reagent capable of specifically binding to CYFRA, a reagent capable of specifically binding to CRP, a reagent capable of specifically binding to CA19-9, and a reagent capable of specifically binding to ferritin to quantify the levels of CEA, CYFRA, CRP, CA19-9, and ferritin in the biological sample of a subject; and (b) a reference standard indicating reference levels of CEA, CYFRA, CRP, CA19-9, and ferritin. The kit may further comprise at least one additional reagent capable of specifically binding at least one additional biomarker of Methyl Septin 9 (mS9), Galectin-3 (Gal3), Glycated hemoglobin (HbA1c), C3a, Cathepsin X (Cat X), soluble urokinase receptor (suPAR(I)), Plasminogen activator inhibitor-1 (PAI-1), Enolase 2 (ENO2), or combinations thereof, in the biological sample to quantify the concentration of the at least one additional biomarker in the biological sample, and a reference standard indicating a reference level of the at least one additional biomarker of
Methyl Septin 9 (mS9), Galectin-3 (Gal3), Glycated hemoglobin (HbA1c), C3a, Cathepsin X (Cat X), soluble urokinase receptor (suPAR(I)), Plasminogen activator inhibitor-1 (PAI-1), Enolase 2 (ENO2), or combinations thereof, in the biological sample.

Disclosed herein is also a method of determining whether a male subject is suffering from or at risk of suffering from a cancer other than colorectal cancer (CRC) cancer, wherein the male subject was confirmed not to have CRC by a colonoscopy, the method comprising the steps of: (a) obtaining a biological sample from the male subject; (b) determining the levels of Cytokeratin 19 Fragment (CYFRA), C-reactive protein (CRP), a tissue inhibitor of metalloproteinase 1 (TIMP-1), carbohydrate antigen 19-9 (CA19-9), and alpha-fetoprotein (AFP) in the biological sample from the male subject; (c) comparing the levels of CYFRA, CRP, TIMP-1, CA19-9, and AFP in the biological sample to reference levels of CYFRA, CRP, TIMP-1, CA19-9, and AFP; and (d) providing a diagnosis of the male subject as suffering from or at risk of suffering from a cancer other than CRC if the levels of CYFRA, CRP, TIMP-1, CA19-9, and AFP in the biological sample are greater than the reference levels of CYFRA, CRP, TTMP-1, CA19-9, and AFP. The reference levels of CYFRA, CRP, TIMP-1, CA19-9, and AFP may be the CYFRA, CRP, TIMP-1, CA19-9, and AFP cutoff values determined by adaptive index model methodology from biological samples of a reference group. The reference group may be selected from the group consisting of a control group and a cancer group. The CYFRA, CRP, TIMP-1, CA19-9, and AFP reference level may be higher than or equal to 1.0 ng/mL, 1.1 ng/mL, 1.2 ng/mL, 1.3 ng/mL, 1.4 ng/mL, 1.5 ng/mL, 1.6 ng/mL, 1.7 ng/mL, 1.8 ng/mL, 1.9 ng/mL, 2.0 ng/mL, 2.1 ng/mL, 2.2 ng/mL, 2.3 ng/mL, 2.4 ng/mL, 2.5 ng/mL, 3.0 ng/mL, or 3.5 ng/mL in serum for CYFRA in combination with levels higher than or equal to 14.0 mg/mL, 14.1 mg/mL, 14.2 mg/mL, 14.3 mg/mL, 14.4 mg/mL, 14.5 mg/mL, 14.6 mg/mL, 14.7 mg/mL, 14.8 mg/mL, 14.9 mg/mL, 15.0 mg/mL, 15.1 mg/mL, 15.2 mg/mL, 15.3 mg/mL, 15.4 mg/mL, 15.5 mg/mL, 15.6 mg/mL, 15.7 mg/mL, 15.8 mg/mL, 15.9 mg/mL, 16.0 mg/mL, 16.1 mg/mL, 16.2 mg/mL, 16.3 mg/mL, 16.4 mg/mL, 16.5 mg/mL, 17.0 mg/mL, or 17.5 mg/mL in serum for CRP, levels higher than or equal to 140 ng/mL, 141 ng/mL, 142 ng/mL, 143 ng/mL, 144 ng/mL, 145 ng/mL, 146 ng/mL, 147 ng/mL, 148 ng/mL, 149 ng/mL, 150 ng/mL, 151 ng/mL, 152 ng/mL, 153 ng/mL, 154 ng/mL, 155 ng/mL, 156 ng/mL, 157 ng/mL, 158 ng/mL, 159 ng/mL, or 160 ng/mL in serum for TIMP-1, higher than or equal to 24.0 U/mL, 24.1 U/mL, 24.2 U/mL, 24.3 U/mL, 24.4 U/mL, 24.5 U/mL, 24.6 U/mL, 24.7 U/mL, 24.8 U/mL, 24.9 U/mL, 25.0 U/mL, 25.1 U/mL, 25.2 U/mL, 25.3 U/mL, 25.4 U/mL, 25.5 U/mL, 25.6 U/mL, 25.7 U/mL, 25.8 U/mL, 25.9 U/mL, 26.0 U/mL, or 26.5 U/mL in serum for CA19-9, and higher
than or equal to 5.0 ng/mL, 5.1 ng/mL, 5.2 ng/mL, 5.3 ng/mL, 5.4 ng/mL, 5.5 ng/mL, 5.6 ng/mL, 5.7 ng/mL, 5.8 ng/mL, 5.9 ng/mL, 6.0 ng/mL, 6.1 ng/mL, 6.2 ng/mL, 6.3 ng/mL, 6.4 ng/mL, 6.5 ng/mL, 6.6 ng/mL, 6.7 ng/mL, 6.8 ng/mL, 6.9 ng/mL, 7.0 ng/mL, 7.5 ng/mL, or 8.0 ng/mL in serum for AFP. The reference level of CYFRA may be at least about 2.13 ng/mL, the reference level of CRP may be at least about 15.4 mg/mL, the reference level of TIMP-1 may be at least about 148 ng/mL, the reference level of CA19-9 may be at least about 25.0 U/mL, and the reference level of AFP may be at least about 6.7 ng/mL. The method may further comprise administering a cancer treatment regimen or cancer monitoring regimen to the subject diagnosed as suffering from or at risk of suffering from a cancer other than CRC. The cancer treatment regimen may comprise administering at least one of surgery, radiotherapeutic therapy, radiotherapeutic treatments, chemotherapy, targeted therapy, or combinations thereof, to the subject. The cancer monitoring regimen may comprise determining CYFRA, CRP, TIMP-1, CA19-9, and AFP levels at periodic intervals. Determining the levels of CYFRA, CRP, TIMP-1, CA19-9, and AFP may comprise an immunological method with molecules specifically binding to CYFRA, CRP, TIMP-1, CA19-9, and AFP. The molecules specifically binding to CYFRA, CRP, TIMP-1, CA19-9, and AFP may comprise at least one antibody capable of specifically binding CYFRA, CRP, TIMP-1, CA19-9, and AFP. Determining the level CYFRA, CRP, TIMP-1, CA19-9, and AFP may involve the step of contacting the biological sample with at least one antibody selected from the group consisting of: an antibody that specifically binds to CYFRA, an antibody that specifically binds to CRP, an antibody that specifically binds to TIMP-1, an antibody that specifically binds to CA19-9, an antibody that specifically binds to AFP, and combinations thereof. Determining the level of CYFRA, CRP, TIMP-1, CA19-9, and AFP may involve the step of assaying the biological sample for CYFRA, CRP, TIMP-1, CA19-9, and AFP by an immunoassay that employs at least one capture antibody and at least one antibody labeled with a detectable label, which generates a signal, and comprises comparing a signal generated by the detectable label as a direct or indirect indication of the amount of CYFRA, CRP, TIMP-1, CA19-9, and AFP in the biological sample, wherein the capture antibody and the antibody labeled with a detectable label comprise: (a) at least one capture antibody that specifically binds to CYFRA and at least one antibody labeled with a detectable label; (b) at least one capture antibody that specifically binds to CRP and at least one antibody labeled with a detectable label; (c) at least one capture antibody that specifically binds to TIMP-1 and at least one antibody labeled with a detectable label; (d) at least one capture antibody that specifically binds to CA19-9 and at least one antibody labeled with a detectable label; and (e) at least one capture antibody that specifically binds to AFP and at least one antibody labeled with a detectable label. The immunological method may comprise: (a) measuring the levels of CYFRA by: (i) contacting the test sample with at least one capture antibody, wherein the capture antibody binds to an epitope on CYFRA or a fragment of CYFRA to form a capture antibody-CYFRA antigen complex; (ii) contacting the capture antibody-CYFRA antigen complex with at least one detection antibody comprising a detectable label, wherein the detection antibody binds to an epitope on CYFRA that is not bound by the capture antibody and forms a capture antibody-CYFRA antigen-detection antibody complex; and (iii) determining the CYFRA levels in the test sample based on the signal generated by the detectable label in the capture antibody-CYFRA antigen-detection antibody complex formed in (a)(ii); (b) measuring the levels of CRP by: (i) contacting the test sample with at least one capture antibody, wherein the capture antibody binds to an epitope on CRP or a fragment of CRP to form a capture antibody-CRP antigen complex; (ii) contacting the capture antibody-CRP antigen complex with at least one detection antibody comprising a detectable label, wherein the detection antibody binds to an epitope on CRP that is not bound by the capture antibody and forms a capture antibody-CRP antigen-detection antibody complex; and (iii) determining the CRP levels in the test sample based on the signal generated by the detectable label in the capture antibody-CRP antigen-detection antibody complex formed in (b)(ii); (c) measuring the levels of TIMP-1 by: (i) contacting the test sample with at least one capture antibody, wherein the capture antibody binds to an epitope on TIMP-1 or a fragment of TIMP-1 to form a capture antibody-TIMP-1 antigen complex; (ii) contacting the capture antibody-TIMP-1 antigen complex with at least one detection antibody comprising a detectable label, wherein the detection antibody binds to an epitope on TIMP-1 that is not bound by the capture antibody and forms a capture antibody-TIMP-1 antigen-detection antibody complex; and (iii) determining the TIMP-1 levels in the test sample based on the signal generated by the detectable label in the capture antibody-TIMP-1 antigen-detection antibody complex formed in (c)(ii); (d) measuring the levels of CA19-9 by: (i) contacting the test sample with at least one capture antibody, wherein the capture antibody binds to an epitope on CA19-9 or a fragment of CA19-9 to form a capture antibody-CA19-9 antigen complex; (ii) contacting the capture antibody-CA19-9 antigen complex with at least one detection antibody comprising a detectable label, wherein the detection antibody binds to an epitope on CA19-9 that is not bound by the capture antibody and forms a capture antibody-CA19-9 antigen-detection antibody complex; and (iii) determining the CA19-9 levels in the test sample based on the signal generated by the detectable label in the capture antibody-CA19-9 antigen-detection antibody complex formed in (d)(ii); and (e) measuring the levels of AFP by: (i) contacting the test sample with at least one capture antibody, wherein the capture antibody binds to an epitope on AFP or a fragment of AFP to form a capture antibody-AFP antigen complex; (ii) contacting the capture antibody-AFP antigen complex with at least one detection antibody comprising a detectable label, wherein the detection antibody binds to an epitope on AFP that is not bound by the capture antibody and forms a capture antibody-AFP antigen-detection antibody complex; and (iii) determining the AFP levels in the test sample based on the signal generated by the detectable label in the capture antibody-AFP antigen-detection antibody complex formed in (e)(ii). The antibody may be selected from the group consisting of: a polyclonal antibody, a monoclonal antibody, a human antibody, an immunoglobulin molecule, a disulfide linked Fv, a monoclonal antibody, an affinity matured antibody, a scFv, a chimeric antibody, a single domain antibody, a CDR-grafted antibody, a diabody, a humanized antibody, a multispecific antibody, a Fab, a dual specific antibody, a DVD, a Fab', a bispecific antibody, a F(ab')2, and a Fv. The method may further comprise determining the level of at least one additional biomarker of a cancer other than CRC in the biological sample selected from the group consisting of: Methyl Septin 9 (mS9), Galectin-3 (Gal3), Glycated hemoglobin (HbA1c), C3a, Cathepsin X (Cat X), soluble urokinase receptor (suPAR(I)), Plasminogen activator inhibitor-1 (PAI-1), Enolase 2 (ENO2), and combinations thereof, and comparing the level of the at least one additional biomarker of a cancer other than CRC to a reference level for the at least one additional biomarker of a cancer other than CRC. The cancer may be lung, ovary, breast, kidney, anal canal, unknown primary, B-cell lymphoma, uterine, prostate, gallbladder, larynx, malignant melanoma, pancreas, CLL, malignant myeloma, testes, small intestine, bladder, stomach, mesothelioma, adrenal gland, neuroendocrine, or esophagus cancer. The subject may be a human. The biological sample of a subject may be selected from a tissue sample, bodily fluid, whole blood, plasma, serum, urine, bronchoalveolar lavage fluid, and a cell culture suspension or fraction thereof. The biological sample of a subject may be blood plasma or blood serum. Disclosed herein is also a kit for performing said method. The kit comprising: (a) a reagent capable of specifically binding to CYFRA, a reagent capable of specifically binding to CRP, a reagent capable of specifically binding to TIMP-1, a reagent capable of specifically binding to CA19-9, and a reagent capable of specifically binding AFP to quantify the levels of CYFRA, CRP, TIMP-1, CA19-9, and AFP in the biological sample of a subject; and (b) a reference standard indicating reference levels of CYFRA, CRP, TIMP-1, CA19-9, and AFP. The kit may further comprise at least one additional reagent capable of specifically binding at least one additional biomarker of Methyl Septin 9 (mS9), Galectin-3 (Gal3), Glycated hemoglobin (HbA1c), C3a, Cathepsin X (Cat X), soluble urokinase receptor (suPAR(I)), Plasminogen activator inhibitor-1 (PAI-1), Enolase 2 (ENO2), or combinations thereof, in the biological sample to quantify the concentration of the at least one additional biomarker in the biological sample, and a reference standard indicating a reference level of the at least one additional biomarker of Methyl Septin 9 (mS9), Galectin-3 (Gal3), Glycated hemoglobin (HbA1c), C3a, Cathepsin X (Cat X), soluble urokinase receptor (suPAR(I)), Plasminogen activator inhibitor-1 (PAI-1), Enolase 2 (ENO2), or combinations thereof, in the biological sample.

Disclosed herein is also a method of determining whether a male subject is suffering from or at risk of suffering from a cancer other than colorectal cancer (CRC) cancer, wherein the male subject was confirmed not to have CRC by a colonoscopy. The method comprises the steps of: (a) obtaining a biological sample from a male subject; (b) determining the levels of Cytokeratin 19 Fragment (CYFRA), C-reactive protein (CRP), a tissue inhibitor of metalloproteinase 1 (TIMP-1), carbohydrate antigen 19-9 (CA19-9), and alpha-fetoprotein (AFP) in the biological sample from the male subject; (c) comparing the levels of CYFRA, CRP, TIMP-1, CA19-9, and AFP in the biological sample to reference levels of CYFRA, CRP, TIMP-1, CA19-9, and AFP; (d) providing a CYFRA score for the male subject wherein the male subject gets a score of 1 if the level of CYFRA in the biological sample is greater than the reference level of CYFRA and a score of 0 if the level of CYFRA in the biological sample is equal or less than the reference level of CYFRA; (e) providing a CRP score for the male subject wherein the male subject gets a score of 1 if the level of CRP in the biological sample is greater than the reference level of CRP and a score of 0 if the level of CRP in the biological sample is equal or less than the reference level of CRP; (f) providing a TIMP-1 score for the male subject wherein the male subject gets a score of 1 if the level of TIMP-1 in the biological sample is greater than the reference level of TIMP-1 and a score of 0 if the level of TIMP-1 in the biological sample is equal or less than the reference level of TIMP-1; (g) providing a CA19-9 score for the male subject wherein the male subject gets a score of 1 if the level of CA19-9 in the biological sample is less than the reference level of CA19-9 and a score of 0 if the level of CA19-9 in the biological sample is equal or greater than the reference level of CA19-9; (h) providing a AFP score for the male subject wherein the male subject gets a score of 1 if the level of AFP in the biological sample is less than the reference level of AFP and a score of 0 if the level of AFP in the biological sample is equal or greater than the reference level of AFP; (i) adding the CYFRA score, CRP
score, TIMP-1 score, CA19-9 score, and AFP score to generate a total score; and (j) providing a diagnosis of a male subject suffering from or at risk of suffering from a cancer other than CRC if the total score is greater than a reference score. The reference levels of CYFRA, CRP, TIMP-1, CA19-9, and AFP may be the CYFRA, CRP, TIMP-1, CA19-9, and AFP cutoff values determined by adaptive index model methodology from biological samples of a reference group. The reference group may be selected from the group consisting of a control group and a cancer group. The CYFRA, CRP, TIMP-1, CA19-9, and AFP reference level may be higher than or equal to 1.0 ng/mL, 1.1 ng/mL, 1.2 ng/mL, 1.3 ng/mL, 1.4 ng/mL, 1.5 ng/mL, 1.6 ng/mL, 1.7 ng/mL, 1.8 ng/mL, 1.9 ng/mL, 2.0 ng/mL, 2.1 ng/mL, 2.2 ng/mL, 2.3 ng/mL, 2.4 ng/mL, 2.5 ng/mL, 3.0 ng/mL, or 3.5 ng/mL in serum for CYFRA in combination with levels higher than or equal to 14.0 mg/mL, 14.1 mg/mL, 14.2 mg/mL, 14.3 mg/mL, 14.4 mg/mL, 14.5 mg/mL, 14.6 mg/mL, 14.7 mg/mL, 14.8 mg/mL, 14.9 mg/mL, 15.0 mg/mL, 15.1 mg/mL, 15.2 mg/mL, 15.3 mg/mL, 15.4 mg/mL, 15.5 mg/mL, 15.6 mg/mL, 15.7 mg/mL, 15.8 mg/mL, 15.9 mg/mL, 16.0 mg/mL, 16.1 mg/mL, 16.2 mg/mL, 16.3 mg/mL, 16.4 mg/mL, 16.5 mg/mL, 17.0 mg/mL, or 17.5 mg/mL in serum for CRP, levels higher than or equal to 140 ng/mL, 141 ng/mL, 142 ng/mL, 143 ng/mL, 144 ng/mL, 145 ng/mL, 146 ng/mL, 147 ng/mL, 148 ng/mL, 149 ng/mL, 150 ng/mL, 151 ng/mL, 152 ng/mL, 153 ng/mL, 154 ng/mL, 155 ng/mL, 156 ng/mL, 157 ng/mL, 158 ng/mL, 159 ng/mL, or 160 ng/mL in serum for TIMP-1, higher than or equal to 24.0 U/mL, 24.1 U/mL, 24.2 U/mL, 24.3 U/mL, 24.4 U/mL, 24.5 U/mL, 24.6 U/mL, 24.7 U/mL, 24.8 U/mL, 24.9 U/mL, 25.0 U/mL, 25.1 U/mL, 25.2 U/mL, 25.3 U/mL, 25.4 U/mL, 25.5 U/mL, 25.6 U/mL, 25.7 U/mL, 25.8 U/mL, 25.9 U/mL, 26.0 U/mL, or 26.5 U/mL in serum for CA19-9, and higher than or equal to 5.0 ng/mL, 5.1 ng/mL, 5.2 ng/mL, 5.3 ng/mL, 5.4 ng/mL, 5.5 ng/mL, 5.6 ng/mL, 5.7 ng/mL, 5.8 ng/mL, 5.9 ng/mL, 6.0 ng/mL, 6.1 ng/mL, 6.2 ng/mL, 6.3 ng/mL, 6.4 ng/mL, 6.5 ng/mL, 6.6 ng/mL, 6.7 ng/mL, 6.8 ng/mL, 6.9 ng/mL, 7.0 ng/mL, 7.5 ng/mL, or 8.0 ng/mL in serum for AFP. The reference level of CYFRA may be at least about 2.13 ng/mL, the reference level of CRP may be at least about 15.4 mg/mL, the reference level of TIMP-1 may be at least about 148 ng/mL, the reference level of CA19-9 may be at least about 25.0 U/mL, and the reference level of AFP may be at least about 6.7 ng/mL. The reference score may be 0, 1, 2, 3, 4, or 5. If the total score is greater than 1, the subject may be diagnosed as suffering from or at risk of suffering from cancer other than CRC. The method may further comprise administering a cancer treatment regimen or cancer monitoring regimen to the subject diagnosed as suffering from or at risk of suffering from a cancer other than CRC. The cancer treatment regimen may comprise administering at least one of surgery, radiotherapeutic therapy, radiotherapeutic treatments, chemotherapy, targeted therapy, or combinations thereof, to the subject. The cancer monitoring regimen may comprise determining CYFRA, CRP, TIMP-1, CA19-9, and AFP levels at periodic intervals. Determining the levels of CYFRA, CRP, TIMP-1, CA19-9, and AFP may comprise an immunological method with molecules specifically binding to CYFRA, CRP, TIMP-1, CA19-9, and AFP. The molecules specifically binding to CYFRA, CRP, TIMP-1, CA19-9, and AFP may comprise at least one antibody capable of specifically binding CYFRA, CRP, TIMP-1, CA19-9, and AFP. Determining the level CYFRA, CRP, TIMP-1, CA19-9, and AFP may involve the step of contacting the biological sample with at least one antibody selected from the group consisting of: an antibody that specifically binds to CYFRA, an antibody that specifically binds to CRP, an antibody that specifically binds to TIMP-1, an antibody that specifically binds to CA19-9, an antibody that specifically binds to AFP, and combinations thereof. Determining the level of CYFRA, CRP, TIMP-1, CA19-9, and AFP may involve the step of assaying the biological sample for CYFRA, CRP, TIMP-1, CA19-9, and AFP by an immunoassay that employs at least one capture antibody and at least one antibody labeled with a detectable label, which generates a signal, and comprises comparing a signal generated by the detectable label as a direct or indirect indication of the amount of CYFRA, CRP, TIMP-1, CA19-9, and AFP in the biological sample, wherein the capture antibody and the antibody labeled with a detectable label comprise: (a) at least one capture antibody that specifically binds to CYFRA and at least one antibody labeled with a detectable label; (b) at least one capture antibody that specifically binds to CRP and at least one antibody labeled with a detectable label; (c) at least one capture antibody that specifically binds to TIMP-1 and at least one antibody labeled with a detectable label; (d) at least one capture antibody that specifically binds to CA19-9 and at least one antibody labeled with a detectable label; and (e) at least one capture antibody that specifically binds to AFP and at least one antibody labeled with a detectable label. The immunological method may comprise: (a) measuring the levels of CYFRA by: (i) contacting the test sample with at least one capture antibody, wherein the capture antibody binds to an epitope on CYFRA or a fragment of CYFRA to form a capture antibody-CYFRA antigen complex; (ii) contacting the capture antibody-CYFRA antigen complex with at least one detection antibody comprising a detectable label, wherein the detection antibody binds to an epitope on CYFRA that is not bound by the capture antibody and forms a capture antibody-CYFRA antigen-detection antibody complex; and (iii) determining the CYFRA levels in the test sample based on the signal generated by the detectable label in the capture antibody-CYFRA antigen-detection antibody complex formed in (a)(ii); (b) measuring the levels of CRP by: (i) contacting the test sample with at least one capture antibody, wherein the capture antibody binds to an epitope on CRP or a fragment of CRP to form a capture antibody-CRP antigen complex; (ii) contacting the capture antibody-CRP antigen complex with at least one detection antibody comprising a detectable label, wherein the detection antibody binds to an epitope on CRP that is not bound by the capture antibody and forms a capture antibody-CRP antigen-detection antibody complex; and (iii) determining the CRP levels in the test sample based on the signal generated by the detectable label in the capture antibody-CRP antigen-detection antibody complex formed in (b)(ii); (c) measuring the levels of TIMP-1 by: (i) contacting the test sample with at least one capture antibody, wherein the capture antibody binds to an epitope on TIMP-1 or a fragment of TIMP-1 to form a capture antibody-TIMP-1 antigen complex; (ii) contacting the capture antibody-TIMP-1 antigen complex with at least one detection antibody comprising a detectable label, wherein the detection antibody binds to an epitope on TIMP-1 that is not bound by the capture antibody and forms a capture antibody-TIMP-1 antigen-detection antibody complex; and (iii) determining the TIMP-1 levels in the test sample based on the signal generated by the detectable label in the capture antibody-TIMP-1 antigen-detection antibody complex formed in (c)(ii); (d) measuring the levels of CA19-9 by: (i) contacting the test sample with at least one capture antibody, wherein the capture antibody binds to an epitope on CA19-9 or a fragment of CA19-9 to form a capture antibody-CA19-9 antigen complex; (ii) contacting the capture antibody-CA19-9 antigen complex with at least one detection antibody comprising a detectable label, wherein the detection antibody binds to an epitope on CA19-9 that is not bound by the capture antibody and forms a capture antibody-CA19-9 antigen-detection antibody complex; and (iii) determining the CA19-9 levels in the test sample based on the signal generated by the detectable label in the capture antibody-CA19-9 antigen-detection antibody complex formed in (d)(ii); and (e) measuring the levels of AFP by: (i) contacting the test sample with at least one capture antibody, wherein the capture antibody binds to an epitope on AFP or a fragment of AFP to form a capture antibody-AFP antigen complex; (ii) contacting the capture antibody-AFP antigen complex with at least one detection antibody comprising a detectable label, wherein the detection antibody binds to an epitope on AFP that is not bound by the capture antibody and forms a capture antibody-AFP antigen-detection antibody complex; and (iii) determining the AFP levels in the test sample based on the signal generated by the detectable label in the capture antibody-AFP antigen-detection antibody complex formed in (e)(ii). The antibody may be selected from the group consisting of: a polyclonal antibody, a monoclonal antibody, a human antibody, an immunoglobulin molecule, a disulfide linked Fv, a monoclonal antibody, an affinity matured antibody, a scFv, a chimeric antibody, a single domain antibody, a CDR-grafted antibody, a diabody, a humanized antibody, a multispecific antibody, a Fab, a dual specific antibody, a DVD, a Fab', a bispecific antibody, a F(ab')2, and a Fv. The method may further comprise determining the level of at least one additional biomarker of a cancer other than CRC in the biological sample selected from the group consisting of: Methyl Septin 9 (mS9), Galectin-3 (Gal3), Glycated hemoglobin (HbA1c), C3a, Cathepsin X (Cat X), soluble urokinase receptor (suPAR(I)), Plasminogen activator inhibitor-1 (PAI-1), Enolase 2 (ENO2), and combinations thereof, and comparing the level of the at least one additional biomarker of a cancer other than CRC to a reference level for the at least one additional biomarker of a cancer other than CRC. The cancer may be lung, ovary, breast, kidney, anal canal, unknown primary, B-cell lymphoma, uterine, prostate, gallbladder, larynx, malignant melanoma, pancreas, CLL, malignant myeloma, testes, small intestine, bladder, stomach, mesothelioma, adrenal gland, neuroendocrine, or esophagus cancer. The subject may be a human. The biological sample of a subject may be selected from a tissue sample, bodily fluid, whole blood, plasma, serum, urine, bronchoalveolar lavage fluid, and a cell culture suspension or fraction thereof. The biological sample of a subject may be blood plasma or blood serum. Disclosed herein is also a kit for performing said method. The kit comprising: (a) a reagent capable of specifically binding to CYFRA, a reagent capable of specifically binding to CRP, a reagent capable of specifically binding to TIMP-1, a reagent capable of specifically binding to CA19-9, and a reagent capable of specifically binding AFP to quantify the levels of CYFRA, CRP, TIMP-1, CA19-9, and AFP in the biological sample of a subject; and (b) a reference standard indicating reference levels of CYFRA, CRP, TIMP-1, CA19-9, and AFP. The kit may further comprise at least one additional reagent capable of specifically binding at least one additional biomarker of Methyl Septin 9 (mS9), Galectin-3 (Gal3), Glycated hemoglobin (HbA1c), C3a, Cathepsin X (Cat X), soluble urokinase receptor (suPAR(I)), Plasminogen activator inhibitor-1 (PAI-1), Enolase 2 (ENO2), or combinations thereof, in the biological sample to quantify the concentration of the at least one additional biomarker in the biological sample, and a reference standard indicating a reference level of the at least one additional biomarker of Methyl Septin 9 (mS9), Galectin-3 (Gal3), Glycated hemoglobin (HbA1c), C3a, Cathepsin X (Cat X), soluble urokinase receptor (suPAR(I)), Plasminogen activator inhibitor-1 (PAI-1), Enolase 2 (ENO2), or combinations thereof, in the biological sample.

Disclosed herein is also a method of determining whether a female subject is suffering from or at risk of suffering from a cancer other than colorectal cancer (CRC) cancer, wherein the female subject was confirmed not to have CRC by a colonoscopy. The method comprises the steps of: (a) obtaining a biological sample from the female subject; (b) determining the levels of Cytokeratin 19 Fragment (CYFRA), carcinoembryonic antigen (CEA), and C-reactive protein (CRP), in the biological sample from the female subject; (c) comparing the levels of CYFRA, CEA, and CRP in the biological sample to reference levels of CYFRA, CEA, and CRP; and (d) providing a diagnosis of the female subject as suffering from or at risk of suffering from a cancer other than CRC if the levels of C YFRA, CEA, and CRP in the biological sample are greater than the reference levels of CYFRA, CEA, and CRP. The reference levels of CYFRA, CEA, and CRP may be the CYFRA, CEA, and CRP cutoff values determined by adaptive index model methodology from biological samples of a reference group. The reference group may be selected from the group consisting of a control group and a cancer group. The CYFRA, CEA, and CRP reference level may be higher than or equal to 1.0 ng/mL, 1.1 ng/mL, 1.2 ng/mL, 1.3 ng/mL, 1.4 ng/mL, 1.5 ng/mL, 1.6 ng/mL, 1.7 ng/mL, 1.8 ng/mL, 1.9 ng/mL, 2.0 ng/mL, 2.1 ng/mL, 2.2 ng/mL, 2.3 ng/mL, 2.4 ng/mL, 2.5 ng/mL, 3.0 ng/mL, or 3.5 ng/mL in serum for CYFRA in combination with levels higher than or equal to 4.0 ng/mL, 4.1 ng/mL, 4.2 ng/mL, 4.3 ng/mL, 4.4 ng/mL, 4.5 ng/mL, 4.6 ng/mL, 4.7 ng/mL, 4.8 ng/mL, 4.9 ng/mL, 5.0 ng/mL, 5.1 ng/mL, 5.2 ng/mL, 5.3 ng/mL, 5.4 ng/mL, 5.5 ng/mL, 5.6 ng/mL, 5.7 ng/mL, 5.8 ng/mL, 5.9 ng/mL, 6.0 ng/mL, 6.1 ng/mL, 6.2 ng/mL, 6.3 ng/mL, 6.4 ng/mL, 6.5 ng/mL, 6.6 ng/mL, 6.7 ng/mL, 6.8 ng/mL, 6.9 ng/mL, 7.0 ng/mL, or 7.5 ng/mL in serum for CEA, and levels higher than or equal to 9.0 mg/mL, 9.1 mg/mL, 9.2 mg/mL, 9.3 mg/mL, 9.4 mg/mL, 9.5 mg/mL, 9.6 mg/mL, 9.7 mg/mL, 9.8 mg/mL, 9.9 mg/mL, 10.0 mg/mL, 10.1 mg/mL, 10.2 mg/mL, 10.3 mg/mL, 10.4 mg/mL, 10.5 mg/mL, 10.6 mg/mL, 10.7 mg/mL, 10.8 mg/mL, 10.9 mg/mL, 11.0 mg/mL, 11.5 mg/mL, or 12.0 mg/mL in serum for CRP. The reference level of CYFRA may be at least about 2.06 ng/mL, the reference level of CEA may be at least about 5.7 ng/mL, and the reference level of CRP may be at least about 10.4 mg/mL. The reference score may be 0, 1, 2, or 3. If the total score is greater than 1, the subject may be diagnosed as suffering from or at risk of suffering from CRC. The method of may further comprise administering a cancer treatment regimen or a cancer monitoring regimen to the subject diagnosed as suffering from or at risk of suffering from cancer other than CRC. The cancer treatment regimen may comprise administering at least one of surgery, radiotherapeutic therapy, radiotherapeutic treatments, chemotherapy, targeted therapy, or combinations thereof, to the subject. The cancer monitoring regimen may comprise determining CYFRA, CEA, and CRP levels at periodic intervals. Determining the levels of CYFRA, CEA, and CRP may comprise an immunological method with molecules specifically binding to CYFRA, CEA, and CRP. The molecules specifically binding to CYFRA, CEA, and CRP may comprise at least one antibody capable of specifically binding CYFRA, CEA, and CRP. Determining the level CYFRA, CEA, and CRP may involve the step of contacting the biological sample with at least one antibody selected from the group consisting of: an antibody that specifically binds to CYFRA, an antibody that specifically binds to CEA, an antibody that specifically binds to CRP, and combinations thereof. Determining the level of CYFRA, CEA, and CRP may involve the step of assaying the biological sample for CYFRA, CEA, and CRP by an immunoassay that employs at least one capture antibody and at least one antibody labeled with a detectable label, which generates a signal, and comprises comparing a signal generated by the detectable label as a direct or indirect indication of the amount of CYFRA, CEA, and CRP in the biological sample, wherein the capture antibody and the antibody labeled with a detectable label comprise: (a) at least one capture antibody that specifically binds to CYFRA and at least one antibody labeled with a detectable label; (b) at least one capture antibody that specifically binds to CEA and at least one antibody labeled with a detectable label; and (c) at least one capture antibody that specifically binds to CRP and at least one antibody labeled with a detectable label. The immunological method may comprise: (a) measuring the levels of CYFRA by: (i) contacting the test sample with at least one capture antibody, wherein the capture antibody binds to an epitope on CYFRA or a fragment of CYFRA to form a capture antibody-CYFRA antigen complex; (ii) contacting the capture antibody-CYFRA antigen complex with at least one detection antibody comprising a detectable label, wherein the detection antibody binds to an epitope on CYFRA that is not bound by the capture antibody and forms a capture antibody-CYFRA antigen-detection antibody complex; and (iii) determining the CYFRA levels in the test sample based on the signal generated by the detectable label in the capture antibody-CYFRA antigen-detection antibody complex formed in (a)(ii); (b) measuring the levels of CEA by: (i) contacting the test sample with at least one capture antibody, wherein the capture antibody binds to an epitope on CEA or a fragment of CEA to form a capture antibody-CEA antigen complex; (ii) contacting the capture antibody-CEA antigen complex with at least one detection antibody comprising a detectable label, wherein the detection antibody binds to an epitope on CEA that is not bound by the capture antibody and forms a capture antibody-CEA antigen-detection antibody complex; and (iii) determining the CEA levels in the test sample based on the signal generated by the detectable label in the capture antibody-CEA antigen-detection antibody complex formed in (b)(ii); (c) measuring the levels of CRP by: (i) contacting the test sample with at least one capture antibody, wherein the capture antibody binds to an epitope on CRP or a fragment of CRP to form a capture antibody-CRP antigen complex; (ii) contacting the capture antibody-CRP antigen complex with at least one detection antibody comprising a detectable label, wherein the detection antibody binds to an epitope on CRP that is not bound by the capture antibody and forms a capture antibody-CRP antigen-detection antibody complex; and (iii) determining the CRP levels in the test sample based on the signal generated by the detectable label in the capture antibody-CRP antigen-detection antibody complex formed in (c)(ii). The antibody may be selected from the group consisting of: a polyclonal antibody, a monoclonal antibody, a human antibody, an immunoglobulin molecule, a disulfide linked Fv, a monoclonal antibody, an affinity matured antibody, a scFv, a chimeric antibody, a single domain antibody, a CDR-grafted antibody, a diabody, a humanized antibody, a multi specific antibody, a Fab, a dual specific antibody, a DVD, a Fab', a bispecific antibody, a F(ab')2, and a Fv. The method may further comprise determining the level of at least one additional biomarker of a cancer other than CRC in the biological sample selected from the group consisting of Methyl Septin 9 (mS9), Galectin-3 (Gal3), Glycated hemoglobin (HbA1c), C3a, Cathepsin X (Cat X), soluble urokinase receptor (suPAR(I)), Plasminogen activator inhibitor-1 (PAI-1), Enolase 2 (ENO2), and combinations thereof, and comparing the level of the at least one additional biomarker of a cancer other than CRC to a reference level for the at least one additional biomarker of a cancer other than CRC. The cancer may be lung, ovary, breast, kidney, anal canal, unknown primary, B-cell lymphoma, uterine, prostate, gallbladder, larynx, malignant melanoma, pancreas, CLL, malignant myeloma, testes, small intestine, bladder, stomach, mesothelioma, adrenal gland, neuroendocrine, or esophagus cancer. The subject may be a human. The biological sample of a subject may be selected from a tissue sample, bodily fluid, whole blood, plasma, serum, urine, bronchoalveolar lavage fluid, and a cell culture suspension or fraction thereof. The biological sample of a subject may be blood plasma or blood serum. Disclosed herein is also a kit for performing said method. The kit comprising: (a) a reagent capable of specifically binding to CYFRA, a reagent capable of specifically binding to CEA, and a reagent capable of specifically binding to CRP quantify the levels of CYFRA, CEA, and CRP in the biological sample of a subject; and (b) a reference standard indicating reference levels of CYFRA, CEA, and CRP. The kit may further comprise at least one additional reagent capable of specifically binding at least one additional biomarker of Methyl Septin 9 (mS9), Galectin-3 (Gal3), Glycated hemoglobin (HbA1c), C3a, Cathepsin X (Cat X), soluble urokinase receptor (suPAR(I)), Plasminogen activator inhibitor-1 (PAI-1), Enolase 2 (ENO2), or combinations thereof, in the biological sample to quantify the concentration of the at least one additional biomarker in the biological sample, and a reference standard indicating a reference level of the at least one additional biomarker of Methyl Septin 9 (mS9), Galectin-3 (Gal3), Glycated hemoglobin (HbA1c), C3a, Cathepsin X (Cat X), soluble urokinase receptor (suPAR(I)), Plasminogen activator inhibitor-1 (PAI-1), Enolase 2 (ENO2), or combinations thereof, in the biological sample.

Disclosed herein is also a method of determining whether a female subject is suffering from or at risk of suffering from a cancer other than colorectal cancer (CRC) cancer, wherein the female subject was confirmed not to have CRC by a colonoscopy. The method comprises the steps of: (a) obtaining a biological sample from a female subject; (b) determining the levels of Cytokeratin 19 Fragment (CYFRA), carcinoembryonic antigen (CEA), and C-reactive protein (CRP) in the biological sample from the female subject; (c) comparing the levels of CYFRA, CEA, and CRP in the biological sample to reference levels of CYFRA, CEA, and CRP; (d) providing a CYFRA score for the female subject wherein the female subject gets a score of 1 if the level of CYFRA in the biological sample is greater than the reference level of CYFRA and a score of 0 if the level of CYFRA in the biological sample is equal or less than the reference level of CYFRA; (e) providing a CEA score for the female subject wherein the female subject gets a score of 1 if the level of CEA in the biological sample is greater than the reference level of CEA and a score of 0 if the level of CEA in the biological sample is equal or less than the reference level of CEA; (f) providing a CRP score for the female subject wherein the female subject gets a score of 1 if the level of CRP in the biological sample is greater than the reference level of CRP and a score of 0 if the level of CRP in the biological sample is equal or less than the reference level of CRP; (g) adding the CYFRA score, CEA score, and CRP score to generate a total score; and (h) providing a diagnosis of a female subject suffering from or at risk of suffering from a cancer other than CRC if the total score is greater than a reference score. The reference levels of CYFRA, CEA, and CRP may be the CYFRA, CEA, and CRP cutoff values determined by adaptive index model methodology from biological samples of a reference group. The reference group may be selected from the group consisting of a control group and a cancer group. The CYFRA, CEA, and CRP reference level may be higher than or equal to 1.0 ng/mL, 1.1 ng/mL, 1.2 ng/mL, 1.3 ng/mL, 1.4 ng/mL, 1.5 ng/mL, 1.6 ng/mL, 1.7 ng/mL, 1.8 ng/mL, 1.9 ng/mL, 2.0 ng/mL, 2.1 ng/mL, 2.2 ng/mL, 2.3 ng/mL, 2.4 ng/mL, 2.5 ng/mL, 3.0 ng/mL, or 3.5 ng/mL in serum for CYFRA in combination with levels higher than or equal to 4.0 ng/mL, 4.1 ng/mL, 4.2 ng/mL, 4.3 ng/mL, 4.4 ng/mL, 4.5 ng/mL, 4.6 ng/mL, 4.7 ng/mL, 4.8 ng/mL, 4.9 ng/mL, 5.0 ng/mL, 5.1 ng/mL, 5.2 ng/mL, 5.3 ng/mL, 5.4 ng/mL, 5.5 ng/mL, 5.6 ng/mL, 5.7 ng/mL, 5.8 ng/mL, 5.9 ng/mL, 6.0 ng/mL, 6.1 ng/mL, 6.2 ng/mL, 6.3 ng/mL, 6.4 ng/mL, 6.5 ng/mL, 6.6 ng/mL, 6.7 ng/mL, 6.8 ng/mL, 6.9 ng/mL, 7.0 ng/mL, or 7.5 ng/mL in serum for CEA, and levels higher than or equal to 9.0 mg/mL, 9.1 mg/mL, 9.2 mg/mL, 9.3 mg/mL, 9.4 mg/mL, 9.5 mg/mL, 9.6 mg/mL, 9.7 mg/mL, 9.8 mg/mL, 9.9 mg/mL, 10.0 mg/mL, 10.1 mg/mL, 10.2 mg/mL, 10.3 mg/mL, 10.4 mg/mL, 10.5 mg/mL, 10.6 mg/mL, 10.7 mg/mL, 10.8 mg/mL, 10.9 mg/mL, 11.0 mg/mL, 11.5 mg/mL, or 12.0 mg/mL in serum for CRP. The reference level of CYFRA may be at least about 2.06 ng/mL, the reference level of CEA may be at least about 5.7 ng/mL, and the reference level of CRP may be at least about 10.4 mg/mL. The reference score may be 0, 1, 2, or 3. If the total score is greater than 1, the subject may be diagnosed as suffering from or at risk of suffering from CRC. The method of may further comprise administering a cancer treatment regimen or a cancer monitoring regimen to the subject diagnosed as suffering from or at risk of suffering from cancer other than CRC. The cancer treatment regimen may comprise administering at least one of surgery, radiotherapeutic therapy, radiotherapeutic treatments, chemotherapy, targeted therapy, or combinations thereof, to the subject. The cancer monitoring regimen may comprise determining CYFRA, CEA, and CRP levels at periodic intervals. Determining the levels of CYFRA, CEA, and CRP may comprise an immunological method with molecules specifically binding to CYFRA, CEA, and CRP. The molecules specifically binding to CYFRA, CEA, and CRP may comprise at least one antibody capable of specifically binding CYFRA, CEA, and CRP. Determining the level CYFRA, CEA, and CRP may involve the step of contacting the biological sample with at least one antibody selected from the group consisting of: an antibody that specifically binds to CYFRA, an antibody that specifically binds to CEA, an antibody that specifically binds to CRP, and combinations thereof. Determining the level of CYFRA, CEA, and CRP may involve the step of assaying the biological sample for CYFRA, CEA, and CRP by an immunoassay that employs at least one capture antibody and at least one antibody labeled with a detectable label, which generates a signal, and comprises comparing a signal generated by the detectable label as a direct or indirect indication of the amount of CYFRA, CEA, and CRP in the biological sample, wherein the capture antibody and the antibody labeled with a detectable label comprise: (a) at least one capture antibody that specifically binds to CYFRA and at least one antibody labeled with a detectable label; (b) at least one capture antibody that specifically binds to CEA and at least one antibody labeled with a detectable label; and (c) at least one capture antibody that specifically binds to CRP and at least one antibody labeled with a detectable label. The immunological method may comprise: (a) measuring the levels of CYFRA by: (i) contacting the test sample with at least one capture antibody, wherein the capture antibody binds to an epitope on CYFRA or a fragment of CYFRA to form a capture antibody-CYFRA antigen complex; (ii) contacting the capture antibody-CYFRA antigen complex with at least one detection antibody comprising a detectable label, wherein the detection antibody binds to an epitope on CYFRA that is not bound by the capture antibody and forms a capture antibody-CYFRA antigen-detection antibody complex; and (iii) determining the CYFRA levels in the test sample based on the signal generated by the detectable label in the capture antibody-CYFRA antigen-detection antibody complex formed in (a)(ii); (b) measuring the levels of CEA by: (i) contacting the test sample with at least one capture antibody, wherein the capture antibody binds to an epitope on CEA or a fragment of CEA to form a capture antibody-CEA antigen complex; (ii) contacting the capture antibody-CEA antigen complex with at least one detection antibody comprising a detectable label, wherein the detection antibody binds to an epitope on CEA that is not bound by the capture antibody and forms a capture antibody-CEA antigen-detection antibody complex; and (iii) determining the CEA levels in the test sample based on the signal generated by the detectable label in the capture antibody-CEA antigen-detection antibody complex formed in (b)(ii); (c) measuring the levels of CRP by: (i) contacting the test sample with at least one capture antibody, wherein the capture antibody binds to an epitope on CRP or a fragment of CRP to form a capture antibody-CRP antigen complex; (ii) contacting the capture antibody-CRP antigen complex with at least one detection antibody comprising a detectable label, wherein the detection antibody binds to an epitope on CRP that is not bound by the capture antibody and forms a capture antibody-CRP antigen-detection antibody complex; and (iii) determining the CRP levels in the test sample based on the signal generated by the detectable label in the capture antibody-CRP antigen-detection antibody complex formed in (c)(ii). The antibody may be selected from the group consisting of: a polyclonal antibody, a monoclonal antibody, a human antibody, an immunoglobulin molecule, a disulfide linked Fv, a monoclonal antibody, an affinity matured antibody, a scFv, a chimeric antibody, a single domain antibody, a CDR-grafted antibody, a diabody, a humanized antibody, a multispecific antibody, a Fab, a dual specific antibody, a DVD, a Fab', a bispecific antibody, a F(ab')2, and a Fv. The method may further comprise determining the level of at least one additional biomarker of a cancer other than CRC in the biological sample selected from the group consisting of Methyl Septin 9 (mS9), Galectin-3 (Gal3), Glycated hemoglobin (HbA1c), C3a, Cathepsin X (Cat X), soluble urokinase receptor (suPAR(I)), Plasminogen activator inhibitor-1 (PAI-1), Enolase 2 (ENO2), and combinations thereof, and comparing the level of the at least one additional biomarker of a cancer other than CRC to a reference level for the at least one additional biomarker of a cancer other than CRC. The cancer may be lung, ovary, breast, kidney, anal canal, unknown primary, B-cell lymphoma, uterine, prostate, gallbladder, larynx, malignant melanoma, pancreas, CLL, malignant myeloma, testes, small intestine, bladder, stomach, mesothelioma, adrenal gland, neuroendocrine, or esophagus cancer. The subject may be a human. The biological sample of a subject may be selected from a tissue sample, bodily fluid, whole blood, plasma, serum, urine, bronchoalveolar lavage fluid, and a cell culture suspension or fraction thereof. The biological sample of a subject may be blood plasma or blood serum. Disclosed herein is also a kit for performing said method. The kit comprising: (a) a reagent capable of specifically binding to CYFRA, a reagent capable of specifically binding to CEA, and a reagent capable of specifically binding to CRP quantify the levels of CYFRA, CEA, and CRP in the biological sample of a subject; and (b) a reference standard indicating reference levels of CYFRA, CEA, and CRP. The kit may further comprise at least one additional reagent capable of specifically binding at least one additional biomarker of Methyl Septin 9 (mS9), Galectin-3 (Gal3), Glycated hemoglobin (HbA1c), C3a, Cathepsin X (Cat X), soluble urokinase receptor (suPAR(I)), Plasminogen activator inhibitor-1 (PAI-1), Enolase 2 (ENO2), or combinations thereof, in the biological sample to quantify the concentration of the at least one additional biomarker in the biological sample, and a reference standard indicating a reference level of the at least one additional biomarker of Methyl Septin 9 (mS9), Galectin-3 (Gal3), Glycated hemoglobin (HbA1c), C3a, Cathepsin X (Cat X), soluble urokinase receptor (suPAR(I)), Plasminogen activator inhibitor-1 (PAI-1), Enolase 2 (ENO2), or combinations thereof, in the biological sample.

### BRIEF DESCRIPTION OF THE DRAWINGS

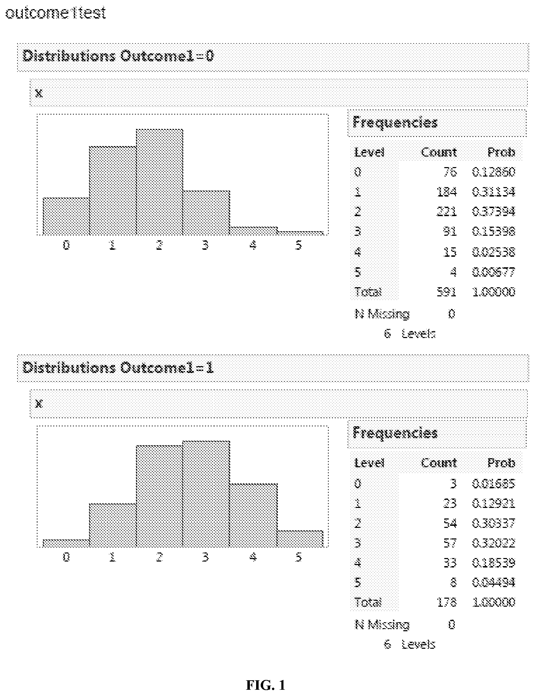

FIG. 1 shows Outcome 1 Test Distribution and male subjects.

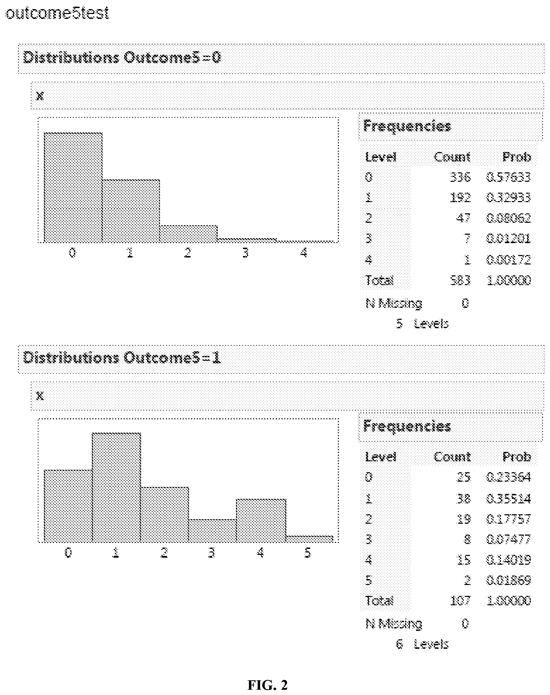

FIG. 2 shows Outcome 5 Test Distribution and male subjects.

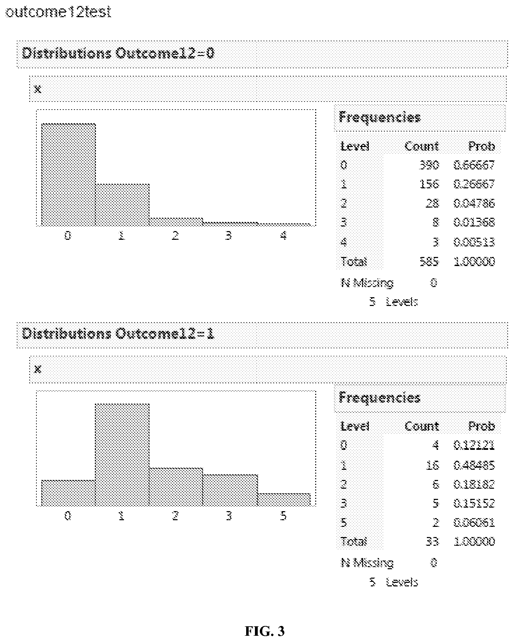

FIG. 3 shows Outcome 12 Test Distribution and male subjects.

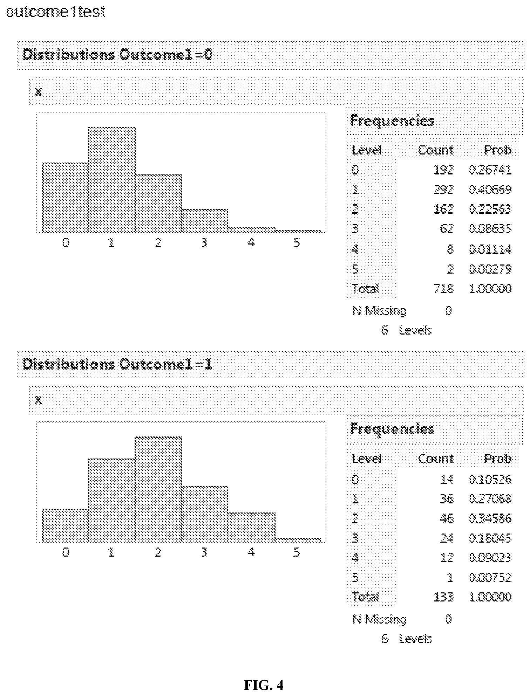

FIG. 4 shows Outcome 1 Test Distribution and female subjects.

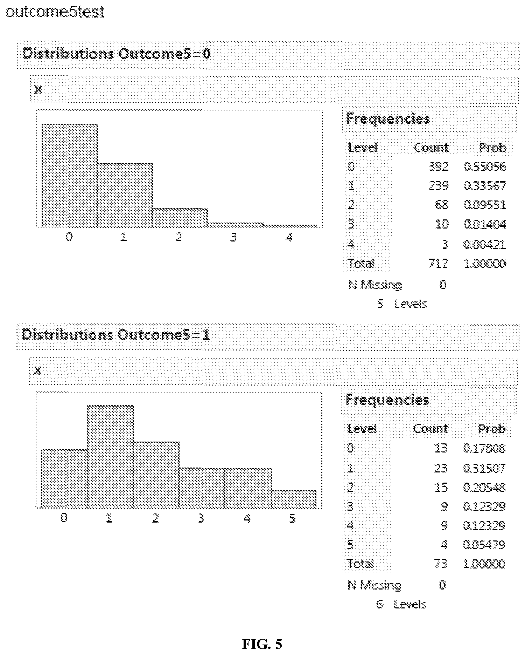

FIG. 5 shows Outcome 5 Test Distribution and female subjects.

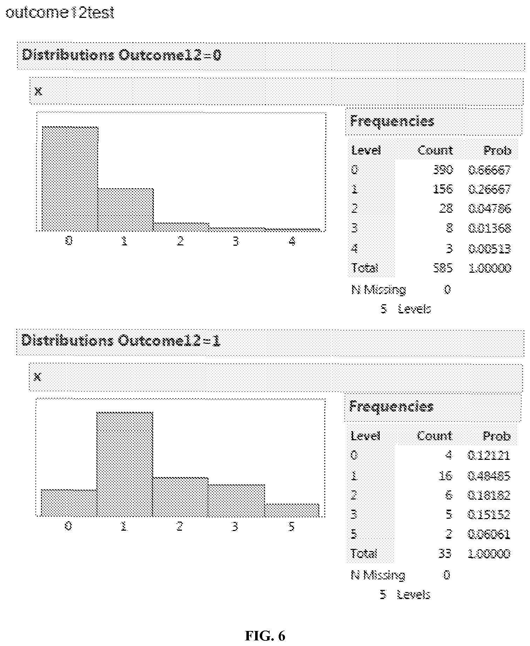

FIG. 6 shows Outcome 12 Test Distribution and female subjects.

### DETAILED DESCRIPTION

The present invention is directed to analyzing the levels of Alpha-fetoprotein (AFP), Carcinoembryonic antigen (CEA), ferritin, CYFRA 21-1 (CYFRA), Carbohydrate antigen 19-9 (CA19-9), Tissue Inhibitor of MetalloProteinnases-1 (TIMP-1), Galectin-3 (Gal3), and C-reactive protein (CRP) to identify, diagnose and treat colorectal cancer in a subject in need thereof. The biomarkers can be used to identify or diagnose, which includes aiding in identifying or diagnosing, a patient with colorectal cancer, to identify or aid in identifying whether a patient is suffering from colorectal cancer or high risk adenomas, to identify or aid in identifying a patient as a candidate for a CRC structural screening regimen or a CRC treatment regimen, to classify a patient's risk of developing colorectal cancer, as well as to determine or aid in determining a diagnosis, prognosis or treatment regimen. The methods described herein may be used in conjunction with other biomarkers and other factors, such as age and/or comorbidities with other diseases, to identify and diagnose, which includes aiding in identifying or diagnosing, patients suffering from high risk adenomas and/or CRC. The methods described herein can be adapted for use in an automated system or a semi-automated system. The methods of the present invention differ over previous colorectal cancer diagnostic methods by using a unique combination of AFP, CEA, ferritin, CYFRA, CA19-9, TIMP-1, Gal3, and CRP biomarkers to identify and diagnose, which includes aiding in identifying or diagnosing, patients suffering from colorectal cancer. These methods may accurately predict and determine the risk of colorectal cancer in a subject. These methods may be used to screen for high risk patients that should be referred to a colonoscopy to confirm and also screen for low risk patients that do not need a colonoscopy. The restriction of colonoscopy to individuals at risk of having CRC will improve the cost-effectiveness of the overall screening procedure.

Section headings as used in this section and the entire disclosure herein are merely for organization purposes and are not intended to be limiting.

### 1. Definitions

Unless otherwise defined, all technical and scientific terms used herein have the same meaning as commonly understood by one of ordinary skill in the art. In case of conflict, the present document, including definitions, will control. Preferred methods and materials are described below, although methods and materials similar or equivalent to those described herein can be used in practice or testing of the present invention. All publications, patent applications, patents and other references mentioned herein are incorporated by reference in their entirety. The materials, methods, and examples disclosed herein are illustrative only and not intended to be limiting.

As used herein, the singular forms "a," "an" and "the" include plural referents unless the context clearly dictates otherwise. For the recitation of numeric ranges herein, each intervening number there between with the same degree of precision is explicitly contemplated. For example, for the range 6-9, the numbers 7 and 8 are contemplated in addition to 6 and 9, and for the range 6.0-7.0, the numbers 6.0, 6.1, 6.2, 6.3, 6.4, 6.5, 6.6, 6.7, 6.8, 6.9 and 7.0 are explicitly contemplated.

The use of "or" means "and/or" unless stated otherwise. Furthermore, the use of the terms "including" and "having," as well as other forms of those terms, such as "includes," "included", "has," and "have" are not limiting.

As used throughout the specification and the claims, the following terms have the following meanings:

The term "adenoma" refers to a growth of epithelial cells of glandular origin which may be benign or malignant. They are also referred to as adenomatous polyps. Adenomas may be peduculated (large head with a narrow stalk) or sessile (broad based). They may be classified as tubular adenomas, tubulovillous adenomas, villous adenomas, and flat adenomas. The adenoma may be an adenocarcinoma. The adenoma may be an adenoma from a human patient which may be a large adenoma greater than 10 cm, a small adenoma less than 5 cm, or an adenoma between 0.5 cm and 15 cm in length. Adenomas originate in the glandular epithelium and have a dysplastic morphology. Some of these adenomas mature into large polyps, undergo abnormal growth and development, and ultimately progress into CRC.

An "adenoma of the colon," also called adenomatous polyps or tubular adenomas, are quite prevalent and are commonly found at colonoscopy. About 70% of polyps removed are of this type. Adenomas carry a cancer risk that rises as the polyp grows larger. Adenomatous polyps usually cause no symptoms, but, if detected early, can be removed during a colonoscopy before any cancer cells form. These polyps grow slowly and may take years to turn into cancer. Because of their tendency to become malignant and possibly leading to colon cancer, these are removed.

Villous and tubulovillous adenomas account for about 15% of the polyps that are removed. These are the most serious type of polyps with a very high cancer risk as they grow larger. Often, they are sessile (without a stem) making removal more difficult. Smaller ones can be removed in pieces sometimes over several colonoscopies. Larger sessile villous adenomas may require surgery for complete removal.

"Alpha-fetoprotein" or "AFP" as used interchangeably herein refers to a protein that is encoded by the *AFP* gene in humans. AFP is sometimes known as α-fetoprotein, alpha-1-fetoprotein, alpha-fetoglobulin, and alpha fetal protein. The *AFP* gene is located on the q arm of chromosome 4. AFP is a major plasma protein produced by the yolk sac and the liver during fetal development. It is thought to be the fetal form of serum albumin. AFP binds to copper, nickel, fatty acids, and bilirubin and is found in monomeric, dimeric, and trimeric forms. AFP is a glycoprotein of 591 amino acids and a carbohydrate moiety. AFP may be detected and quantified using Abbott ARCHITECT® AFP.

The "area under curve" or "AUC" refers to area under a ROC curve. AUC under a ROC curve is a measure of accuracy. An area of 1 represents a perfect test, whereas an area of 0.5 represents an insignificant test. A preferred AUC may be at least approximately 0.700, at least approximately 0.750, at least approximately 0.800, at least approximately 0.850, at least approximately 0.900, at least approximately 0.910, at least approximately 0.920, at least approximately 0.930, at least approximately 0.940, at least approximately 0.950, at least approximately 0.960, at least approximately 0.970, at least approximately 0.980, at least approximately 0.990, or at least approximately 0.995.

"C3a" as used herein refers to one of the proteins formed by the cleavage of complement component 3; the other being C3b. C3a stimulates mast cell degranulation, thus triggering an immune response. C3a plays an important role in chemotaxis. C3a is also an anaphylatoxin and the precursor of the important cytokine (adipokine) ASP through its interaction with carboxypeptidase B.

"Cancer" as used herein refers to the uncontrolled and unregulated growth of abnormal cells in the body. Cancerous cells are also called malignant cells. Cancer may invade nearby parts of the body and may also spread to more distant parts of the body through the lymphatic system or bloodstream. Cancers include Adrenocortical Carcinoma, Anal Cancer, Bladder Cancer, Brain Tumor, Breast Cancer, Carcinoid Tumor, Gastrointestinal, Carcinoma of Unknown Primary, Cervical Cancer, Colon Cancer, Endometrial Cancer, Esophageal Cancer, Extrahepatic Bile Duct Cancer, Ewings Family of Tumors (PNET), Extracranial Germ Cell Tumor, Intraocular Melanoma Eye Cancer, Gallbladder Cancer, Gastric Cancer (Stomach), Extragonadal Germ Cell Tumor, Gestational Trophoblastic Tumor, Head and Neck Cancer, Hypopharyngeal Cancer, Islet Cell Carcinoma, Kidney Cancer (renal cell cancer), Laryngeal Cancer, Acute Lymphoblastic Leukemia, Leukemia, Acute Myeloid, Chronic Lymphocytic Leukemia, Chronic Myelogenous Leukemia, Hairy Cell Leukemia, Lip and Oral Cavity Cancer, Liver Cancer, Non-Small Cell Lung Cancer, Small Cell Lung Cancer, AIDS-Related Lymphoma, Central Nervous System (Primary) Lymphoma, Cutaneous T-Cell Lymphoma, Hodgkin's Disease Lymphoma, Non-Hodgkin's Disease Lymphoma, Malignant Mesothelioma, Melanoma, Merkel Cell Carcinoma, Metasatic Squamous Neck Cancer with Occult Primary, Multiple Myeloma and Other Plasma Cell Neoplasms, Mycosis Fungoides, Myelodysplastic Syndrome, Myeloproliferative Disorders, Nasopharyngeal Cancer, euroblastoma, Oral Cancer, Oropharyngeal Cancer, Osteosarcoma, Ovarian Epithelial Cancer, Ovarian Germ Cell Tumor, Pancreatic Cancer, Exocrine, Pancreatic Cancer, Islet Cell Carcinoma, Paranasal Sinus and Nasal Cavity Cancer, Parathyroid Cancer, Penile Cancer, Pituitary Cancer, Plasma Cell Neoplasm, Prostate Cancer, Rhabdomyosarcoma, Rectal Cancer, Renal Cell Cancer (cancer of the kidney), Transitional Cell Renal Pelvis and Ureter, Salivary Gland Cancer, Sezary Syndrome, Skin Cancer, Small Intestine Cancer, Soft Tissue Sarcoma, Testicular Cancer, Malignant Thymoma, Thyroid Cancer, Urethral Cancer, Uterine Cancer, Unusual Cancer of Childhood, Vaginal Cancer, Vulvar Cancer, and Wilms' Tumor.

The term "cancer subject" as used herein means a subject having clinical signs or symptoms of CRC, high risk adenomas, and/or cancer other than CRC. The cancer subject is clinically evaluated for signs or symptoms of CRC, high risk adenomas, and/or cancer other than CRC, which evaluation may include routine physical examination and/or laboratory testing. A "cancer group" as used herein refers to a group of cancer subjects or subjects who have clinical signs or symptoms of CRC, high risk adenomas, and/or cancer other than CRC.

"Carbohydrate antigen 19-9," "CA 19-9" or "CA19-9" as used interchangeably herein refers to a mucin-glycoprotein derived from a human colorectal carcinoma cell line. It is related to the Lewis blood group protein and is present in the epithelial tissue of the stomach, gall bladder, pancreas, and prostate. CA19-9 has been FDA approved for use as an aid in the management of pancreatic cancer and is intended to be used in conjunction with other diagnostic information such as CT and MRI imaging procedures. CA19-9 belongs to the sialylated Lewis blood group antigen. Some individuals may be undetectable for CA19-9 because they are Lewis antigen negative. Increased serum CA19-9 may also be elevated in patients with nonmalignant conditions such as pancreatitis and other gastrointestinal disorders (Abbott ARCHITECT CA19-9 XR Package Insert 015-550 11/05). Elevated levels of CA19-9 may be informative for some CRC cancers. CA19-9 may be detected and quantified using Abbott ARCHITECT® CA19-9 XR.

"Carcinoembryonic antigen" or "CEA" as used interchangeably herein refers to a glycosyl phosphatidyl inositol (GPI)-cell surface anchored glycoprotein whose specialized sialofucosylated glycoforms serve as functional colon carcinoma L-selectin and E-selectin ligands. CEA is involved in cell adhesion. CEA is a tumor associated antigen that is characterized by a glycoprotein that is approximately 200 kDa in size. CEA is FDA approved and intended to be used as an aid in the prognosis and management of cancer patients in patients with changing concentrations of CEA. CEA is a marker related to CRC and has been clinically approved for monitoring GI cancers including CRC. Its performance to detect some CRC has been characterized. Clinical relevance has been shown in colorectal, gastric, lung, prostate, pancreatic, and ovarian cancers (Abbott ARCHITECT CEA Package Insert 34-4067/R4). CEA may be detected and quantified using Abbott ARCHITECT® CEA.

The term "cardiovascular disease," "cardiovascular disorder," or "CVD" as used interchangeably herein refers to a number of diseases that affect the heart and circulatory system. Cardiovascular disease encompasses diseases and conditions including, but not limited to arteriosclerosis, atherosclerosis, myocardial infarction, acute coronary syndrome, angina, congestive heart failure, aortic aneurysm, aortic dissection, iliac or femoral aneurysm, pulmonary embolism, primary hypertension, atrial fibrillation, stroke, transient ischemic attack, systolic dysfunction, diastolic dysfunction, myocarditis, atrial tachycardia, ventricular fibrillation, endocarditis, arteriopathy, vasculitis, atherosclerotic plaque, vulnerable plaque, acute coronary syndrome, acute ischemic attack, sudden cardiac death, peripheral vascular disease, coronary artery disease (CAD), peripheral artery disease (PAD), and cerebrovascular disease.

"Cathepsin X" or "Cat X" as used interchangeably herein refers to a lysosomal cysteine protease involved in mechanisms of malignant progression and is associated with disorders of the immune system and neurodegenerative diseases. Cat X is also known as cathepsin B2, cysteine-type carboxypeptidase, cathepsin IV, cathepsin Z, acid carboxypeptidase, and lysosomal carboxypeptidase B. Cat X is secreted from tumor cells as a proenzyme and may serve to predict the disease status and risk of death for cancer patients, as well as predict responsiveness to particular treatments. Higher serum levels of Cat X correlates with shorter overall survival of colorectal patients.

"Colorectal cancer" or "CRC" as used interchangeably herein refers to cancer that originate in the large intestine (colon) or the rectum (end of the colon). Nearly all CRCs begin as noncancerous (benign) polyps that slowly develop into cancer. "Colon cancer" as used herein refers to cancer of the large intestine (colon). "Rectal cancer" as used herein refers to cancer of the last several inches of the colon that connects to the anus.

The term "control subject" as used herein means a healthy subject, i.e. a subject having no clinical signs or symptoms of CRC, high risk adenomas, or cancer other than CRC. A control subject may have low risk adenomas but does not have any cancer, including CRC, or high risk adenomas. The control subject is clinically evaluated for otherwise undetected signs or symptoms of CRC, high risk adenomas, or cancer other than CRC, which evaluation may include routine physical examination and/or laboratory testing. A "control group" as used herein refers to a group of control subjects or healthy subjects, i.e. a group of subjects who have no clinical signs or symptoms of CRC, high risk adenomas, or cancer other than CRC.

"C-reactive protein" or "CRP" as used interchangeably herein refers to an annular, pentameric protein found in the blood plasma. CRP is an acute-phase protein whose levels rises in response to inflammation. CRP binds to phosphocholine expressed on the surface of dead or dying cells (and some types of bacteria) in order to activate the complement system via the C1Q complex. CRP is synthesized by the liver in response to factors released by macrophages and fat cells (adipocytes). CRP has 224 amino acids, has a monomer molecular mass of 25106 Da, and has an annular pentameric discoid shape. A high-sensitivity CRP (hs-CRP) test may be used to measure low levels of CRP using laser nephelometry. The test gives results in 25 minutes with a sensitivity down to 0.04 mg/L. Inflammation is an early hallmark in cancer development, and CRP measurement may be helpful in assessment for patient risk for cancer.

"CYFRA 21-1" or "CYFRA" as used interchangeably herein refers to a cytokeratin 19 fragment that is soluble in serum and may be useful circulating tumor marker. Cytokeratins are epithelial proteins whose expression is not lost during malignant transformation. Although expressed in all body fluids, CYFRA major occurrence is in the lung. Levels rise significantly in patients with epithelial cell-associated carcinomas. CYFRA and other cytokeratin fragments may be elevated in many CRC patients. CYFRA may be detected and quantified using Abbott ARCHITECT® CYFRA 21-1.

"Diabetes" or "diabetic condition" as used interchangeably herein refers to a disease in which the body does not produce or use insulin correctly. Diabetic conditions include various types of diabetes mellitus, including autoimmune and idiopathic Type 1 diabetes mellitus and Type 2 diabetes mellitus. Diabetes mellitus is defined by the World Health Organization as having a fasting plasma glucose concentration greater than or equal to 7.0 mmol/l (126 mg/dl) or a 2-hour glucose level greater than equal to 11.1 mmol/l (200 mg/dl). Type 1 diabetes is an autoimmune disease in which the body actually fails to produce any insulin. Type 1 diabetes is characterized by total loss of beta cells so that the patient requires insulin by injection. Type 1 diabetes accounts for 10-15% of all diabetes. Type 1 diabetes is strongly associated with auto-antibodies and this association has become part of the definition/classification of type 1 diabetes. Type 2 diabetes is a metabolic disorder resulting from the body's inability to make enough, or properly use, insulin. It is the most common form of the disease and accounts for 85-90% of diabetes. Diabetic conditions may also include pre-diabetes, which refers to a physiological state in which the patient's glucose levels are above normal, but not yet at established levels for diabetes. While there are some individuals diagnosed as pre-diabetic that will eventually develop Type 2 diabetes, many individuals with pre-diabetic conditions will not convert.

The term "effective dosage" as used herein means a dosage of a drug effective for periods of time necessary, to achieve the desired therapeutic result. An effective dosage may be determined by a person skilled in the art and may vary according to factors such as the disease state, age, sex, and weight of the individual, and the ability of the drug to elicit a desired response in the individual.

"Enolase 2," "gamma-enolase," or "ENO2" as used interchangeably herein refers to an enzyme that in humans is encoded by the *ENO2* gene. ENO2, also known as neuron specific enolase, is a phosphopyruvate hydratase found in mature neurons and cells of neuronal origin. A switch from alpha enolase to gamma enolase occurs in neural tissue during development in rats and primates. Detection of ENO2 with antibodies can be used to identify neuronal cells and cells with neuroendocrine differentiation. ENO2 is produced by small cell carcinomas which are neuroendocrine in origin. ENO2 is a useful tumor marker for lung cancer patients

"Ferritin" as used herein refers to a ubiquitous intracellular protein that stores iron and releases it in a controlled fashion. The protein is produced by almost all living organisms, including algae, bacteria, higher plants, and animals. In humans, ferritin acts as a buffer against iron deficiency and iron overload. Ferritin is found in most tissues as a cytosolic protein, but small amounts are secreted into the serum where it functions as an iron carrier. Plasma ferritin is also an indirect marker of the total amount of iron stored in the body, hence serum ferritin is used as a diagnostic test for iron deficiency anemia. Ferritin is a globular protein complex consisting of 24 protein subunits and is the primary intracellular iron-storage protein in both prokaryotes and eukaryotes, keeping iron in a soluble and nontoxic form. Ferritin that is not combined with iron is called apoferritin. Ferritin, like CRP, is considered an acute phase reactant, with levels increasing in various clinical conditions including infections and cancers. Some ferritin complexes in vertebrates are hetero-oligomers of two highly related gene products with slightly different physiological properties. The ratio of the two homologous proteins in the complex depends on the relative expression levels of the two genes. Ferritin may be detected and quantified using Abbott ARCHITECT® ferritin.

"Galectin-3" or "Gal3" as used interchangeably herein refers to a multifunctional β-galactoside-binding protein involved in tumor progression and metastasis. Gal3 is increased in cancers and its binding partner Galectin-3 ligand is elevated in colorectal cancers. Gal3 is a member of family of proteins, i.e., the galectin family, which is defined by their binding specificity for β-galactoside sugars, such as N-acetyllactosamine (Galβ1-3GlcNAc or Galβ1-4GlcNAc), which can be bound to proteins by either N-linked or O-linked glycosylation. Unlike the majority of lectins, galectins are not membrane bound, but are soluble proteins with both intra- and extracellular functions. Galectins have distinct but overlapping distributions but are found primarily in the cytosol, nucleus, extracellular matrix or in circulation. Gal3 may be detected and quantified using Abbott ARCHITECT® Galectin-3.

"Glycated hemoglobin" or "HbA1c" as used interchangeably herein refers to a form of hemoglobin that is measured primarily to identify the average plasma glucose concentration over prolonged periods of time. HbAlc is formed in a non-enzymatic glycation pathway by hemoglobin's exposure to plasma glucose. Normal levels of glucose produce a normal amount of glycated hemoglobin. As the average amount of plasma glucose increases, the fraction of glycated hemoglobin increases in a predictable way. This serves as a marker for average blood glucose levels over the previous months prior to the measurement.

"High risk adenoma" as used herein refers to patients with tubular adenomas that are greater than 1 cm, 3 or more adenomas, villous elements, or high grade neoplasia (by pathology). High risk adenomas may be considered pre-cancerous lesions.

The term "normal control" or "healthy control" as used herein means a sample or specimen taken from a subject, or an actual subject who does not have CRC or any cancer, or is not at risk of developing cancer.

"Low risk adenomas" as used herein refers to patients with 1-2 tubular adenomas less than 1 cm in diameter with no high-grade dysplasia.

"Methyl Septin 9" or "mS9" as used interchangeably herein refers to a marker with clinical indications related to aiding in early detection of CRC. Methylation of the Septin 9 promoter region, also termed epigenetic modification, has been associated with colorectal cancer. Determination of epigenetic events is a useful tool for early detection of disease since regulation of gene expression by aberrant DNA methylation is a well-characterized event in tumor biology, and is extensively described for CRC. Increased levels of cell-free circulating methylated DNA in the blood of cancer patients compared to healthy controls have been reported and can be used as a target for CRC detection. Abbott RealTime mS9 Colorectal Cancer assay is an in vitro polymerase chain reaction (PCR) test for the qualitative detection of the presence of methylated Septin 9 in plasma.

"Neoplasia" as used herein refers to an abnormal growth of tissue. Neoplasia is commonly referred to as a tumor. The abnormal growth usually but not always forms a mass. "Colorectal neoplasia" as used herein refers to an abnormal growth in the colon or rectum.

"Non-CRC malignancies" or "non-CRC cancer" as used interchangeably herein refers to a cancer that is not CRC.

"Plasminogen activator inhibitor-1" or "PAI-1" as used interchangeably herein refers to a protein that is encoded by the *SERPINE1* gene in humans. PAI-1 is also known as endothelial plasminogen activator inhibitor or serpin E1. PAI-1 is a serine protease inhibitor (serpin) that functions as the principal inhibitor of tissue plasminogen activator (tPA) and urokinase (uPA), the activators of plasminogen and hence fibrinolysis (the physiological breakdown of blood clots). PAI-1 is the main inhibitor of the plasminogen activators.

"Predetermined cutoff," "cutoff," "predetermined level," and "reference level" as used herein refer to an assay cutoff value that is used to assess diagnostic, prognostic, or therapeutic efficacy results by comparing the assay results against the predetermined cutoff/level, where the predetermined cutoff/level already has been linked or associated with various clinical parameters (e.g., presence of disease, stage of disease, severity of disease, progression, non-progression, or improvement of disease, etc.). The disclosure provides exemplary predetermined levels and reference levels. However, it is well-known that cutoff values may vary depending on the nature of the immunoassay (e.g., antibodies employed, reaction conditions, sample purity, etc.). It further is well within the ordinary skill of one in the art to adapt the disclosure herein for other immunoassays to obtain immunoassay-specific cutoff values for those other immunoassays based on the description provided by this disclosure. Whereas the precise value of the predetermined cutoff/level may vary between assays, the correlations as described herein should be generally applicable.

"Risk assessment," "risk classification," "risk identification," or "risk stratification" of subjects (e.g., patients) as used herein refers to the evaluation of factors including biomarkers, to predict the risk of occurrence of future events including disease onset or disease progression, so that treatment decisions regarding the subject may be made on a more informed basis.

"Sample," "biological sample," "test sample," "specimen," "sample from a subject," and "patient sample" as used herein may be used interchangeable and may be a sample of blood, tissue, urine, serum, plasma, amniotic fluid, cerebrospinal fluid, placental cells or tissue, endothelial cells, leukocytes, or monocytes. The sample can be used directly as obtained from a patient or can be pre-treated, such as by filtration, distillation, extraction, concentration, centrifugation, inactivation of interfering components, addition of reagents, and the like, to modify the character of the sample in some manner as discussed herein or otherwise as is known in the art.

Any cell type, tissue, or bodily fluid may be utilized to obtain a sample. Such cell types, tissues, and fluid may include sections of tissues such as biopsy and autopsy samples, frozen sections taken for histologic purposes, blood (such as whole blood), plasma, serum, sputum, stool, tears, mucus, saliva, bronchoalveolar lavage (BAL) fluid, hair, skin, red blood cells, platelets, interstitial fluid, ocular lens fluid, cerebral spinal fluid, sweat, nasal fluid, synovial fluid, menses, amniotic fluid, semen, etc. Cell types and tissues may also include lymph fluid, ascetic fluid, gynecological fluid, urine, peritoneal fluid, cerebrospinal fluid, a fluid collected by vaginal rinsing, or a fluid collected by vaginal flushing. A tissue or cell type may be provided by removing a sample of cells from an animal, but can also be accomplished by using previously isolated cells (e.g., isolated by another person, at another time, and/or for another purpose). Archival tissues, such as those having treatment or outcome history, may also be used. Protein or nucleotide isolation and/or purification may not be necessary.

Methods well-known in the art for collecting, handling and processing urine, blood, serum and plasma, and other body fluids, are used in the practice of the present disclosure. The test sample can comprise further moieties in addition to the analyte of interest, such as antibodies, antigens, haptens, hormones, drugs, enzymes, receptors, proteins, peptides, polypeptides, oligonucleotides or polynucleotides. For example, the sample can be a whole blood sample obtained from a subject. It can be necessary or desired that a test sample, particularly whole blood, be treated prior to immunoassay as described herein, e.g., with a pretreatment reagent. Even in cases where pretreatment is not necessary (e.g., most urine samples, a pre-processed archived sample, etc.), pretreatment of the sample is an option that can be performed for mere convenience (e.g., as part of a protocol on a commercial platform). The sample may be used directly as obtained from the subject or following pretreatment to modify a characteristic of the sample. Pretreatment may include extraction, concentration, inactivation of interfering components, and/or the addition of reagents.

The term "sensitivity" as used herein refers to the number of true positives divided by the number of true positives plus the number of false negatives, where sensitivity ("sens") may be within the range of 0<sens<1. Ideally, method embodiments herein have the number of false negatives equaling zero or close to equaling zero, so that no subject is wrongly identified as not having adenoma when they indeed have adenoma. Conversely, an assessment often is made of the ability of a prediction algorithm to classify negatives correctly, a complementary measurement to sensitivity.

The term "specificity" as used herein refers to the number of true negatives divided by the number of true negatives plus the number of false positives, where specificity ("spec") may be within the range of 0<spec<1. Ideally, the methods described herein have the number of false positives equaling zero or close to equaling zero, so that no subject is wrongly identified as having adenoma when they do not in fact have adenoma. Hence, a method that has both sensitivity and specificity equaling one, or 100%, is preferred.

The term "subject", "patient" or "subject in the method" as used herein interchangeably, means any vertebrate, including, but not limited to, a mammal (e.g., cow, pig, camel, llama, horse, goat, rabbit, sheep, hamsters, guinea pig, cat, dog, rat, and mouse, a non-human primate (for example, a monkey, such as a cynomolgous or rhesus monkey, chimpanzee, etc.) and a human. In some embodiments, the subject or subject may be a human or a non-human. In some embodiments, the subject may be a human subject at risk or suspected at being at risk for developing or already having high risk adenomas, CRC, and/or cancer other than CRC. In some embodiments, the subject may be a human subject that may have cardiovascular disease and/or diabetes and may be at risk for developing or already having high risk adenomas, CRC, and/or cancer other than CRC.

"Soluble urokinase receptor" or "suPAR(I)" as used interchangeably herein refers to the soluble form of uPAR, which is a membrane bound receptor for urokinase (uPA). suPAR(I) is also known as suPAR and soluble urokinase-type plasminogen activator receptor. suPAR results from the cleavage and release of membrane-bound uPAR. suPAR concentration positively correlates to the activation level of the immune system and is present in plasma, urine, blood, serum, and cerebrospinal fluid. suPAR is a biomarker for activation of the inflammatory and immune systems. suPAR levels are positively correlated with pro-inflammatory biomarkers, such as tumor necrosis factor-α, leukocyte counts, and C-reactive protein. Elevated levels of suPAR are associated with increased risk of systemic inflammatory response syndrome (SIRS), cancer, Focal segmental glomerulosclerosis, cardiovascular disease, type 2 diabetes, infectious diseases, HIV, and mortality. suPARnostic is a prognostic test used to detect suPAR levels in blood plasma.

"Tissue Inhibitor of MetalloProteinnases-1" or "TIMP-1" as used interchangeably herein refers to a glycoprotein that is a member of the TIMP family and expressed from several tissues of organisms. TIMP-1 is a molecule with functions within growth, invasion and dissemination processes. TIMP-1 is a natural inhibitor of the matrix metalloproteinases (MMPs), a group of peptidases involved in degradation of the extracellular matrix. In addition, the encoded protein is able to promote cell proliferation in a wide range of cell types, and may also have an anti-apoptotic function. TIMP-1 was found to be elevated in more than 85% of the patients, who were scheduled to undergo surgery for CRC, compared with other malignant or inflammatory diseases and individuals with no disease. TIMP-1 may be elevated in plasma of many CRC patients. TIMP-1 may be detected and quantified using Abbott ARCHITECT® Tissue Inhibitor of Metalloproteinases-1 (TIMP-1).

The terms "treat," "treated," or "treating" as used herein refers to a therapeutic wherein the object is to slow down (lessen) an undesired physiological condition, disorder or disease, or to obtain beneficial or desired clinical results. For the purposes of this invention, beneficial or desired clinical results include, but are not limited to, alleviation of symptoms; diminishment of the extent of the condition, disorder or disease; stabilization (i.e., not worsening) of the state of the condition, disorder or disease; delay in onset or slowing of the progression of the condition, disorder or disease; amelioration of the condition, disorder or disease state; and remission (whether partial or total), whether detectable or undetectable, or enhancement or improvement of the condition, disorder or disease. Treatment also includes prolonging survival as compared to expected survival if not receiving treatment.

Unless otherwise defined herein, scientific and technical terms used in connection with the present disclosure shall have the meanings that are commonly understood by those of ordinary skill in the art. For example, any nomenclatures used in connection with, and techniques of, cell and tissue culture, molecular biology, immunology, microbiology, genetics and protein and nucleic acid chemistry and hybridization described herein are those that are well known and commonly used in the art. The meaning and scope of the terms should be clear; in the event however of any latent ambiguity, definitions provided herein take precedent over any dictionary or extrinsic definition. Further, unless otherwise required by context, singular terms shall include pluralities and plural terms shall include the singular.

### 2. Combination of Biomarkers

The present invention is directed to methods of using a combination of biomarkers for the detection of colon and rectum neoplasias (e.g., high grade adenomas) and early stage colorectal cancers (CRCs). These biomarkers, i.e., AFP, CEA, ferritin, CYFRA, CA19-9, TIMP-1, Gal3, and CRP biomarkers, may be used in combination with each other and/or with other factors in the methods of the present invention to (1) identify and determine whether or not a subject is suffering from or at risk of suffering from high risk adenomas and/or CRC; (2) identify and determine whether or not a male or female subject is suffering from or at risk of suffering from a cancer other than CRC; (3) provide a diagnosis of or aid in diagnosing high risk adenomas and/or CRC in a subject; (4) provide or aid in providing a diagnosis, prognosis and/or risk stratification of high risk adenomas and/or CRC in a subject having or suspected of high risk adenomas and/or CRC; (5) differentiate between subjects with high risk adenomas and/or CRC and low risk adenomas and/or no cancer; (6) differentiate between subjects with CRC and no cancer; and (7) monitor the progression of CRC in a subject.

For example, at least two biomarkers, at least three biomarkers, at least four biomarkers, at least five biomarkers, at least six biomarkers, at least seven biomarkers, or at least eight biomarkers selected from the group of AFP, CEA, ferritin, CYFRA, CA19-9, TIMP-1, Gal3, and CRP biomarkers may be used in the methods described below. For example, a combination of biomarkers may include AFP and at least one, at least two, at least three, at least four, at least five, at least six, or at least seven of CEA, ferritin, CYFRA, CA19-9, TIMP-1, Gal3, or CRP; CEA and at least one, at least two, at least three, at least four, at least five, at least six, or at least seven of AFP, ferritin, CYFRA, CA19-9, TIMP-1, Gal3, or CRP; ferritin and at least one, at least two, at least three, at least four, at least five, at least six, or at least seven of AFP, CEA, CYFRA, CA19-9, TIMP-1, Gal3, or CRP; CYFRA and at least one, at least two, at least three, at least four, at least five, at least six, or at least seven of AFP, CEA, ferritin, CA19-9, TIMP-1, Gal3, or CRP; CA19-9 and at least one, at least two, at least three, at least four, at least five, at least six, or at least seven of AFP, CEA, ferritin, CYFRA, TIMP-1, Gal3, or CRP; TIMP-1 and at least one, at least two, at least three, at least four, at least five, at least six, or at least seven of AFP, CEA, ferritin, CYFRA, CA19-9, Gal3, or CRP; Gal3 and at least one, at least two, at least three, at least four, at least five, at least six, or at least seven of AFP, CEA, ferritin, CYFRA, CA19-9, TIMP-1, or CRP; or CRP and at least one, at least two, at least three, at least four, at least five, at least six, or at least seven of AFP, CEA, ferritin, CYFRA, CA19-9, Gal3, or TIMP-1.

The methods include obtaining a biological sample from a subject, determining the levels of the chosen combination of biomarkers in the biological sample, and comparing the levels of the chosen biomarkers in the biological sample to reference levels of the chosen biomarkers. The differences in the levels between the chosen biomarkers in the biological sample and the reference levels may be used to provide a diagnosis of the subject.

The methods include obtaining a biological sample from a subject, determining the levels of the chosen combination of biomarkers in the biological sample, and comparing the levels of the chosen biomarkers in the biological sample to reference levels of the chosen biomarkers. The difference in the levels between the chosen biomarkers in the biological sample and the reference levels may be used to generate scores, which are used to provide a diagnosis of the subject. For a particular biomarker, a score of 1 or 0 is given if the level of the biomarker in the biological sample is greater than or less than the reference level of the biomarker. The scores generated for each of the biomarkers is added together to generate a total score. The total score is compared to a reference score and the diagnosis of the subject is based on whether the total score is greater than the reference score. The reference score may be a number between 0 and the maximum total score possible for a particular combination of biomarkers. The maximum total score for a particular combination of biomarkers is the number of biomarkers used in the method. For example, the reference score may be 0, 1, or 2 for a combination of 2 biomarkers, the reference score may be 0, 1, 2, or 3 for a combination of 3 biomarkers, the reference score may be 0, 1, 2, 3, or 4 for a combination of 4 biomarkers, the reference score may be 0, 1, 2, 3, 4, or 5 for a combination of 5 biomarkers, the reference score may be 0, 1, 2, 3, 4, 5, or 6 for a combination of 6 biomarkers, the reference score may be 0, 1, 2, 3, 4, 5, 6, or 7 for a combination of 7 biomarkers, the reference score may be 0, 1, 2, 3, 4, 5, 6, 7, or 8 for a combination of 8 biomarkers, the reference score may be 0, 1, 2, 3, 4, 5, 6, 7, 8, or 9 for a combination of 9 biomarkers, or the reference score may be 0, 1, 2, 3, 4, 5, 6, 7, 8, 9, or 10 for a combination of 10 biomarkers. The combination of biomarkers may be used with other biomarkers and/or factors, which may each generate a score that is included in the total score.

The sex of the subject may determine the combination of biomarkers, reference levels of the biomarkers, and/or reference score used to diagnose the subject. A combination of biomarkers with reference levels for the biomarkers may be used to diagnose a male subject. The same combination of biomarkers and the same reference levels for the biomarkers may be used to diagnose a female subject. The same combination of biomarkers and different reference levels for the biomarkers may be used to diagnose a female subject. A different combination of biomarkers and reference levels may be used to diagnose a female subject. A reference score used to diagnose a male subject may be the same or different from the reference score used to diagnose a female subject.

The combination of biomarkers may be used to diagnose all stages of CRC. Several classification systems have been devised to stage the extent of CRC, including the Dukes' system and the more detailed International Union against Cancer-American Joint Committee on Cancer TNM staging system. The TNM system, which is used for either clinical or pathological staging, is divided into four stages, each of which evaluates the extent of cancer growth with respect to primary tumor (T), regional lymph nodes (N), and distant metastasis (M). The system focuses on the extent of tumor invasion into the intestinal wall; invasion of adjacent structures; the number of regional lymph nodes that have been affected; and whether distant metastasis has occurred.

Stage 0 is characterized by *in situ* carcinoma (Tis), in which the cancer cells are located inside the glandular basement membrane (intraepithelial) or lamina propria (intramucosal). In this stage, the cancer has not spread to the regional lymph nodes (N0), and there is no distant metastasis (M0). In stage I, there is still no spread of the cancer to the regional lymph nodes and no distant metastasis, but the tumor has invaded the submucosa (T1) or has progressed further to invade the muscularis propria (T2). Stage II also involves no spread of the cancer to the regional lymph nodes and no distant metastasis, but the tumor has invaded the subserosa, or the nonperitonealized pericolic or perirectal tissues (T3), or has progressed to invade other organs or structures, and/or has perforated the visceral peritoneum (T4). Stage III is characterized by any of the T substages, no distant metastasis, and either spread to 1 to 3 regional lymph nodes (N1) or spread to four or more regional lymph nodes (N2). Lastly, stage IV involves any of the T or N substages, as well as distant metastasis (M1a or M1b). Physicians will also assign a grade, that is, characterize CRC based on the appearance of the cells ranging from G1 (well-differentiated, almost normal) to G4 (undifferentiated, very abnormal) where a high grade is an indication of a poor prognosis.

### 3. Additional Factors

The methods as described herein can further comprise quantifying or determining additional factors to use in combination with the specific biomarker combinations discussed above (i.e., combinations of AFP, CEA, ferritin, CYFRA, CA19-9, TIMP-1, Gal3, and/or CRP). These additional factors may include additional biomarkers of CRC, analytes, age, comorbidity of disease, and/or other screening methods of CRC. These additional factors may be used in the methods disclosed herein to (1) identify and determine whether or not a subject is suffering from or at risk of suffering from high risk adenomas and/or CRC; (2) identify and determine whether or not a male or female subject is suffering from or at risk of suffering from a cancer other than CRC; (3) provide a diagnosis of or aid in diagnosing high risk adenomas and/or CRC in a subject; (4) provide or aid in providing a diagnosis, prognosis and/or risk stratification of high risk adenomas and/or CRC in a subject having or suspected of high risk adenomas and/or CRC; (5) differentiate between subjects with high risk adenomas and/or CRC and low risk adenomas and/or no cancer; (6) differentiate between subjects with CRC and no cancer; and (7) monitor the progression of CRC in a subject. These additional factors may be used to determine the total score.

### a. Additional Biomarkers of CRC

The methods as described herein can further comprise quantifying or determining an additional biomarker of CRC to use in combination with the specific biomarker combinations discussed above (i.e., combinations of AFP, CEA, ferritin, CYFRA, CA19-9, TIMP-1, Gal3, and/or CRP). In some embodiments, the method may further comprising quantifying or determining the level of at least one additional biomarker of CRC in the biological sample and comparing the level of the at least one additional biomarker of CRC to a reference level for the at least one biomarker of CRC in the biological sample. The additional biomarkers of CRC may be mS9, HbA1c, C3a, Cat X, suPAR(I), PAI-1, ENO2, or any combinations thereof. Levels higher than or equal to the reference level of at least one of mS9, HbA1c, C3a, Cat X, suPAR(I), PAI-1, ENO2, and combinations thereof, may be used in the methods of the present invention. The additional biomarker or analyte of CRC levels may be used to determine the total score.

### b. Age

The methods as described herein can further comprise determining the age of the subject. Older individuals may be at higher risk of having high risk adenomas and/or CRC. The age of the subject may be used to determine the total score. If the subject is male, the reference age may be 50, 51, 52, 53, 54, 55, 56, 57, 58, 59, 60, 61, 62, 63, 64, 65, 66, 67, 68, 69, or 70. For example, if the subject is male, the reference age may be 54. If the subject is female, the reference age may be 50, 51, 52, 53, 54, 55, 56, 57, 58, 59, 60, 61, 62, 63, 64, 65, 66, 67, 68, 69, or 70. For example, if the subject is female, the reference age may be 63.

### c. Comorbidity

The methods as described herein can further comprise determining comorbidity of disease in the subject. Comorbidity of disease with the biomarkers may be used to determine the total score. Individuals with heart disease, i.e., cardiovascular disease (CVD) may have a higher chance of having colorectal cancer. Cardiovascular disease (CVD) comorbidity may be used to determine the total score. Type 2 diabetes has been linked to colon cancer. Patients with diabetic (I or II) may be at higher risk of colonic neoplasias. Diabetes comorbidity may be used to determine the total score. Comorbidity count as a categorical variable (0, 1 or 2+) may be used to determine the total score. For example, comorbidity with diabetes I, diabetes II, cardiac ischemia, pulmonary embolism, subarachnoid hemorrhage, cerebral hemorrhage, cerebral infarct, apoplexia, thrombosis in the pre-cerebral arteries, and/or cerebral aneurisms without rupture may be used to determine the total score.

### d. Other Analytes

The methods as described herein can further comprise quantifying or determining circulating tumor cells, circulating free DNA, plasma miRNAs, plasma SNPs, nucleosomes, methyl vimentin and other methylated gene products. Assays for measuring the analytes include the Cell Death Detection^{plus}-ELISA (Roche Diagnostics) and Cologuard™ sDNA-based CRC screening test (Exact Sciences Corporation).

### e. Screening Methods

The methods as described herein can further comprise performing CRC screening methods or regimens on the subject. The subject may be screened by fecal tests and/or CRC structural screening regimens, i.e., structural examination tests. Examples of fecal tests are (i) the fecal occult blood test ("FOBT"); (ii) the fecal immunochemical test ("FIT"); and (iii) the stool DNA ("sDNA") test. Structural examination tests are (i) colonoscopy; (ii) flexible sigmoidoscopy; (iii) double-contrast barium enema ("DCBE"); (iv) CT colonography (virtual colonoscopy); and (v) capsule endoscopy. Current fecal tests suffer from issues of accuracy, precision, inter-and intra-individual variability, and compliance due to patient's being uncomfortable with sample collection. If a fecal test is positive, a patient is typically referred for a colonoscopy for a thorough examination and intervention (removal of adenomas) if necessary. The structural examination tests require both purging of a patient's bowels and pumping air into the colon to aid visualization. The results of the CRC screening methods may be used to determine the total score.

### (1) Fecal Blood Tests

Both the FOBT and FIT screen for CRC by detecting the amount of blood in the stool. The tests are based on the premise that neoplastic tissue, particularly malignant tissue, bleeds more than typical mucosa, with the amount of bleeding increasing with polyp size and cancer stage. Multiple testing is recommended because of intermittent bleeding. While fecal blood tests may detect some early stage tumors in the lower colon, they are unable to detect (i) CRC in the upper colon because any blood will be metabolized and/or (ii) smaller adenomatous polyps, thus creating false negatives. Any gastro-intestinal bleeding due to hemorrhoids, fissures, inflammatory disorders (ulcerative colitis, Crohn's disease), infectious diseases, even long distance running, will create false positives.

### (2) Fecal Occult Blood Test ("FOBT")

FOBTs are guaiac-based and measure the peroxidase activity of heme or hemoglobin. They are inexpensive and relatively easy to administer. Commercially available products are HemeOccult® II, and HemeOccult® Sensa® (Beckman-Coulter Inc., Los Angeles, Calif.). In addition to the false positives and false negatives mentioned above, certain foods with peroxidase activity (uncooked fruits and vegetables, red meat) also create false positives.

### (3) Fecal Immunochemistry Test ("FIT")

FIT is generally more accurate than FOBT. Rather than FOBT's chemical reaction to detect heme from blood, FIT uses antibodies to detect blood related proteins such as hemoglobin. Commercially available products are InSure® (Enterix Inc., a Quest Diagnostics company, Lyndhurst, N.J.); Hemoccult®-ICT (Beckman Coulter, Inc.); MonoHaem (Chemicon International, Inc., Temecula, Calif.); OC Auto Micro 80 (Polymedco, Cortland Manor. NY); and Magstream 1000/Hem SP (Fujirebio Inc. Tokyo, Japan). In addition to the issues from false positives or false negatives associated with blood in stools and/or metabolism, any metabolic denaturing or digestion of globin proteins or post-collection sample handling that denatures globin epitopes will create false negatives for the FIT.

### (4) Stool DNA ("sDNA") Test

The sDNA test measures a variety of DNA markers measured in a lab from a stool sample collected by the patient. One sDNA test, available from Exact Sciences Corp. (Madison, Wis.), measures mutations in K-ras, APC, P53 genes; BAT-26 (an MSI marker); a marker for DNA integrity; and methylation of the vimentin gene. While some guidelines recommend sDNA testing other guidelines are more conservative and do not recommend sDNA testing. A version of the sDNA test may be superior to FOBT, but it still only detected 15% of the advanced adenomas.

### (5) Colonoscopy and Sigmoidoscopy

Colonoscopy allows direct visualization of the bowel, and enables one to detect, biopsy, and remove adenomatous polyps. Colonoscopy is the "gold standard" diagnostic for colon cancer. Despite these advantages, there are downsides. In addition to the patient discomfort discussed above, colonoscopy is a relatively expensive procedure and there are risks of possible bowel perforation and hemorrhaging. Moreover, the skill and experience of doctors vary and some studies have reported missing 6-12% of large adenomas (=10 mm) and failing to detect cancer in 5% of the cases.

Flexible sigmoidoscopy, by definition, is limited to the sigmoid colon. A sigmoidscope is about 60 cm long (^{∼}2 feet). Thus, a doctor can only examine the rectum and the lower half of the colon. Sigmoidoscopy requires the same preparation and invasiveness as colonoscopy, with those drawbacks. For the portions examined, it has the advantages of the colonoscopy. However, flexible sigmoidoscopy does only half the job.

### (6) Double-Contrast Barium Enema and CT Colonography

Double-contrast barium enema ("DCBE") is also referred to as air-contrast enema. It requires the same prep as a colonoscopy to purge the patient's colon and the patient's colon is imaged using X-rays with a barium contrast agent. While it is recommended by most guidelines, DCBE suffers from patient discomfort during the prep and examination and not providing the opportunity for a biopsy or polypectomy if something suspicious is seen. Thus, if there is a positive test result, the patient will need a colonoscopy follow up. CT colonography also known as a virtual colonoscopy uses a computed tomography (CT or CAT) scan to image the rectum and colon. Though it requires a colon preparation, it is minimally invasive and gaining acceptance. Unfortunately, like the DCBE, a positive test will require a colonoscopy to investigate and intervene if necessary.

### (7) Capsule Endoscopy

Capsule endoscopy involves the ingestion of a small capsule with video cameras at each end. As it passes through the colon images are transmitted and recorded. Some studies have reported detection of 73% of the advanced adenomas and 74% of the CRC cases. The shortcomings are similar to DCBE or CT colonography because it requires similar patient preparation and positive results require a subsequent colonoscopy. In addition, insufficient battery life and inadequate imaging in periods of rapid motility are disadvantages for the current generation capsule endoscopy products.

### 4. Methods of Identifying and Determining Subjects Suffering from or at Risk of Suffering from High Risk Adenomas and/or Colorectal Cancer

In one aspect, the present disclosure is directed to identifying a subject suffering from or at risk of suffering from high risk adenomas and/or CRC by quantifying or determining the levels of the following combination of markers: CEA, CYFRA, CRP, and ferritin. A subject may be a high risk individual if the subject demonstrates unfavorable concentrations or amounts of CEA, CYFRA, CRP, and ferritin, as described herein. The method can comprise the steps of: (1) obtaining a biological sample from a subject; (2) determining the levels of CEA, CYFRA, CRP, and ferritin in the biological sample from the subject; (3) comparing the levels of CEA, CYFRA, CRP, and ferritin in the biological sample to reference levels of CEA, CYFRA, CRP, and ferritin; and (4) determining or identifying the subject suffering from or at risk of suffering from high risk adenomas and/or CRC if the levels of CEA, CYFRA, and CRP in the biological sample are greater than or unfavorable with respect to the reference levels of CEA, CYFRA, and CRP, and if the levels of ferritin in the biological sample are less than or unfavorable with respect to the reference level of ferritin. The subject is determined or identified not to be suffering from or not at risk of suffering from CRC if the levels of CEA, CYFRA, and CRP in the biological sample are less than or favorable with respect to the reference levels of CEA, CYFRA, and CRP and if the level of ferritin in the biological sample are greater than or favorable with respect to the reference level of ferritin.

A subject may be a high risk individual if the subject demonstrates an unfavorable score, as determined by unfavorable concentrations or amounts of CEA, CYFRA, CRP, and ferritin, as described herein, with other biomarkers and/or factors. The method can comprise the steps of: (1) obtaining a biological sample from a subject; (2) determining the levels of CEA, CYFRA, CRP, and ferritin in the biological sample from the subject; (3) comparing the levels of CEA, CYFRA, CRP, and ferritin in the biological sample to reference levels of CEA, CYFRA, CRP, and ferritin; (i) providing a CEA score for the subject wherein the subject gets a score of 1 if the level of CEA in the biological sample is greater than the reference level of CEA and a score of 0 if the level of CEA in the biological sample is equal or less than the reference level of CEA; (ii) providing a CYFRA score for the subject wherein the subject gets a score of 1 if the level of CYFRA in the biological sample is greater than the reference level of CYFRA and a score of 0 if the level of CYFRA in the biological sample is equal or less than the reference level of CYFRA; (iii) providing a CRP score for the subject wherein the subject gets a score of 1 if the level of CRP in the biological sample is greater than the reference level of CRP and a score of 0 if the level of CRP in the biological sample is equal or less than the reference level of CRP; and (iv) providing a ferritin score for the subject wherein the subject gets a score of 1 if the level of ferritin in the biological sample is less than the reference level of ferritin and a score of 0 if the level of ferritin in the biological sample is equal or greater than the reference level of ferritin; (4) adding the scores from step (3) to generate a total score; and (5) providing a diagnosis of a subject as suffering from or at risk of suffering from high risk adenomas and/or CRC if the total score is greater than a reference score, or a diagnosis of a subject as not suffering from or not at risk of suffering from high risk adenomas and/or CRC if the total score is equal to or less than the reference score. The reference levels for the biomarkers may be the same or different for male and female subjects. The reference score may be 0, 1, 2, 3, or 4. The reference score may the same or different for male and female subjects. The method may further include determining the age of the subject, comparing the age of the subject to a reference age, and providing an age score for the subject wherein the subject gets a score of 1 if the age of the subject is greater than or equal to the reference age and a score of 0 if the age of the subject is less than the reference age. The reference age may be the same or different for male and female subjects. The reference score may be 0, 1, 2, 3, 4, or 5. The reference score may the same or different for male and female subjects.

The method may further comprise confirming a diagnosis or risk of high risk adenomas and/or CRC by CRC structural screening regimen. The method may still further comprise the step of a CRC treatment and/or CRC monitoring regimen.

### 5. Methods of Identifying and Determining Subjects Suffering from or at Risk of Suffering from CRC

In one aspect, the present disclosure is also directed to identifying a subject suffering from or at risk of suffering from CRC by quantifying or determining the levels of the following combination of markers: CEA, CYFRA, CRP, CA19-9, and ferritin. A subject may be a high risk individual if the subject demonstrates unfavorable concentrations or amounts of CEA, CYFRA, CRP, CA19-9, and ferritin, as described herein. The method may include the steps of: (1) obtaining a biological sample from a subject; (2) determining the levels of CEA, CYFRA, CRP, CA19-9, and ferritin in the biological sample from the subject; (3) comparing the levels of CEA, CYFRA, CRP, CA19-9, and ferritin in the biological sample to reference levels of CEA, CYFRA, CRP, CA19-9, and ferritin; and (4) providing a diagnosis to a subject as suffering from or at risk of suffering from CRC if the levels of CEA, CYFRA, CRP, and CA19-9 in the biological sample are greater than or unfavorable with respect to the reference levels of CEA, CYFRA, CRP, and CA19-9, and if the levels of ferritin in the biological sample are less than or unfavorable with respect to the reference level of ferritin. A subject is determined not to be suffering from or at risk of suffering from CRC if the levels of CEA, CYFRA, CRP, and CA19-9 in the biological sample are less than or favorable with respect to the reference levels of CEA, CYFRA, CRP, and CA19-9 and if the level of ferritin in the biological sample are greater than or favorable with respect to the reference level of ferritin.

A subject may be a high risk individual if the subject demonstrates an unfavorable score, as determined by unfavorable concentrations or amounts of CEA, CYFRA, CRP, CA19-9, and ferritin, as described herein, with other biomarkers and/or factors. The method may include the steps of: (1) obtaining a biological sample from a subject; (2) determining the levels of CEA, CYFRA, CRP, CA19-9, and ferritin in the biological sample from the subject; (3) comparing the levels of CEA, CYFRA, CRP, CA19-9, and ferritin in the biological sample to reference levels of CEA, CYFRA, CRP, CA19-9, and ferritin; (4) providing a CEA score for the subject wherein the subject gets a score of 1 if the level of CEA in the biological sample is greater than the reference level of CEA and a score of 0 if the level of CEA in the biological sample is equal or less than the reference level of CEA; (5) providing a CYFRA score for the subject wherein the subject gets a score of 1 if the level of CYFRA in the biological sample is greater than the reference level of CYFRA and a score of 0 if the level of CYFRA in the biological sample is equal or less than the reference level of CYFRA; (6) providing a CRP score for the subject wherein the subject gets a score of 1 if the level of CRP in the biological sample is greater than the reference level of CRP and a score of 0 if the level of CRP in the biological sample is equal or less than the reference level of CRP; (7) providing a CA19-9 score for the subject wherein the subject gets a score of 1 if the level of CA19-9 in the biological sample is greater than the reference level of CA19-9 and a score of 0 if the level of CA19-9 in the biological sample is equal or less than the reference level of CA19-9; (8) providing a ferritin score for the subject wherein the subject gets a score of 1 if the level of ferritin in the biological sample is less than the reference level of ferritin and a score of 0 if the level of ferritin in the biological sample is equal or greater than the reference level of ferritin; (9) adding the scores from steps (4)-(8) to generate a total score; and (10) providing a diagnosis of a subject as suffering from or at risk of suffering from CRC if the total score is greater than a reference score, or a diagnosis of a subject as not suffering from or not at risk of suffering from CRC if the total score is equal to or less than the reference score. The reference levels for the biomarkers may be the same or different for male and female subjects. The reference score may be 0, 1, 2, 3, 4, or 5. The reference score may the same or different for male and female subjects.

The method may further comprise confirming a diagnosis or risk of CRC by CRC structural screening regimen. The method may still further comprise the step of a CRC treatment and/or CRC monitoring regimen.

### 6. Methods of Identifying and Determining Male Subjects Suffering from or at Risk of Suffering from Cancer Other than CRC

In one aspect, the present disclosure is also directed to determining whether a male subject is suffering from or at risk of suffering from a cancer other than colorectal cancer (CRC) cancer, wherein the male subject was confirmed not to have CRC by a colonoscopy. The method includes quantifying or determining the levels of the following combination of markers: CYFRA, CRP, TIMP-1, CA19-9, and AFP. A subject may be a high risk individual if the subject demonstrates unfavorable concentrations or amounts of CYFRA, CRP, TIMP-1, CA19-9, and AFP, as described herein. The method may include (1) obtaining a biological sample from the male subject; (2) determining the levels of Cytokeratin 19 Fragment (CYFRA), C-reactive protein (CRP), a tissue inhibitor of metalloproteinase 1 (TIMP-1), carbohydrate antigen 19-9 (CA19-9), and alpha-fetoprotein (AFP) in the biological sample from the male subject; (3) comparing the levels of CYFRA, CRP, TIMP-1, CA19-9, and AFP in the biological sample to reference levels of CYFRA, CRP, TIMP-1, CA19-9, and AFP; and (4) providing a diagnosis of the male subject as suffering from or at risk of suffering from a cancer other than CRC if the levels of CYFRA, CRP, TIMP-1, CA19-9, and AFP in the biological sample are greater than the reference levels of CYFRA, CRP, TIMP-1, CA19-9, and AFP.

A subject may be a high risk individual if the subject demonstrates an unfavorable score, as determined by unfavorable concentrations or amounts of CYFRA, CRP, TIMP-1, CA19-9, and AFP, as described herein, with other biomarkers and/or factors. The method may include obtaining a biological sample from a male subject; (1) determining the levels of Cytokeratin 19 Fragment (CYFRA), C-reactive protein (CRP), a tissue inhibitor of metalloproteinase 1 (TIMP-1), carbohydrate antigen 19-9 (CA19-9), and alpha-fetoprotein (AFP) in the biological sample from the male subject; (2) comparing the levels of CYFRA, CRP, TIMP-1, CA19-9, and AFP in the biological sample to reference levels of CYFRA, CRP, TIMP-1, CA19-9, and AFP; (3) providing a CYFRA score for the male subject wherein the male subject gets a score of 1 if the level of CYFRA in the biological sample is greater than the reference level of CYFRA and a score of 0 if the level of CYFRA in the biological sample is equal or less than the reference level of CYFRA; (4) providing a CRP score for the male subject wherein the male subject gets a score of 1 if the level of CRP in the biological sample is greater than the reference level of CRP and a score of 0 if the level of CRP in the biological sample is equal or less than the reference level of CRP; (5) providing a TIMP-1 score for the male subject wherein the male subject gets a score of 1 if the level of TIMP-1 in the biological sample is greater than the reference level of TIMP-1 and a score of 0 if the level of TIMP-1 in the biological sample is equal or less than the reference level of TIMP-1; (6) providing a CA19-9 score for the male subject wherein the male subject gets a score of 1 if the level of CA19-9 in the biological sample is less than the reference level of CA19-9 and a score of 0 if the level of CA19-9 in the biological sample is equal or greater than the reference level of CA19-9; (7) providing a AFP score for the male subject wherein the male subject gets a score of 1 if the level of AFP in the biological sample is less than the reference level of AFP and a score of 0 if the level of AFP in the biological sample is equal or greater than the reference level of AFP; (8) adding the CYFRA score, CRP score, TIMP-1 score, CA19-9 score, and AFP score to generate a total score; and (9) providing a diagnosis of a male subject suffering from or at risk of suffering from a cancer other than CRC if the total score is greater than a reference score. The reference score may be 0, 1, 2, 3, 4, or 5.

The method may still further comprise the step of a cancer treatment and/or cancer monitoring regimen.

### 7. Methods of Identifying and Determining Female Subjects Suffering from or at Risk of Suffering from Cancer Other than CRC

In one aspect, the present disclosure is also directed to determining whether a female subject is suffering from or at risk of suffering from a cancer other than colorectal cancer (CRC) cancer, wherein the female subject was confirmed not to have CRC by a colonoscopy. The method includes quantifying or determining the levels of the following combination of markers: CYFRA, CEA, and CRP. A subject may be a high risk individual if the subject demonstrates unfavorable concentrations or amounts of CYFRA, CEA, and CRP, as described herein. The method may include (1) obtaining a biological sample from the female subject; (2) determining the levels of Cytokeratin 19 Fragment (CYFRA), carcinoembryonic antigen (CEA), and C-reactive protein (CRP) in the biological sample from the female subject; (3) comparing the levels of CYFRA, CEA, and CRP in the biological sample to reference levels of CYFRA, CEA, and CRP; and (4) providing a diagnosis of the female subject as suffering from or at risk of suffering from a cancer other than CRC if the levels of CYFRA, CEA, and CRP in the biological sample are greater than the reference levels of CYFRA, CEA, and CRP.

A subject may be a high risk individual if the subject demonstrates an unfavorable score, as determined by unfavorable concentrations or amounts of CYFRA, CEA, and CRP, as described herein, with other biomarkers and/or factors. The method may include obtaining a biological sample from a female subject; (1) determining the levels of Cytokeratin 19 Fragment (CYFRA), carcinoembryonic antigen (CEA), and C-reactive protein (CRP) in the biological sample from the female subject; (2) comparing the levels of CYFRA, CEA, and CRP in the biological sample to reference levels of CYFRA, CEA, and CRP; (3) providing a CYFRA score for the female subject wherein the female subject gets a score of 1 if the level of CYFRA in the biological sample is greater than the reference level of CYFRA and a score of 0 if the level of CYFRA in the biological sample is equal or less than the reference level of CYFRA; (4) providing a CEA score for the female subject wherein the female subject gets a score of 1 if the level of CEA in the biological sample is greater than the reference level of CEA and a score of 0 if the level of CEA in the biological sample is equal or less than the reference level of CRP (5) providing a CRP score for the female subject wherein the female subject gets a score of 1 if the level of CRP in the biological sample is greater than the reference level of CRP and a score of 0 if the level of CRP in the biological sample is equal or less than the reference level of CRP; (6) adding the CYFRA score, CEA score, and CRP score to generate a total score; and (7) providing a diagnosis of a female subject suffering from or at risk of suffering from a cancer other than CRC if the total score is greater than a reference score. The reference score may be 0, 1, 2, or 3.

The method may still further comprise the step of a cancer treatment and/or cancer monitoring regimen.

### 8. Method for Diagnosis, Prognosis, and/or Risk Stratification of CRC

In one aspect, the present disclosure is also directed to providing a diagnosis, prognosis, and/or risk stratification of high risk adenomas and/or CRC, particularly CRC, in a subject having or suspected of having CRC by (1) quantifying or determining the levels of at least two of AFP, CEA, ferritin, CYFRA, CA19-9, TIMP-1, Gal3, and/or CRP in a biological sample from the subject. By measuring the levels of at least two of AFP, CEA, ferritin, CYFRA, CA19-9, TIMP-1, Gal3, and/or CRP, the method allows the early detection of CRC to be more accurately diagnosed and subsequently treated more successfully, compared to other commercially available assays.

The method includes the steps of obtaining a biological sample from a subject, determining the level of at least two of AFP, CEA, ferritin, CYFRA, CA19-9, TIMP-1, Gal3, and/or CRP in the biological sample, comparing the levels of at least two of AFP, CEA, ferritin, CYFRA, CA19-9, TIMP-1, Gal3, and/or CRP in the biological sample to levels of the respective combination of markers (*i.e.,* at least two of AFP, CEA, ferritin, CYFRA, CA19-9, TIMP-1, Gal3, and/or CRP in the biological sample is compared to a reference level of the at least two of AFP, CEA, ferritin, CYFRA, CA19-9, TIMP-1, Gal3, and/or CRP) in a subject having low risk adenomas or no cancer, or alternatively, a subject having high risk adenomas and/or CRC, and providing a diagnosis, prognosis and/or risk stratification of CRC in the subject if the levels of the at least two AFP, CEA, ferritin, CYFRA, CA19-9, TIMP-1, Gal3, and/or CRP in the biological sample are greater or unfavorable with respect to the reference levels of the at least two AFP, CEA, ferritin, CYFRA, CA19-9, TIMP-1, Gal3, and/or CRP. The method may further comprise confirming a diagnosis or risk of CRC (particularly early CRC). The method may still further comprise the step of a CRC treatment and/or CRC monitoring regimen.

### 9. Methods of Differentiating Between Subjects with High Risk Adenomas and/or CRC and Low Risk Adenomas and/or No Cancer

In one aspect, the present disclosure is directed to differentiating between subjects having or at risk of having high risk adenomas and/or CRC and subjects having low risk adenomas and/or no cancer by quantifying or determining the levels of the following combination of markers: CEA, CYFRA, CRP, and ferritin. A subject may have high risk adenomas and/or CRC if the subject demonstrates unfavorable concentrations or amounts of CEA, CYFRA, CRP, and ferritin, as described herein. The method can comprise the steps of: (1) obtaining a biological sample from a subject; (2) determining the levels of CEA, CYFRA, CRP, and ferritin in the biological sample from the subject; (3) comparing the levels of CEA, CYFRA, CRP, and ferritin in the biological sample to reference levels of CEA, CYFRA, CRP, and ferritin; and (4) determining or identifying the subject having or at risk of having high risk adenomas and/or CRC if the levels of CEA, CYFRA, and CRP in the biological sample are greater than or unfavorable with respect to the reference levels of CEA, CYFRA, and CRP, and if the levels of ferritin in the biological sample are less than or unfavorable with respect to the reference level of ferritin. The subject is determined or identified as having low risk adenomas and/or no cancer if the levels of CEA, CYFRA, and CRP in the biological sample are less than or favorable with respect to the reference levels of CEA,
CYFRA, and CRP and if the level of ferritin in the biological sample are greater than or favorable with respect to the reference level of ferritin.

A subject may have high risk adenomas and/or CRC if the subject demonstrates an unfavorable score, as determined by unfavorable concentrations or amounts of CEA, CYFRA, CRP, and ferritin, as described herein, with other biomarkers and/or factors. The method can comprise the steps of: (1) obtaining a biological sample from a subject; (2) determining the levels of CEA, CYFRA, CRP, and ferritin in the biological sample from the subject; (3) comparing the levels of CEA, CYFRA, CRP, and ferritin in the biological sample to reference levels of CEA, CYFRA, CRP, and ferritin; (i) providing a CEA score for the subject wherein the subject gets a score of 1 if the level of CEA in the biological sample is greater than the reference level of CEA and a score of 0 if the level of CEA in the biological sample is equal or less than the reference level of CEA; (ii) providing a CYFRA score for the subject wherein the subject gets a score of 1 if the level of CYFRA in the biological sample is greater than the reference level of CYFRA and a score of 0 if the level of CYFRA in the biological sample is equal or less than the reference level of CYFRA; (iii) providing a CRP score for the subject wherein the subject gets a score of 1 if the level of CRP in the biological sample is greater than the reference level of CRP and a score of 0 if the level of CRP in the biological sample is equal or less than the reference level of CRP; and (iv) providing a ferritin score for the subject wherein the subject gets a score of 1 if the level of ferritin in the biological sample is less than the reference level of ferritin and a score of 0 if the level of ferritin in the biological sample is equal or greater than the reference level of ferritin; (4) adding the scores from step (3) to generate a total score; and (5) providing a diagnosis of a subject as having or at risk of having high risk adenomas and/or CRC if the total score is greater than a reference score, or a diagnosis of a subject as having low risk adenomas and/or no cancer if the total score is equal to or less than the reference score. The reference levels for the biomarkers may be the same or different for male and female subjects. The reference score may be 0, 1, 2, 3, or 4. The reference score may the same or different for male and female subjects. The method may further include determining the age of the subject and comparing the age of the subject to a reference age and providing an age score for the subject wherein the subject gets a score of 1 if the age of the subject is greater than or equal to the reference age and a score of 0 if the age of the subject is less than the reference age. The reference age may be the same or different for male and female subjects. The reference score may be 0, 1, 2, 3, 4, or 5. The reference score may the same or different for male and female subjects.

The method may further comprise confirming a diagnosis or risk of high risk adenomas and/or CRC by CRC structural screening regimen. The method may still further comprise the step of a CRC treatment and/or CRC monitoring regimen.

### 10. Methods of Differentiating between Subjects with CRC and no cancer

In one aspect, the present disclosure is also directed to differentiating whether a subject has or is suffering from CRC or no cancer by quantifying or determining the levels of the following combination of markers: CEA, CYFRA, CRP, CA19-9, and ferritin.

The method may include the steps of: (1) obtaining a biological sample from a subject; (2) determining the levels of CEA, CYFRA, CRP, CA19-9, and ferritin in the biological sample from the subject; (3) comparing the levels of CEA, CYFRA, CRP, CA19-9, and ferritin in the biological sample to reference levels of CEA, CYFRA, CRP, CA19-9, and ferritin; and (4) providing a diagnosis to a subject as having CRC if the levels of CEA, CYFRA, CRP, and CA19-9 in the biological sample are greater than or unfavorable with respect to the reference levels of CEA, CYFRA, CRP, and CA19-9, and if the levels of ferritin in the biological sample are less than or unfavorable with respect to the reference level of ferritin. A subject is determined as having no cancer if the levels of CEA, CYFRA, CRP, and CA19-9 in the biological sample are less than or favorable with respect to the reference levels of CEA, CYFRA, CRP, and CA19-9 and if the level of ferritin in the biological sample are greater than or favorable with respect to the reference level of ferritin.

The method may include the steps of: (1) obtaining a biological sample from a subject; (2) determining the levels of CEA, CYFRA, CRP, CA19-9, and ferritin in the biological sample from the subject; (3) comparing the levels of CEA, CYFRA, CRP, CA19-9, and ferritin in the biological sample to reference levels of CEA, CYFRA, CRP, CA19-9, and ferritin; (4) providing a CEA score for the subject wherein the subject gets a score of 1 if the level of CEA in the biological sample is greater than the reference level of CEA and a score of 0 if the level of CEA in the biological sample is equal or less than the reference level of CEA; (5) providing a CYFRA score for the subject wherein the subject gets a score of 1 if the level of CYFRA in the biological sample is greater than the reference level of CYFRA and a score of 0 if the level of CYFRA in the biological sample is equal or less than the reference level of CYFRA; (6) providing a CRP score for the subject wherein the subject gets a score of 1 if the level of CRP in the biological sample is greater than the reference level of CRP and a score of 0 if the level of CRP in the biological sample is equal or less than the reference level of CRP; (7) providing a CA19-9 score or the subject wherein the subject gets a score of 1 if the level of CA19-9 in the biological sample is greater than the reference level of CA19-9 and a score of 0 if the level of CA19-9 in the biological sample is equal or less than the reference level of CA19-9; (8) providing a ferritin score for the subject wherein the subject gets a score of 1 if the level of ferritin in the biological sample is less than the reference level of ferritin and a score of 0 if the level of ferritin in the biological sample is equal or greater than the reference level of ferritin; (9) adding the scores from steps (4)-(8) to generate a total score; and (10) providing a diagnosis of a subject as having CRC if the total score is greater than a reference score, or a diagnosis of a subject as having no cancer if the total score is equal to or less than the reference score. The reference levels for the biomarkers may be the same or different for male and female subjects. The reference score may be 0, 1, 2, 3, 4, or 5. The reference score may the same or different for male and female subjects.

The method may further comprise confirming a diagnosis or risk of CRC by CRC structural screening regimen. The method may still further comprise the step of a CRC treatment and/or CRC monitoring regimen.

### 11. Methods of Differentiating between Subjects with Stage I or II CRC and no cancer

In one aspect, the present disclosure is directed to differentiating between subjects having or at risk of having Stage I or II CRC and subjects having low risk adenomas and/or no cancer by quantifying or determining the levels of the following combination of markers: CEA, CYFRA, CRP, and ferritin. A subject may have Stage I or II CRC if the subject demonstrates unfavorable concentrations or amounts of CEA, CYFRA, CRP, and ferritin, as described herein. The method can comprise the steps of: (1) obtaining a biological sample from a subject; (2) determining the levels of CEA, CYFRA, CRP, and ferritin in the biological sample from the subject; (3) comparing the levels of CEA, CYFRA, CRP, and ferritin in the biological sample to reference levels of CEA, CYFRA, CRP, and ferritin; and (4) determining or identifying the subject having or at risk of having Stage I or II CRC if the levels of CEA, CYFRA, and CRP in the biological sample are greater than or unfavorable with respect to the reference levels of CEA, CYFRA, and CRP, and if the levels of ferritin in the biological sample are less than or unfavorable with respect to the reference level of ferritin. The subject is determined or identified as having low risk adenomas and/or no cancer if the levels of CEA, CYFRA, and CRP in the biological sample are less than or favorable with respect to the reference levels of CEA, CYFRA, and CRP and if the level of ferritin in the biological sample are greater than or favorable with respect to the reference level of ferritin.

A subject may have Stage I or II CRC if the subject demonstrates an unfavorable score, as determined by unfavorable concentrations or amounts of CEA, CYFRA, CRP, and ferritin, as described herein, with other biomarkers and/or factors. The method can comprise the steps of: (1) obtaining a biological sample from a subject; (2) determining the levels of CEA, CYFRA, CRP, and ferritin in the biological sample from the subject; (3) comparing the levels of CEA, CYFRA, CRP, and ferritin in the biological sample to reference levels of CEA, CYFRA, CRP, and ferritin; (i) providing a CEA score for the subject wherein the subject gets a score of 1 if the level of CEA in the biological sample is greater than the reference level of CEA and a score of 0 if the level of CEA in the biological sample is equal or less than the reference level of CEA; (ii) providing a CYFRA score for the subject wherein the subject gets a score of 1 if the level of CYFRA in the biological sample is greater than the reference level of CYFRA and a score of 0 if the level of CYFRA in the biological sample is equal or less than the reference level of CYFRA; (iii) providing a CRP score for the subject wherein the subject gets a score of 1 if the level of CRP in the biological sample is greater than the reference level of CRP and a score of 0 if the level of CRP in the biological sample is equal or less than the reference level of CRP; and (iv) providing a ferritin score for the subject wherein the subject gets a score of 1 if the level of ferritin in the biological sample is less than the reference level of ferritin and a score of 0 if the level of ferritin in the biological sample is equal or greater than the reference level of ferritin; (4) adding the scores from step (3) to generate a total score; and (5) providing a diagnosis of a subject as having or at risk of having Stage I or II CRC if the total score is greater than a reference score, or a diagnosis of a subject as having low risk adenomas and/or no cancer if the total score is equal to or less than the reference score. The reference levels for the biomarkers may be the same or different for male and female subjects. The reference score may be 0, 1, 2, 3, or 4. The reference score may the same or different for male and female subjects. The method may further include determining the age of the subject and comparing the age of the subject to a reference age and providing an age score for the subject wherein the subject gets a score of 1 if the age of the subject is greater than or equal to the reference age and a score of 0 if the age of the subject is less than the reference age. The reference age may be the same or different for male and female subjects. The reference score may be 0, 1, 2, 3, 4, or 5. The reference score may the same or different for male and female subjects.

The method may further comprise confirming a diagnosis or risk of Stage I or II CRC by CRC structural screening regimen. The method may still further comprise the step of a CRC treatment and/or CRC monitoring regimen.

### 12. Methods of Differentiating between Subjects with Stage III or IV CRC and no cancer

In one aspect, the present disclosure is directed to differentiating between subjects having or at risk of having Stage III or IV CRC and subjects having low risk adenomas and/or no cancer by quantifying or determining the levels of the following combination of markers: CEA, CYFRA, CRP, and ferritin. A subject may have Stage III or IV CRC if the subject demonstrates unfavorable concentrations or amounts of CEA, CYFRA, CRP, and ferritin, as described herein. The method can comprise the steps of: (1) obtaining a biological sample from a subject; (2) determining the levels of CEA, CYFRA, CRP, and ferritin in the biological sample from the subject; (3) comparing the levels of CEA, CYFRA, CRP, and ferritin in the biological sample to reference levels of CEA, CYFRA, CRP, and ferritin; and (4) determining or identifying the subject having or at risk of having Stage III or IV CRC if the levels of CEA, CYFRA, and CRP in the biological sample are greater than or unfavorable with respect to the reference levels of CEA, CYFRA, and CRP, and if the levels of ferritin in the biological sample are less than or unfavorable with respect to the reference level of ferritin. The subject is determined or identified as having low risk adenomas and/or no cancer if the levels of CEA, CYFRA, and CRP in the biological sample are less than or favorable with respect to the reference levels of CEA, CYFRA, and CRP and if the level of ferritin in the biological sample are greater than or favorable with respect to the reference level of ferritin.

A subject may have Stage III or IV CRC if the subject demonstrates an unfavorable score, as determined by unfavorable concentrations or amounts of CEA, CYFRA, CRP, and ferritin, as described herein, with other biomarkers and/or factors. The method can comprise the steps of: (1) obtaining a biological sample from a subject; (2) determining the levels of CEA, CYFRA, CRP, and ferritin in the biological sample from the subject; (3) comparing the levels of CEA, CYFRA, CRP, and ferritin in the biological sample to reference levels of CEA, CYFRA, CRP, and ferritin; (i) providing a CEA score for the subject wherein the subject gets a score of 1 if the level of CEA in the biological sample is greater than the reference level of CEA and a score of 0 if the level of CEA in the biological sample is equal or less than the reference level of CEA; (ii) providing a CYFRA score for the subject wherein the subject gets a score of 1 if the level of CYFRA in the biological sample is greater than the reference level of CYFRA and a score of 0 if the level of CYFRA in the biological sample is equal or less than the reference level of CYFRA; (iii) providing a CRP score for the subject wherein the subject gets a score of 1 if the level of CRP in the biological sample is greater than the reference level of CRP and a score of 0 if the level of CRP in the biological sample is equal or less than the reference level of CRP; and (iv) providing a ferritin score for the subject wherein the subject gets a score of 1 if the level of ferritin in the biological sample is less than the reference level of ferritin and a score of 0 if the level of ferritin in the biological sample is equal or greater than the reference level of ferritin; (4) adding the scores from step (3) to generate a total score; and (5) providing a diagnosis of a subject as having or at risk of having Stage III or IV CRC if the total score is greater than a reference score, or a diagnosis of a subject as having low risk adenomas and/or no cancer if the total score is equal to or less than the reference score. The reference levels for the biomarkers may be the same or different for male and female subjects. The reference score may be 0, 1, 2, 3, or 4. The reference score may the same or different for male and female subjects. The method may further include determining the age of the subject and comparing the age of the subject to a reference age and providing an age score for the subject wherein the subject gets a score of 1 if the age of the subject is greater than or equal to the reference age and a score of 0 if the age of the subject is less than the reference age. The reference age may be the same or different for male and female subjects. The reference score may be 0, 1, 2, 3, 4, or 5. The reference score may the same or different for male and female subjects.

The method may further comprise confirming a diagnosis or risk of Stage III or IV CRC by CRC structural screening regimen. The method may still further comprise the step of a CRC treatment and/or CRC monitoring regimen.

### 13. Methods of Differentiating between Subjects with Non-CRC Cancer and no cancer

In one aspect, the present disclosure is directed to differentiating between subjects having or at risk of having Non-CRC and subjects having low risk adenomas and/or no cancer by quantifying or determining the levels of the following combination of markers: CEA, CYFRA, CRP, and ferritin. A subject may have Non-CRC if the subject demonstrates unfavorable concentrations or amounts of CEA, CYFRA, CRP, and ferritin, as described herein. The method can comprise the steps of: (1) obtaining a biological sample from a subject; (2) determining the levels of CEA, CYFRA, CRP, and ferritin in the biological sample from the subject; (3) comparing the levels of CEA, CYFRA, CRP, and ferritin in the biological sample to reference levels of CEA, CYFRA, CRP, and ferritin; and (4) determining or identifying the subject having or at risk of having Non-CRC if the levels of CEA, CYFRA, and CRP in the biological sample are greater than or unfavorable with respect to the reference levels of CEA, CYFRA, and CRP, and if the levels of ferritin in the biological sample are less than or unfavorable with respect to the reference level of ferritin.
The subject is determined or identified as having low risk adenomas and/or no cancer if the levels of CEA, CYFRA, and CRP in the biological sample are less than or favorable with respect to the reference levels of CEA, CYFRA, and CRP and if the level of ferritin in the biological sample are greater than or favorable with respect to the reference level of ferritin.

A subject may have Non-CRC if the subject demonstrates an unfavorable score, as determined by unfavorable concentrations or amounts of CEA, CYFRA, CRP, and ferritin, as described herein, with other biomarkers and/or factors. The method can comprise the steps of: (1) obtaining a biological sample from a subject; (2) determining the levels of CEA, CYFRA, CRP, and ferritin in the biological sample from the subject; (3) comparing the levels of CEA, CYFRA, CRP, and ferritin in the biological sample to reference levels of CEA, CYFRA, CRP, and ferritin; (i) providing a CEA score for the subject wherein the subject gets a score of 1 if the level of CEA in the biological sample is greater than the reference level of CEA and a score of 0 if the level of CEA in the biological sample is equal or less than the reference level of CEA; (ii) providing a CYFRA score for the subject wherein the subject gets a score of 1 if the level of CYFRA in the biological sample is greater than the reference level of CYFRA and a score of 0 if the level of CYFRA in the biological sample is equal or less than the reference level of CYFRA; (iii) providing a CRP score for the subject wherein the subject gets a score of 1 if the level of CRP in the biological sample is greater than the reference level of CRP and a score of 0 if the level of CRP in the biological sample is equal or less than the reference level of CRP; and (iv) providing a ferritin score for the subject wherein the subject gets a score of 1 if the level of ferritin in the biological sample is less than the reference level of ferritin and a score of 0 if the level of ferritin in the biological sample is equal or greater than the reference level of ferritin; (4) adding the scores from step (3) to generate a total score; and (5) providing a diagnosis of a subject as having or at risk of having Non-CRC if the total score is greater than a reference score, or a diagnosis of a subject as having low risk adenomas and/or no cancer if the total score is equal to or less than the reference score. The reference levels for the biomarkers may be the same or different for male and female subjects. The reference score may be 0, 1, 2, 3, or 4. The reference score may the same or different for male and female subjects. The method may further include determining the age of the subject and comparing the age of the subject to a reference age and providing an age score for the subject wherein the subject gets a score of 1 if the age of the subject is greater than or equal to the reference age and a score of 0 if the age of the subject is less than the reference age. The reference age may be the same or different for male and female subjects. The reference score may be 0, 1, 2, 3, 4, or 5. The reference score may the same or different for male and female subjects.

The method may further comprise confirming a diagnosis or risk of Non-CRC by CRC structural screening regimen. The method may still further comprise the step of a CRC treatment and/or CRC monitoring regimen.

### 14. Methods of Monitoring the Progression of CRC in a Subject

The methods described herein also can be used to monitor the progression of CRC, particularly early CRC, in a subject by determining the levels of AFP, CEA, ferritin, CYFRA, CA19-9, TIMP-1, Gal3, and/or CRP in a subject. The method may include the steps of (a) determining the concentrations or amounts of AFP, CEA, ferritin, CYFRA, CA19-9, TIMP-1, Gal3, and/or CRP in a test sample from a subject, (b) determining the concentrations or amounts of AFP, CEA, ferritin, CYFRA, CA19-9, TIMP-1, Gal3, and/or CRP in a later test sample from a subject, and (c) comparing the concentrations or amounts of AFP, CEA, ferritin, CYFRA, CA19-9, TIMP-1, Gal3, and/or CRP as determined in step (b) with the concentrations or amounts of AFP, CEA, ferritin, CYFRA, CA19-9, TIMP-1, Gal3, and/or CRP determined in step (a), wherein if the concentrations or amounts determined in step (b) is greater than, unchanged or is unfavorable when compared to the concentrations or amounts of AFP, CEA, ferritin, CYFRA, CA19-9, TIMP-1, Gal3, and/or CRP determined in step (a), then the disease in the subject is determined to have continued, progressed or worsened. By comparison, if the concentrations or amounts of AFP, CEA, ferritin, CYFRA, CA19-9, TIMP-1, Gal3, and/or CRP as determined in step (b) is lower or favorable when compared to the concentrations or amounts of AFP, CEA, ferritin, CYFRA, CA19-9, TIMP-1, Gal3, and/or CRP as determined in step (a), then the CRC in the subject is determined to have discontinued, regressed or improved. In some embodiments, a biomarker score is generated for each biomarker based on the difference between the level of the biomarker in the sample compared to a reference level of the biomarker and a total score is generated from these biomarker scores. The total score is compared to a reference score and the diagnosis is based on the this comparison.

Optionally, the method further comprises comparing the concentrations or amounts of AFP, CEA, ferritin, CYFRA, CA19-9, TIMP-1, Gal3, and/or CRP as determined in step (b), for example, with predetermined levels. Further, optionally the method comprises treating the subject with one or more treatment regimens (namely, surgery, chemotherapy, radiotherapeutic therapy, radiotherapeutic treatments, target therapy or any combination thereof) for a period of time if the comparison shows that the concentrations or amounts of AFP, CEA, ferritin, CYFRA, CA19-9, TIMP-1, Gal3, and/or CRP as determined in step (b), for example, are greater than or unfavorably altered with respect to the predetermined levels.

Still further, the methods can be used to monitor a subject receiving treatment with one or more of surgery, chemotherapy, radiotherapeutic therapy, radiotherapeutic treatments, target therapy or any combination thereof (collectively "treatment regimens"). Specifically, such methods involve providing a first test sample from a subject before the subject has been administered one or more treatment regimens. Next, the concentrations or amounts in a first test sample from a subject of AFP, CEA, ferritin, CYFRA, CA19-9, TIMP-1, Gal3, and/or CRP are determined (e.g., using methods known in the art). After the concentrations or amounts of AFP, CEA, ferritin, CYFRA, CA19-9, TIMP-1, Gal3, and/or CRP are determined, optionally the concentrations or amounts of AFP, CEA, ferritin, CYFRA, CA19-9, TIMP-1, Gal3, and/or CRP are then compared with predetermined levels. If the concentrations or amounts of AFP, CEA, ferritin, CYFRA, CA19-9, TIMP-1, Gal3, and/or CRP as determined in the first test sample are lower or favorably with respect to the predetermined levels, then the subject is not treated with one or more treatment regimens or, alternatively, the subject may be treated with one or more treatment regimens. If the concentrations or amounts of AFP, CEA, ferritin, CYFRA, CA19-9, TIMP-1, Gal3, and/or CRP as determined in the first test sample are higher or unfavorably with respect to the predetermined levels, then the subject is treated with one or more treatment regimens for a period of time or alternatively, the subject is not treated with one or more treatment regimens. The period of time that the subject is treated with the one or more treatment regimens can be determined by one skilled in the art (for example, the period of time can be from about seven (7) days to about two years, preferably from about fourteen (14) days to about one (1) year).

During the course of treatment with the one or more treatment regimens, second and subsequent test samples are then obtained from the subject. The number of test samples and the time in which said test samples are obtained from the subject are not critical. For example, a second test sample could be obtained seven (7) days after the subject is first administered the one or more treatment regimens, a third test sample could be obtained two (2) weeks after the subject is first administered the one or more treatment regimens, a fourth test sample could be obtained three (3) weeks after the subject is first administered the one or more treatment regimens, a fifth test sample could be obtained four (4) weeks after the subject is first administered the one or more treatment regimens, etc.

After each second or subsequent test sample is obtained from the subject, the concentrations or amounts AFP, CEA, ferritin, CYFRA, CA19-9, TIMP-1, Gal3, and/or CRP are determined in the second or subsequent test sample (e.g., using methods known in the art). The concentrations or amounts of AFP, CEA, ferritin, CYFRA, CA19-9, TIMP-1, Gal3, and/or CRP as determined in each of the second and subsequent test samples are then compared with the concentrations or amounts of AFP, CEA, ferritin, CYFRA, CA19-9, TIMP-1, Gal3, and/or CRP as determined in the first test sample (e.g., the test sample that was originally optionally compared to the predetermined level). If the concentrations or amounts of AFP, CEA, ferritin, CYFRA, CA19-9, TIMP-1, Gal3, and/or CRP as determined in step (c) are lower than or favorable when compared to the concentrations or amounts of AFP, CEA, ferritin, CYFRA, CA19-9, TIMP-1, Gal3, and/or CRP as determined in step (a), then the disease in the subject is determined to have discontinued, regressed, or improved, and the subject should continue to be administered the one or treatment regimens of step (b). However, if the concentrations or amounts determined in step (c) are greater than, unchanged or are unfavorable when compared to the concentrations or amounts of AFP, CEA, ferritin, CYFRA, CA19-9, TIMP-1, Gal3, and/or CRP as determined in step (a), then the disease in the subject is determined to have continued, progressed or worsened, and the subject should be treated with a higher concentration of the one or more treatment regimens administered to the subject in step (b) or the subject should be treated with one or more treatment regimens that are different from the one or more treatment regimens administered to the subject in step (b). Specifically, the subject can be treated with one or more treatment regimens that are different from the one or more treatment regimens that the subject had previously received to decrease or lower said subject's AFP, CEA, ferritin, CYFRA, CA19-9, TIMP-1, Gal3, and/or CRP levels.

Furthermore, the above assays can be performed using a first test sample obtained from a subject where the first test sample is obtained from one source, such as urine, serum, or plasma. Optionally the above assays can then be repeated using a second test sample obtained from the subject where the second test sample is obtained from another source. For example, if the first test sample was obtained from urine, the second test sample can be obtained from serum or plasma. The results obtained from the assays using the first test sample and the second test sample can be compared. The comparison can be used to assess the status of a disease or condition in the subject.

### 15. Reference Levels

The methods described herein use reference levels of AFP, CEA, ferritin, CYFRA, CA19-9, TIMP-1, Gal3, and/or CRP of a subject to (1) identify and determine whether or not a subject is suffering from or at risk of suffering from high risk adenomas and/or CRC; (2) identify and determine whether or not a male or female subject is suffering from or at risk of suffering from a cancer other than CRC; (3) provide a diagnosis of high risk adenomas and/or CRC in a subject; (4) provide or aid in providing a diagnosis, prognosis and/or risk stratification of high risk adenomas and/or CRC in a subject having or suspected of high risk adenomas and/or CRC; (5) differentiate between subjects with high risk adenomas and/or CRC and low risk adenomas and/or no cancer; (6) differentiate between subjects with CRC and no cancer; and (7) monitor the progression of CRC in a subject. Levels higher than or equal to the reference levels of AFP, CEA, CYFRA, CA19-9, TIMP-1, Gal3, and/or CRP, and/or levels lower than or equal to the reference levels of ferritin, identify the patient as having high risk adenomas and/or CRC or at risk of having or developing high risk adenomas and/or CRC. Levels lower than the reference level of AFP, CEA, CYFRA, CA19-9, TIMP-1, Gal3, and/or CRP, and/or levels higher than the reference level of ferritin identify the patient as having low risk adenomas and/or no cancer. Levels higher than or equal to the reference levels of AFP, CEA, CYFRA, CA19-9, TIMP-1, Gal3, and/or CRP, and/or levels lower than or equal to the reference levels of ferritin may also identify the patient as having a cancer other than CRC or at risk of having or developing a cancer other than CRC. Levels lower than the reference level of AFP, CEA, CYFRA, CA19-9, TIMP-1, Gal3, and/or CRP, and/or levels higher than the reference level of ferritin may also identify the patient as having no cancer.

### a. Reference levels for markers AFP, CEA, ferritin, CYFRA, CA19-9, TIMP-1, Gal3, and/or CRP

Generally, predetermined or reference levels can be employed as a benchmark against which to assess results obtained upon assaying a test sample for AFP, CEA, ferritin, CYFRA, CA19-9, TIMP-1, Gal3, and/or CRP. Generally, in making such a comparison, the predetermined levels are obtained by running a particular assay a sufficient number of times and under appropriate conditions such that a linkage or association of the analyte present, amount or concentration with a particular stage or endpoint of CRC with particular indicia can be made. Typically, the predetermined levels are obtained with assays of reference subjects (or populations of subjects). The reference subject may be a control subject or a cancer subject. The reference population or reference group may be a control group or a cancer group. The AFP, CEA, ferritin, CYFRA, CA19-9, TIMP-1, Gal3, and/or CRP measured can include AFP, CEA, ferritin, CYFRA, CA19-9, TIMP-1, Gal3, and/or CRP fragments thereof, degradation products thereof, and/or enzymatic cleavage products thereof.

In particular, with respect to predetermined levels as employed for monitoring disease progression and/or treatment, the amount or concentration of AFP, CEA, ferritin, CYFRA, CA19-9, TIMP-1, Gal3, and/or CRP or AFP, CEA, ferritin, CYFRA, CA19-9, TIMP-1, Gal3, and/or CRP fragments may be "unchanged," "favorable" (or "favorably altered"), or "unfavorable" (or "unfavorably altered"). "Elevated" or "increased" refers to an amount or a concentration in a test sample that is higher or greater than a typical or normal level or range (e.g., predetermined level), such as a typical or normal level found in a control group or cancer group, or is higher or greater than another reference level or range (e.g., earlier or baseline sample). The term "lowered" or "reduced" refers to an amount or a concentration in a test sample that is lower or less than a typical or normal level or range (e.g., predetermined level), such as a typical or normal level found in a control group or cancer group, or is lower or less than another reference level or range (e.g., earlier or baseline sample). The term "altered" refers to an amount or a concentration in a sample that is altered (increased or decreased) over a typical or normal level or range (e.g., predetermined level), such as a typical or normal level found in a control group or cancer group, or over another reference level or range (e.g., earlier or baseline sample).

The typical or normal levels or ranges for AFP, CEA, ferritin, CYFRA, CA19-9, TIMP-1, Gal3, and/or CRP are defined in accordance with standard practice. A so-called altered level or alteration can be considered to have occurred when there is any net change as compared to the typical or normal level or range, or reference level or range that cannot be explained by experimental error or sample variation. In some embodiments, the level measured in a particular sample will be compared with the level or range of levels determined in similar samples from a so-called normal subject, i.e., control subject. In this context, a "normal" (sometimes termed "control" or "healthy") subject is an individual with no detectable cancer, and a "normal" patient or population is/are one(s) that exhibit(s) no detectable cancer, for example. An "apparently normal subject" is one in which AFP, CEA, ferritin, CYFRA, CA19-9, TIMP-1, Gal3, and/or CRP has not been or is being assessed. The level of an analyte is said to be "elevated" when the analyte is normally undetectable (e.g., the normal level is zero, or within a range of from about 25 to about 75 percentiles of normal populations), but is detected in a test sample, as well as when the analyte is present in the test sample at a higher than normal level. In some embodiments, the level measured in a particular sample will be compared with the level or range of levels determined in similar samples from a cancer subject.

For example, in one aspect, reference levels higher (or greater) than or equal to 3.5 ng/mL, 4.0 ng/mL, 4.3 ng/mL, 4.4 ng/mL, 4.5 ng/mL, 4.6 ng/mL, 4.7 ng/mL, 4.8 ng/mL, 4.9 ng/mL, 5.0 ng/mL, 5.1 ng/mL, 5.2 ng/mL, 5.3 ng/mL, 5.0 ng/mL, 5.5 ng/mL, 5.6 ng/mL, 5.7 ng/mL, 5.8 ng/mL, 5.9 ng/mL, 6.0 ng/mL, 6.1 ng/mL, 6.2 ng/mL, 6.3 ng/mL, 6.4 ng/mL, 6.5 ng/mL, 6.6 ng/mL, 6.7 ng/mL, 6.8 ng/mL, 6.9 ng/mL, 7.0 ng/mL, 7.1 ng/mL, 7.2 ng/mL, 7.3 ng/mL, 7.4 ng/mL, 7.5 ng/mL, 7.6 ng/mL, 7.7 ng/mL, 7.8 ng/mL, 7.9 ng/mL, or 8.0 ng/mL in serum for CEA, reference levels higher than or equal to 1.50 ng/mL, 1.55 ng/mL, 1.60 ng/mL, 1.65 ng/mL, 1.67 ng/mL, 1.68 ng/mL, 1.69 ng/mL, 1.70 ng/mL, 1.71 ng/mL, 1.72 ng/mL, 1.73 ng/mL, 1.74 ng/mL, 1.75 ng/mL, 1.76 ng/mL, 1.77 ng/mL, 1.80 ng/mL, 1.85 ng/mL, 1.90 ng/mL, 1.95 ng/mL, 1.96 ng/mL, 1.97 ng/mL, 1.98 ng/mL, 1.99 ng/mL, 2.00 ng/mL, 2.01 ng/mL, 2.02 ng/mL, 2.03 ng/mL, 2.04 ng/mL, 2.05 ng/mL, 2.06 ng/mL, 2.07 ng/mL, 2.08 ng/mL, 2.09 ng/mL, 2.10 ng/mL, 2.11 ng/mL, 2.12 ng/mL, 2.13 ng/mL, 2.14 ng/mL, 2.15 ng/mL, 2.16 ng/mL, 2.17 ng/mL, 2.18 ng/mL, 2.19 ng/mL, 2.20 ng/mL, 2.21 ng/mL, 2.22 ng/mL, 2.23 ng/mL, 2.24 ng/mL, 2.25 ng/mL, 2.50 ng/mL, or 3.00 ng/mL in serum for CYFRA, reference levels higher than or equal to 1.0 mg/mL, 1.5 mg/mL, 1.6 mg/mL, 1.7 mg/mL, 1.8 mg/mL, 1.9 mg/mL, 2.0 mg/mL, 2.5 mg/mL, 4.0 mg/mL, 4.5 mg/mL, 5.0 mg/mL, 5.1 mg/mL, 5.2 mg/mL, 5.3 mg/mL, 5.4 mg/mL, 5.5 mg/mL, 5.6 mg/mL, 5.7 mg/mL, 5.8 mg/mL, 5.9 mg/mL, 6.0 mg/mL, 6.5 mg/mL, 6.6 mg/mL, 6.7 mg/mL, 6.8 mg/mL, 6.9 mg/mL, 7.0 mg/mL, 7.1 mg/mL, 7.2 mg/mL, 7.3 mg/mL, 7.4 mg/mL, 7.5 mg/mL, 7.6 mg/mL, 7.7 mg/mL, 7.8 mg/mL, 7.9 mg/mL, 8.0 mg/mL, 8.1 mg/mL, 8.2 mg/mL, 8.3 mg/mL, 8.4 mg/mL, 8.5 mg/mL, 8.6 mg/mL, 8.7 mg/mL, 8.8 mg/mL, 8.9 mg/mL, 9.0 mg/mL, 9.5 mg/mL, 10.0 mg/mL, 15.0 mg/mL, 17.0 mg/mL, 17.1 mg/mL, 17.2 mg/mL, 17.3 mg/mL, 17.4 mg/mL, 17.5 mg/mL, 17.6 mg/mL, 17.7 mg/mL, 17.8 mg/mL, 17.9 mg/mL, 18.0 mg/mL, 18.1 mg/mL, 18.2 mg/mL, 18.3 mg/mL, 18.4 mg/mL, 18.5 mg/mL, 18.6 mg/mL, 18.7 mg/mL, 18.8 mg/mL, 18.9 mg/mL 19.0 mg/mL, 19.5 mg/mL, or 20.0 mg/mL in serum for CRP, reference levels lower than or equal to 120.0 ng/mL, 115.0 ng/mL, 114.0 ng/mL, 113.0 ng/mL, 112.0 ng/mL, 111.0 ng/mL, 110.0 ng/mL, 109.0 ng/mL, 108.0 ng/mL, 107.0 ng/mL, 106.0 ng/mL, 105.0 ng/mL, 104.0 ng/mL, 103.0 ng/mL, 102.0 ng/mL, 101.0 ng/mL, 100.0 ng/mL, 99.0 ng/mL, 98.0 ng/mL, 97.0 ng/mL, 96.0 ng/mL, 95.0 ng/mL, 94.0 ng/mL, 93.0 ng/mL, 92.0 ng/mL, 91.0 ng/mL, 90.0 ng/mL, 89.0 ng/mL, 88.0 ng/mL, 87.0 ng/mL, 86.0 ng/mL, 85.0 ng/mL, 80.0 ng/mL, 75.0 ng/mL, 70.0 ng/mL, 65.0 ng/mL, 60.0 ng/mL, 55.0 ng/mL, 50.0 ng/mL, 45.0 ng/mL, 44.0 ng/mL, 43.0 ng/mL, 42.0 ng/mL, 41.0 ng/mL, 40.0 ng/mL, 39.0 ng/mL, 38.0 ng/mL, 37.0 ng/mL, 36.0 ng/mL, 35.0 ng/mL, 34.0 ng/mL, 33.0 ng/mL, 32.0 ng/mL, 31.0 ng/mL, 30.0 ng/mL, or 25.0 ng/mL in serum for ferritin, reference levels higher than or equal to 23.0 U/mL, 23.1 U/mL, 23.2 U/mL, 23.3 U/mL, 23.4 U/mL, 23.5 U/mL, 23.6 U/mL, 23.7 U/mL, 23.8 U/mL, 23.9 U/mL, 24.0 U/mL, 24.1 U/mL, 24.2 U/mL, 24.3 U/mL, 24.4 U/mL, 24.5 U/mL, 24.6 U/mL, 24.7 U/mL, 24.8 U/mL, 24.9 U/mL, 25.0 U/mL, 25.1 U/mL, 25.2 U/mL, 25.3 U/mL, 25.4 U/mL, 25.5 U/mL, 25.6 U/mL, 25.7 U/mL, 25.8 U/mL, 25.9 U/mL, 26.0 U/mL, 26.1 U/mL, 26.2 U/mL, 26.3 U/mL, 26.4 U/mL, 26.5 U/mL, 26.6 U/mL, 26.7 U/mL, 26.8 U/mL, 26.9 U/mL, 27.0 U/mL, 27.1 U/mL, 27.2 U/mL, 27.3 U/mL, 27.4 U/mL, 27.5 U/mL, 27.6 U/mL, 27.7 U/mL, 27.8 U/mL, 27.9 U/mL, 28.0 U/mL, 28.5 U/mL, or 29.0 U/mL in serum for CA19-9, or combinations thereof, identify the subject as having high risk adenomas and/or CRC. In another aspect, reference levels higher (or greater) than or equal to 3.5 to 8.0 ng/mL in serum for CEA, reference levels higher than or equal to 1.50 to 3.00 ng/mL in serum for CYFRA, reference levels higher than or equal to 1.0 to 20.0 mg/mL in serum for CRP, reference levels lower than or equal to 120.0 to 25.0 ng/mL in serum for ferritin, reference levels higher than or equal to 23.0 to 29.0 U/mL in serum for CA19-9, or combinations thereof, identify the subject as having high risk adenomas and/or CRC.

For example, in one aspect, reference levels higher than or equal to 1.0 ng/mL, 1.1 ng/mL, 1.2 ng/mL, 1.3 ng/mL, 1.4 ng/mL, 1.5 ng/mL, 1.6 ng/mL, 1.7 ng/mL, 1.8 ng/mL, 1.9 ng/mL, 2.0 ng/mL, 2.1 ng/mL, 2.2 ng/mL, 2.3 ng/mL, 2.4 ng/mL, 2.5 ng/mL, 3.0 ng/mL, or 3.5 ng/mL in serum for CYFRA, reference levels higher than or equal to 9.0 mg/mL, 9.1 mg/mL, 9.2 mg/mL, 9.3 mg/mL, 9.4 mg/mL, 9.5 mg/mL, 9.6 mg/mL, 9.7 mg/mL, 9.8 mg/mL, 9.9 mg/mL, 10.0 mg/mL, 10.1 mg/mL, 10.2 mg/mL, 10.3 mg/mL, 10.4 mg/mL, 10.5 mg/mL, 10.6 mg/mL, 10.7 mg/mL, 10.8 mg/mL, 10.9 mg/mL, 11.0 mg/mL, 11.5 mg/mL, 12.0 mg/mL, 14.0 mg/mL, 14.1 mg/mL, 14.2 mg/mL, 14.3 mg/mL, 14.4 mg/mL, 14.5 mg/mL, 14.6 mg/mL, 14.7 mg/mL, 14.8 mg/mL, 14.9 mg/mL, 15.0 mg/mL, 15.1 mg/mL, 15.2 mg/mL, 15.3 mg/mL, 15.4 mg/mL, 15.5 mg/mL, 15.6 mg/mL, 15.7 mg/mL, 15.8 mg/mL, 15.9 mg/mL, 16.0 mg/mL, 16.1 mg/mL, 16.2 mg/mL, 16.3 mg/mL, 16.4 mg/mL, 16.5 mg/mL, 17.0 mg/mL, or 17.5 mg/mL in serum for CRP, reference levels higher than or equal to 140 ng/mL, 141 ng/mL, 142 ng/mL, 143 ng/mL, 144 ng/mL, 145 ng/mL, 146 ng/mL, 147 ng/mL, 148 ng/mL, 149 ng/mL, 150 ng/mL, 151 ng/mL, 152 ng/mL, 153 ng/mL, 154 ng/mL, 155 ng/mL, 156 ng/mL, 157 ng/mL, 158 ng/mL, 159 ng/mL, or 160 ng/mL in serum for TIMP-1, reference levels higher than or equal to 24.0 U/mL, 24.1 U/mL, 24.2 U/mL, 24.3 U/mL, 24.4 U/mL, 24.5 U/mL, 24.6 U/mL, 24.7 U/mL, 24.8 U/mL, 24.9 U/mL, 25.0 U/mL, 25.1 U/mL, 25.2 U/mL, 25.3 U/mL, 25.4 U/mL, 25.5 U/mL, 25.6 U/mL, 25.7 U/mL, 25.8 U/mL, 25.9 U/mL, 26.0 U/mL, or 26.5 U/mL in serum for CA19-9, reference levels higher than or equal to 5.0 ng/mL, 5.1 ng/mL, 5.2 ng/mL, 5.3 ng/mL, 5.4 ng/mL, 5.5 ng/mL, 5.6 ng/mL, 5.7 ng/mL, 5.8 ng/mL, 5.9 ng/mL, 6.0 ng/mL, 6.1 ng/mL, 6.2 ng/mL, 6.3 ng/mL, 6.4 ng/mL, 6.5 ng/mL, 6.6 ng/mL, 6.7 ng/mL, 6.8 ng/mL, 6.9 ng/mL, 7.0 ng/mL, 7.5 ng/mL, or 8.0 ng/mL in serum for AFP, reference levels higher than or equal to 4.0 ng/mL, 4.1 ng/mL, 4.2 ng/mL, 4.3 ng/mL, 4.4 ng/mL, 4.5 ng/mL, 4.6 ng/mL, 4.7 ng/mL, 4.8 ng/mL, 4.9 ng/mL, 5.0 ng/mL, 5.1 ng/mL, 5.2 ng/mL, 5.3 ng/mL, 5.4 ng/mL, 5.5 ng/mL, 5.6 ng/mL, 5.7 ng/mL, 5.8 ng/mL, 5.9 ng/mL, 6.0 ng/mL, 6.1 ng/mL, 6.2 ng/mL, 6.3 ng/mL, 6.4 ng/mL, 6.5 ng/mL, 6.6 ng/mL, 6.7 ng/mL, 6.8 ng/mL, 6.9 ng/mL, 7.0 ng/mL, or 7.5 ng/mL in serum for CEA, or combinations thereof, identify the subject as having a cancer other than CRC. In another aspect, reference levels higher than or equal to 1.0 to 3.5 ng/mL in serum for CYFRA, reference levels higher than or equal to 9.0 to 17.5 mg/mL in serum for CRP, reference levels higher than or equal to 140 to 160 ng/mL in serum for TIMP-1, reference levels higher than or equal to 24.0 to 26.5 U/mL in serum for CA19-9, reference levels higher than or equal to 5.0 to 8.0 ng/mL in serum for AFP, reference levels higher than or equal to 4.0 to 7.5 ng/mL in serum for CEA, or combinations thereof, identify the subject as having a cancer other than CRC.

Cutoff values (or predetermined cutoff values) may be determined by Adaptive Index Model (AIM) methodology. Cutoff values (or predetermined cutoff values) may be determined by a receiver operating curve (ROC) analysis from biological samples of the patient group. ROC analysis, as generally known in the biological arts, is a determination of the ability of a test to discriminate one condition from another, e.g., to determine the performance of each marker in identifying a patient having CRC. A description of ROC analysis as applied according to the present disclosure is provided in P.J. Heagerty et al., Time-dependent ROC curves for censored survival data and a diagnostic marker, *Biometrics* 56:337-44(2000), the disclosure of which is hereby incorporated by reference in its entirety. Alternatively, cutoff values can be determined by a quartile analysis of biological samples of a patient group. For example, a cutoff value can be determined by selecting a value that corresponds to any value in the 25th-75th percentile range, preferably a value that corresponds to the 25th percentile, the 50th percentile or the 75th percentile, and more preferably the 75th percentile.

Such statistical analyses can be performed using any method known in the art and can be implemented through any number of commercially available software packages (e.g., from Analyse-it Software Ltd., Leeds, UK; StataCorp LP, College Station, TX; SAS Institute Inc., Cary, NC.).

### b. Reference levels for additional biomarkers

Generally, predetermined or reference levels can be employed as a benchmark against which to assess results obtained upon assaying a test sample for mS9, HbA1c, C3a, Cat X, suPAR(I), PAI-1, ENO2, or any combinations thereof. Generally, in making such a comparison, the predetermined levels are obtained by running a particular assay a sufficient number of times and under appropriate conditions such that a linkage or association of the analyte present, amount or concentration with a particular stage or endpoint of CRC with particular indicia can be made. Typically, the predetermined levels are obtained with assays of reference subjects (or populations of subjects). The reference subject may be a control subject or a cancer subject. The reference population or reference group may be a control group or a cancer group. The mS9, HbA1c, C3a, Cat X, suPAR(I), PAI-1, and/or ENO2 measured can include mS9, HbA1c, C3a, Cat X, suPAR(I), PAI-1, and/or ENO2 fragments thereof, degradation products thereof, and/or enzymatic cleavage products thereof.

In particular, with respect to predetermined levels as employed for monitoring disease progression and/or treatment, the amount or concentration of mS9, HbA1c, C3a, Cat X, suPAR(I), PAI-1, and/or ENO2 or mS9, HbA1c, C3a, Cat X, suPAR(I), PAI-1, and/or ENO2 fragments may be "unchanged," "favorable" (or "favorably altered"), or "unfavorable" (or "unfavorably altered"). "Elevated" or "increased" refers to an amount or a concentration in a test sample that is higher or greater than a typical or normal level or range (e.g., predetermined level), such as a typical or normal level found in a control group or cancer group, or is higher or greater than another reference level or range (e.g., earlier or baseline sample). The term "lowered" or "reduced" refers to an amount or a concentration in a test sample that is lower or less than a typical or normal level or range (e.g., predetermined level), such as a typical or normal level found in a control group or cancer group, or is lower or less than another reference level or range (e.g., earlier or baseline sample). The term "altered" refers to an amount or a concentration in a sample that is altered (increased or decreased) over a typical or normal level or range (e.g., predetermined level), such as a typical or normal level found in a control group or cancer group, or over another reference level or range (e.g., earlier or baseline sample).

The typical or normal levels or ranges for mS9, HbA1c, C3a, Cat X, suPAR(I), PAI-1, and/or ENO2 are defined in accordance with standard practice. A so-called altered level or alteration can be considered to have occurred when there is any net change as compared to the typical or normal level or range, or reference level or range that cannot be explained by experimental error or sample variation. Thus, the level measured in a particular sample will be compared with the level or range of levels determined in similar samples from a so-called normal subject. In this context, a "normal" (sometimes termed "control" or "healthy") subject is an individual with no detectable cancer, and a "normal" patient or population is/are one(s) that exhibit(s) no detectable cancer, for example. An "apparently normal subject" is one in which mS9, HbA1c, C3a, Cat X, suPAR(I), PAI-1, and/or ENO2 has not been or is being assessed. The level of an analyte is said to be "elevated" when the analyte is normally undetectable (e.g., the normal level is zero, or within a range of from about 25 to about 75 percentiles of normal populations), but is detected in a test sample, as well as when the analyte is present in the test sample at a higher than normal level. In some embodiments, the level measured in a particular sample will be compared with the level or range of levels determined in similar samples from a cancer subject.

Cutoff values (or predetermined cutoff values) may be determined by Adaptive Index Model (AIM) methodology. Cutoff values (or predetermined cutoff values) may be determined by a receiver operating curve (ROC) analysis from biological samples of the patient group. ROC analysis, as generally known in the biological arts, is a determination of the ability of a test to discriminate one condition from another, e.g., to determine the performance of each marker in identifying a patient having CRC. A description of ROC analysis as applied according to the present disclosure is provided in P.J. Heagerty et al., Time-dependent ROC curves for censored survival data and a diagnostic marker, Biometrics 56:337-44(2000), the disclosure of which is hereby incorporated by reference in its entirety.

Alternatively, cutoff values can be determined by a quartile analysis of biological samples of a patient group. For example, a cutoff value can be determined by selecting a value that corresponds to any value in the 25th-75th percentile range, preferably a value that corresponds to the 25th percentile, the 50th percentile or the 75th percentile, and more preferably the 75th percentile.

Such statistical analyses can be performed using any method known in the art and can be implemented through any number of commercially available software packages (e.g., from Analyse-it Software Ltd., Leeds, UK; StataCorp LP, College Station, TX; SAS Institute Inc., Cary, NC.).

### 16. Means for Confirming the CRC Status of a Subject

The subject identified in the methods described above having levels of AFP, CEA, ferritin, CYFRA, CA19-9, TIMP-1, Gal3, and/or CRP greater than the values discussed above are identified as patients suffering from high risk adenomas and/or CRC. The subject may then be administered a means for confirming the CRC status. A means for confirming CRC status may include performing a CRC screening method, such as fecal tests and/or CRC structural screening tests, as described above. The stage of the CRC may be confirmed by pathologic examination of resected colon, sigmoidoscopy, colonoscopy, and various imaging techniques, as well as proximal lymph node evaluation, sentinel node evaluation, chest/abdominal/pelvic CT, MRI scans, positron emission tomography ("PET") scans, liver functionality tests (for liver metastases), and blood tests (complete blood count ("CBC"), carcinoembryonic antigen ("CEA"), CA 19-9).

### 17. Means for Monitoring the CRC Status of a Subject

The subject identified in the methods described above having levels of AFP, CEA, ferritin, CYFRA, CA19-9, TIMP-1, Gal3, and/or CRP greater than the values discussed are identified as patients suffering from CRC or cancer other than CRC. Many patients may develop a recurrence of CRC following surgical resection, particularly in the first 2 or 3 years. Accordingly, CRC patients must be closely monitored to determine response to therapy and to detect persistent or recurrent disease and metastasis.

The subject may then be placed on a CRC or cancer monitoring regimen. Specifically, the subject may be administered a means for monitoring the effectiveness of any treatment regimens (such as surgery, radiotherapeutic therapy, radiotherapeutic treatments, chemotherapy, target therapy, or any combinations thereof) being used to treat the cancer as well as to assess the progress (or lack thereof) of the disease. The CRC or cancer monitoring regimen may involve conducting determining AFP, CEA, ferritin, CYFRA, CA19-9, TIMP-1, Gal3, and/or CRP at periodic intervals (such periodic intervals being once a week, once a month, once every two months, once every three months, once every four months, once every five months, once every six months, once every seven months, once every eight months, once every nine months, once every ten months, once every eleven months, and once a year) or any combinations thereof.

### 18. Treatment Regimens

The subject identified in the methods described above having levels of AFP, CEA, ferritin, CYFRA, CA19-9, TIMP-1, Gal3, and/or CRP greater than the values discussed are identified as patients suffering from high risk adenomas, CRC, and/or a cancer other than CRC. The subjects are then treated for high risk adenomas, CRC, and/or a cancer. Any number or variety of treatment regimens can be used. For example, such treatment regimens may include one or more of surgery, chemotherapy, radiotherapeutic therapy, radiotherapeutic treatments, target therapy or any combinations thereof.

### a. Cancer Treatment

Chemotherapy involves the use of one or more chemotherapeutic agents. The phrase "chemotherapeutic agent," as used herein, is intended to refer to any chemotherapeutic agent known to those of skill in the art to be effective for the treatment or amelioration of cancer. Chemotherapeutic agents include, but are not limited to; small molecules; synthetic drugs; peptides; polypeptides; proteins; nucleic acids (e.g., DNA and RNA nucleotides including, but not limited to, antisense nucleotide sequences, triple helices and nucleotide sequences encoding biologically active proteins, polypeptides or peptides); antibodies; synthetic or natural inorganic molecules; mimetic agents; and synthetic or natural organic molecules. Any agent which is known to be useful, or which has been used or is currently being used for the treatment or amelioration of cancer can be used in combination with an active vitamin D compound in accordance with the disclosure herein. See, e.g., Hardman et al., eds., 1996, Goodman & Gilman's The Pharmacological Basis Of Basis Of Therapeutics 9th Ed, McGraw-Hill, New York, N.Y. for information regarding therapeutic agents which have been or are currently being used for the treatment or amelioration of cancer.

Chemotherapeutic agents may include alkylating agents, antimetabolites, anti-mitotic agents, epipodophyllotoxins, antibiotics, hormones and hormone antagonists, enzymes, platinum coordination complexes, anthracenediones, substituted ureas, methylhydrazine derivatives, imidazotetrazine derivatives, cytoprotective agents, DNA topoisomerase inhibitors, biological response modifiers, retinoids, therapeutic antibodies, differentiating agents, immunomodulatory agents, and angiogenesis inhibitors.

Examples of chemotherapeutic agents that may be used include those that have been used, are currently used, or are known to be useful for the treatment or amelioration of CRC. Preferred agents include, but are not limited to, cisplatin, carboplatin, paclitaxel, docetaxel, etoposide, vincristine, vinblastine, cyclophosphamide, doxorubicin, vinorelbine, topotecan, gemcitabine, irinotecan, gifitinib, ifosfamide, tarceva, oblimersen, and TLK286.

Other chemotherapeutic agents that may be used include abarelix, aldesleukin, alemtuzumab, alitretinoin, allopurinol, altretamine, amifostine, anastrozole, arsenic trioxide, asparaginase, azacytidine, BCG live, bevaceizumab, bexarotene, bleomycin, bortezomib, busulfan, calusterone, camptothecin, capecitabine, carboplatin, carmustine, celecoxib, cetuximab, chlorambucil, cinacalcet, cisplatin, cladribine, cyclophosphamide, cytarabine, dacarbazine, dactinomycin, darbepoetin alfa, daunorubicin, denileukin diftitox, dexrazoxane, docetaxel, doxorubicin, dromostanolone, Elliott's B solution, epirubicin, epoetin alfa, estramustine, etoposide, exemestane, filgrastim, floxuridine, fludarabine, fluorouracil, fulvestrant, gemcitabine, gemtuzumab ozogamicin, gefitinib, goserelin, hydroxyurea, ibritumomab tiuxetan, idarubicin, ifosfamide, imatinib, interferon alfa-2a, interferon alfa-2b, irinotecan, letrozole, leucovorin, levamisole, lomustine, meclorethamine, megestrol, melphalan, mercaptopurine, mesna, methotrexate, methoxsalen, methylprednisolone, mitomycin C, mitotane, mitoxantrone, nandrolone, nofetumomab, oblimersen, oprelvekin, oxaliplatin, paclitaxel, pamidronate, pegademase, pegaspargase, pegfilgrastim, pemetrexed, pentostatin, pipobroman, plicamycin, polifeprosan, porfimer, procarbazine, quinacrine, rasburicase, rituximab, sargramostim, SN-38, streptozocin, talc, tamoxifen, tarceva, temozolomide, teniposide, testolactone, thioguanine, thiotepa, topotecan, toremifene, tositumomab, trastuzumab, tretinoin, uracil mustard, valrubicin, vinblastine, vincristine, vinorelbine, and zoledronate.

Chemotherapeutic agents also include anti-inflammatory drugs which are known to be useful for ameliorating inflammation. Suitable anti-inflammatory drugs include, but are not limited to, salicylates (such as aspirin, choline inagnessium trisalicylate, methyl salicylate, salsalte and diflunisal), acetic acids (such as indomethacin, sulindac, tolmetin, aceclofenac and diclofenac), 2-arylpropionic acids or profens (such as ibuprofen, ketoprofen, naproxen, fenoprofen, flurbiprofen and oxaprozin), N-arylanthranilic acids or fenamic acids (such as mefenamic acid, flufenamic acid, and meclofenamate), enolic acids or oxicams (such as piroxicam and meloxicam), cox inhibitors (such as celecoxib, rofecoxib (withdrawn from market), valdecoxib, parecoxib and etoricoxib), sulphonanilides such as nimesulide; naphthylalkanones (such as nabumetone), pyranocarboxylic acids (such as etodolac) and pyrroles (such as ketorolac).

Chemotherapeutic agents further include immunomodulatory agents. As used herein, the phrase "immunomodulatory agent" and variations thereof including, but not limited to, immunomodulatory agents, immunomodulants, immunomodulators or immunomodulatory drugs, refer to an agent that modulates a host's immune system. In particular, an immunomodulatory agent is an agent that alters the ability of a subject's immune system to respond to one or more foreign antigens. In one aspect, an immunomodulatory agent is an agent that shifts one aspect of a subject's immune response, e.g., the agent shifts the immune response from a Th1 to a Th2 response. In another aspect, an immunomodulatory agent is an agent that inhibits or reduces a subject's immune system (i.e., an immunosuppressant agent). In still certain other aspects, an immunomodulatory agent is an agent that activates or increases a subject's immune system (i.e., an immunostimulatory agent).

Immunomodulatory agents that may be used include small molecules, peptides, polypeptides, proteins, nucleic acids (e.g., DNA and RNA nucleotides including, but not limited to, antisense nucleotide sequences, triple helices and nucleotide sequences encoding biologically active proteins, polypeptides or peptides), antibodies, synthetic or natural inorganic molecules, mimetic agents, and synthetic or natural organic molecules. A particularly useful immunomodulatory agent is thalidomide.

Examples of immunosuppressant agents that may be used in the methods of the present disclosure include glucocorticoid receptor agonists (e.g., cortisone, dexamethasone, hydrocortisone, betamethasone), calcineurin inhibitors (e.g., macrolides such as tacrolimus and pimecrolimus), immunophilins (e.g., cyclosporin A) and mTOR inhibitors (e.g., sirolimus, marketed as RAPAMUNE® by Wyeth). Immunostimulant agents useful for the present disclosure include interferon and Zidovudine (AZT).

Chemotherapeutic agents may be administered at doses that are recognized by those of skill in the art to be effective for the treatment of CRC.

Radiotherapeutic therapy involves the use of one or more radiotherapeutic agents. The phrase "radiotherapeutic agent," as used herein, is intended to refer to any radiotherapeutic agent known to one of skill in the art to be effective to treat or ameliorate cancer, without limitation. For instance, the radiotherapeutic agent can be an agent such as those administered in radionuclide therapy.

Radionuclide therapy can be administered according to any schedule, dose, or method known to one of skill in the art to be effective in the treatment or amelioration of cancer, without limitation. In general, radionuclide therapy comprises systemic administration of a radioisotope that preferentially accumulates in or binds to the surface of cancerous cells. The preferential accumulation of the radionuclide can be mediated by a number of mechanisms, including, but not limited to, incorporation of the radionuclide into rapidly proliferating cells, specific accumulation of the radionuclide by the cancerous tissue without special targeting, or conjugation of the radionuclide to a biomolecule specific for a neoplasm.

Representative radioisotopes that can be administered in radionuclide therapy include, but are not limited to, phosphorus 32, yttrium 90, dysprosium 165, indium 111, strontium 89, samarium 153, rhenium 186, iodine 131, iodine 125, lutetium 177, and bismuth 213. While all of these radioisotopes may be linked to a biomolecule providing specificity of targeting, iodine 131, indium 111, phosphorus 32, samarium 153, and rhenium 186 may be administered systemically without such conjugation. One of skill in the art may select a specific biomolecule for use in targeting a particular neoplasm for radionuclide therapy based upon the cell-surface molecules present on that neoplasm. Examples of biomolecules providing specificity for particular cell are reviewed in an article by Thomas, Cancer Biother. Radiopharm. 17:71-82 (2002), which is incorporated herein by reference in its entirety. Furthermore, methods of administering and compositions useful for radionuclide therapy may be found in U.S. Patent Nos. 6,426,400, 6,358,194, 5,766,571, and 5,563,250, each of which is incorporated herein by reference in its entirety.

"Radiotherapeutic treatment," as used herein, is intended to refer to any radiotherapeutic treatment known to one of skill in the art to be effective to treat or ameliorate cancer, without limitation. For instance, the radiotherapeutic treatment can be external-beam radiation therapy, thermotherapy, radiosurgery, charged-particle radiotherapy, neutron radiotherapy, or photodynamic therapy.

External-beam radiation therapy can be administered according to any schedule, dose, or method known to one of skill in the art to be effective in the treatment or amelioration of cancer, without limitation. In general, external-beam radiation therapy comprises irradiating a defined volume within a subject with a high energy beam, thereby causing cell death within that volume. The irradiated volume preferably contains the entire cancer to be treated, and preferably contains as little healthy tissue as possible. Methods of administering and apparatuses and compositions useful for external-beam radiation therapy can be found in U.S. Patent Nos. 6,449,336, 6,398,710, 6,393,096, 6,335,961, 6,307,914, 6,256,591, 6,245,005, 6,038,283, 6,001,054, 5,802,136, 5,596,619, and 5,528,652, each of which is incorporated herein by reference in its entirety.

Thermotherapy can be administered according to any schedule, dose, or method known to one of skill in the art to be effective in the treatment or amelioration of cancer, without limitation. In certain embodiments, the thermotherapy can be cryoablation therapy. In other embodiments, the thermotherapy can be hyperthermic therapy. In still other embodiments, the thermotherapy can be a therapy that elevates the temperature of the tumor higher than in hyperthermic therapy.

Cryoablation therapy involves freezing of a neoplastic mass, leading to deposition of intra-and extra-cellular ice crystals; disruption of cellular membranes, proteins, and organelles; and induction of a hyperosmotic environment, thereby causing cell death. Methods for and apparatuses useful in cryoablation therapy are described in Murphy et al., Sem. Urol. Oncol. 19:133-140 (2001) and U.S. Patent Nos. 6,383,181, 6,383,180, 5,993,444, 5,654,279, 5,437,673, and 5,147,355, each of which is incorporated herein by reference in its entirety.

Hyperthermic therapy typically involves elevating the temperature of a neoplastic mass to a range from about 42°C to about 44°C. The temperature of the cancer may be further elevated above this range; however, such temperatures can increase injury to surrounding healthy tissue while not causing increased cell death within the tumor to be treated. The tumor may be heated in hyperthermic therapy by any means known to one of skill in the art without limitation. For example, and not by way of limitation, the tumor may be heated by microwaves, high intensity focused ultrasound, ferromagnetic thermoseeds, localized current fields, infrared radiation, wet or dry radiofrequency ablation, laser photocoagulation, laser interstitial thermic therapy, and electrocautery. Microwaves and radiowaves can be generated by waveguide applicators, horn, spiral, current sheet, and compact applicators.

Other methods of and apparatuses and compositions for raising the temperature of a tumor are reviewed in an article by Wust et al., Lancet Oncol. 3:487-97 (2002), and described in U.S. Patent Nos. 6,470,217, 6,379,347, 6,165,440, 6,163,726, 6,099,554, 6,009,351, 5,776,175, 5,707,401, 5,658,234, 5,620,479, 5,549,639, and 5,523,058, each of which is incorporated herein by reference in its entirety.

Radiosurgery can be administered according to any schedule, dose, or method known to one of skill in the art to be effective in the treatment or amelioration of cancer, without limitation. In general, radiosurgery comprises exposing a defined volume within a subject to a manually directed radioactive source, thereby causing cell death within that volume. The irradiated volume preferably contains the entire cancer to be treated, and preferably contains as little healthy tissue as possible. Typically, the tissue to be treated is first exposed using conventional surgical techniques, then the radioactive source is manually directed to that area by a surgeon. Alternatively, the radioactive source can be placed near the tissue to be irradiated using, for example, a laparoscope. Methods and apparatuses useful for radiosurgery are further described in Valentini et al., Eur. J. Surg. Oncol. 28:180-185 (2002) and in U.S. Patent Nos. 6,421,416, 6,248,056, and 5,547,454, each of which is incorporated herein by reference in its entirety.

Charged-particle radiotherapy can be administered according to any schedule, dose, or method known to one of skill in the art to be effective in the treatment or amelioration of cancer, without limitation. In certain embodiments, the charged-particle radiotherapy can be proton beam radiotherapy. In other embodiments, the charged-particle radiotherapy can be helium ion radiotherapy. In general, charged-particle radiotherapy comprises irradiating a defined volume within a subject with a charged-particle beam, thereby causing cellular death within that volume. The irradiated volume preferably contains the entire cancer to be treated, and preferably contains as little healthy tissue as possible. A method for administering charged-particle radiotherapy is described in U.S. Patent No. 5,668,371, which is incorporated herein by reference in its entirety.

Neutron radiotherapy can be administered according to any schedule, dose, or method known to one of skill in the art to be effective in the treatment or amelioration of cancer, without limitation. In certain embodiments, the neutron radiotherapy can be a neutron capture therapy. In such embodiments, a compound that emits radiation when bombarded with neutrons and preferentially accumulates in a neoplastic mass is administered to a subject. Subsequently, the tumor is irradiated with a low energy neutron beam, activating the compound and causing it to emit decay products that kill the cancerous cells. The compound to be activated can be caused to preferentially accumulate in the target tissue according to any of the methods useful for targeting of radionuclides, as described above, or in the methods described in Laramore, Semin. Oncol. 24:672-685 (1997) and in U.S. Patent Nos. 6,400,796, 5,877,165, 5,872,107, and 5,653,957, each of which is incorporated herein by reference in its entirety.

In other embodiments, the neutron radiotherapy can be a fast neutron radiotherapy. In general, fast neutron radiotherapy comprises irradiating a defined volume within a subject with a neutron beam, thereby causing cellular death within that volume.

Photodynamic therapy can be administered according to any schedule, dose, or method known to one of skill in the art to be effective in the treatment or amelioration of cancer, without limitation. In general, photodynamic therapy comprises administering a photosensitizing agent that preferentially accumulates in a neoplastic mass and sensitizes the neoplasm to light, then exposing the tumor to light of an appropriate wavelength. Upon such exposure, the photosensitizing agent catalyzes the production of a cytotoxic agent, such as, e.g., singlet oxygen, which kills the cancerous cells. Methods of administering and apparatuses and compositions useful for photodynamic therapy are disclosed in Hopper, Lancet Oncol. 1:212-219 (2000) and U.S. Patent Nos. 6,283,957, 6,071,908, 6,011,563, 5,855,595, 5,716,595, and 5,707,401, each of which is incorporated herein by reference in its entirety.

Radiotherapy can be administered to destroy tumor cells before or after surgery, before or after chemotherapy, and sometimes during chemotherapy. Radiotherapy may also be administered for palliative reasons to relieve symptoms of cancer, for example, to lessen pain. Among the types of tumors that can be treated using radiotherapy are localized tumors that cannot be excised completely and metastases and tumors whose complete excision would cause unacceptable functional or cosmetic defects or be associated with unacceptable surgical risks.

It will be appreciated that both the particular radiation dose to be utilized in treating CRC and the method of administration will depend on a variety of factors. Thus, the dosages of radiation that can be used according to the methods of the present disclosure are determined by the particular requirements of each situation. The dosage will depend on such factors as the size of the tumor, the location of the tumor, the age and sex of the patient, the frequency of the dosage, the presence of other tumors, possible metastases and the like. Those skilled in the art of radiotherapy can readily ascertain the dosage and the method of administration for any particular tumor by reference to Hall, E. J., Radiobiology for the Radiobiologist, 5th edition, Lippincott Williams & Wilkins Publishers, Philadelphia, Pa., 2000; Gunderson, L. L. and Tepper J. E., eds., Clinical Radiation Oncology, Churchill Livingstone, London, England, 2000; and Grosch, D. S., Biological Effects of Radiation, 2nd edition, Academic Press, San Francisco, Calif., 1980, each of which is incorporated herein by reference.

### b. CRC Treatment

For the treatment of CRC, surgical resection results in a cure for roughly 50% of patients. Surgical methods for colorectal cancer includes colon surgery, including open colectomy (also known as hemicolectomy, partial colectomy, or segmental resection), laparoscopic-assisted colectomy, and polypectomy and local excision, and rectal surgery, including polypectomy and local excision, local transanal resection (full thickness resection), transanal endoscopic microsurgery (TEM), low anterior resection, proctectomy with coloanal anastomosis, abdominoperineal resection (APR), and pelvic exenteration. Other treatments include radiofrequency ablation, ethanol (alcohol) ablation (also known as percutaneous ethanol injection (PEI)), cryosurgery (cryotherapy), and hepatic artery embolization.

Chemotherapy and irradiation maybe used both preoperatively (neoadjuvant) and postoperatively (adjuvant) in treating CRC. Chemotherapeutic agents, particularly 5-fluorouracil (5-FU), are powerful weapons in treating CRC. Other agents include oxaliplatin (Eloxatin®), irinotecan (Camptosar®), leucovorin, capecitabine (Xeloda®), bevacizumab (Avastin®), cetuximab (Erbitux®), and panitumumab (Vectibix®). These drugs are frequently combined. Common combinations are FOLFOX (5-FU, leucovorin, oxaliplatin); FOLFIRI (5-FU, leucovorin, irinotecan); and FOLFOXIRI (5-FU, leucovorin, irinotecan, oxaliplatin). Bevacizumab is a targeted therapeutic, specifically a monoclonal antibody that binds to vascular endothelial growth factor (VEGF) to prevent formation of blood vessels around the tumor. Cetuximab and panitumumab are monoclonal antibodies that target epidermal growth factor receptor (EGFR).

### 19. Immunoassays to Measure Markers

The methods described above quantify levels of the following combination of markers selected from the group consisting of AFP, CEA, ferritin, CYFRA, CA19-9, TIMP-1, Gal3, and/or CRP. The biomarkers, i.e., AFP, CEA, ferritin, CYFRA, CA19-9, TIMP-1, Gal3, and/or CRP, may be analyzed for the methods described above using an immunoassay. The presence or amount of marker can be determined using antibodies that specifically bind to each marker (namely, AFP, CEA, ferritin, CYFRA, CA19-9, TIMP-1, Gal3, and/or CRP as well as any additional analytes if such additional analytes are used). Examples of antibodies that can be used include a polyclonal antibody, a monoclonal antibody, a human antibody, an immunoglobulin molecule, a disulfide linked Fv, a monoclonal antibody, an affinity matured, a scFv, a chimeric antibody, a single domain antibody, a CDR-grafted antibody, a diabody, a humanized antibody, a multispecific antibody, a Fab, a dual specific antibody, a DVD, a Fab', a bispecific antibody, a F(ab')2, a Fv, and combinations thereof. For example, the immunological method may include (a) measuring the levels of AFP by: (i) contacting the test sample with at least one capture antibody, wherein the capture antibody binds to an epitope on AFP or a fragment of AFP to form a capture antibody-AFP antigen complex; (ii) contacting the capture antibody-AFP antigen complex with at least one detection antibody comprising a detectable label, wherein the detection antibody binds to an epitope on AFP that is not bound by the capture antibody and forms a capture antibody-AFP-antigen-detection antibody complex; and (iii) determining the AFP levels in the test sample based on the signal generated by the detectable label in the capture antibody-AFP-antigen-detection antibody complex formed in (a)(ii); (b) measuring the levels of CEA by: (i) contacting the test sample with at least one capture antibody, wherein the capture antibody binds to an epitope on CEA or a fragment of CEA to form a capture antibody-CEA antigen complex; (ii) contacting the capture antibody-CEA antigen complex with at least one detection antibody comprising a detectable label, wherein the detection antibody binds to an epitope on CEA that is not bound by the capture antibody and forms a capture antibody-CEA antigen-detection antibody complex; and (iii) determining the CEA levels in the test sample based on the signal generated by the detectable label in the capture antibody-CEA-antigen-detection antibody complex formed in (b)(ii); (c) measuring the levels of ferritin by: (i) contacting the test sample with at least one capture antibody, wherein the capture antibody binds to an epitope on ferritin or a fragment of ferritin to form a capture antibody-ferritin antigen complex; (ii) contacting the capture antibody-ferritin antigen complex with at least one detection antibody comprising a detectable label, wherein the detection antibody binds to an epitope on ferritin that is not bound by the capture antibody and forms a capture antibody-ferritin-antigen-detection antibody complex; and (iii) determining the ferritin levels in the test sample based on the signal generated by the detectable label in the capture antibody-ferritin-antigen-detection antibody complex formed in (c)(ii); (d) measuring the levels of CYFRA by: (i) contacting the test sample with at least one capture antibody, wherein the capture antibody binds to an epitope on CYFRA or a fragment of CYFRA to form a capture antibody-CYFRA antigen complex; (ii) contacting the capture antibody-CYFRA antigen complex with at least one detection antibody comprising a detectable label, wherein the detection antibody binds to an epitope on CYFRA that is not bound by the capture antibody and forms a capture antibody-CYFRA-antigen-detection antibody complex; and (iii) determining the CYFRA levels in the test sample based on the signal generated by the detectable label in the capture antibody-CYFRA-antigen-detection antibody complex formed in (d)(ii); (e) measuring the levels of CA19-9 by: (i) contacting the test sample with at least one capture antibody, wherein the capture antibody binds to an epitope on CA19-9 or a fragment of CA19-9 to form a capture antibody-CA19-9 antigen complex; (ii) contacting the capture antibody-CA19-9 antigen complex with at least one detection antibody comprising a detectable label, wherein the detection antibody binds to an epitope on CA19-9 that is not bound by the capture antibody and forms a capture antibody-CA19-9-antigen-detection antibody complex; and (iii) determining the CA19-9 levels in the test sample based on the signal generated by the detectable label in the capture antibody-CA19-9-antigen-detection antibody complex formed in (e)(ii); (f) measuring the levels of TIMP-1 by: (i) contacting the test sample with at least one capture antibody, wherein the capture antibody binds to an epitope on TIMP-1 or a fragment of TIMP-1 to form a capture antibody-TIMP-1 antigen complex; (ii) contacting the capture antibody-TIMP-1 antigen complex with at least one detection antibody comprising a detectable label, wherein the detection antibody binds to an epitope on TIMP-1 that is not bound by the capture antibody and forms a capture antibody-TIMP-1-antigen-detection antibody complex; and (iii) determining the TIMP-1 levels in the test sample based on the signal generated by the detectable label in the capture antibody-TIMP-1-antigen-detection antibody complex formed in (f)(ii); (g) measuring the levels of GAL3 by: (i) contacting the test sample with at least one capture antibody, wherein the capture antibody binds to an epitope on GAL3 or a fragment of GAL3 to form a capture antibody-GAL3 antigen complex; (ii) contacting the capture antibody-GAL3 antigen complex with at least one detection antibody comprising a detectable label, wherein the detection antibody binds to an epitope on GAL3 that is not bound by the capture antibody and forms a capture antibody-GAL3-antigen-detection antibody complex; and (iii) determining the GAL3 levels in the test sample based on the signal generated by the detectable label in the capture antibody-GAL3-antigen-detection antibody complex formed in (g)(ii); and/or (h) measuring the levels of CRP by: (i) contacting the test sample with at least one capture antibody, wherein the capture antibody binds to an epitope on CRP or a fragment of CRP to form a capture antibody-CRP antigen complex; (ii) contacting the capture antibody-CRP antigen complex with at least one detection antibody comprising a detectable label, wherein the detection antibody binds to an epitope on CRP that is not bound by the capture antibody and forms a capture antibody-CRP-antigen-detection antibody complex; and (iii) determining the CRP levels in the test sample based on the signal generated by the detectable label in the capture antibody-CRP-antigen-detection antibody complex formed in (h)(ii); or a combination thereof.

Any immunoassay may be utilized. The immunoassay may be an enzyme-linked immunoassay (ELISA), radioimmunoassay (RIA), a competitive inhibition assay, such as forward or reverse competitive inhibition assays, a fluorescence polarization assay, or a competitive binding assay, for example. The ELISA may be a sandwich ELISA. Specific immunological binding of the antibody to the marker can be detected via direct labels, such as fluorescent or luminescent tags, metals and radionuclides attached to the antibody or via indirect labels, such as alkaline phosphatase or horseradish peroxidase.

The use of immobilized antibodies or fragments thereof may be incorporated into the immunoassay. The antibodies may be immobilized onto a variety of supports, such as magnetic or chromatographic matrix particles, the surface of an assay plate (such as microtiter wells), pieces of a solid substrate material, and the like. An assay strip can be prepared by coating the antibody or plurality of antibodies in an array on a solid support. This strip can then be dipped into the test biological sample and then processed quickly through washes and detection steps to generate a measurable signal, such as a colored spot.

The sandwich ELISA measures the amount of antigen between two layers of antibodies (i.e. a capture antibody and a detection antibody (which may be labeled with a detectable label)). The marker, i.e., AFP, CEA, ferritin, CYFRA, CA19-9, TIMP-1, Gal3, and/or CRP, to be measured may contain at least two antigenic sites capable of binding to antibody. Either monoclonal or polyclonal antibodies may be used as the capture and detection antibodies in the sandwich ELISA.

Generally, at least two antibodies are employed to separate and quantify the marker, i.e., AFP, CEA, ferritin, CYFRA, CA19-9, TIMP-1, Gal3, and/or CRP (as well as any additional analytes), in a test or biological sample. More specifically, the at least two antibodies bind to certain epitopes of the marker forming an immune complex which is referred to as a "sandwich". One or more antibodies can be used to capture the marker in the test sample (these antibodies are frequently referred to as a "capture" antibody or "capture" antibodies) and one or more antibodies is used to bind a detectable (namely, quantifiable) label to the sandwich (these antibodies are frequently referred to as the "detection" antibody or "detection" antibodies). In a sandwich assay, both antibodies binding to their epitope may not be diminished by the binding of any other antibody in the assay to its respective epitope. In other words, antibodies may be selected so that the one or more first antibodies brought into contact with a test sample suspected of containing the marker do not bind to all or part of an epitope recognized by the second or subsequent antibodies, thereby interfering with the ability of the one or more second detection antibodies to bind to the marker.

In a preferred embodiment, a test or biological sample suspected of containing the marker, i.e., AFP, CEA, ferritin, CYFRA, CA19-9, TIMP-1, Gal3, and/or CRP, can be contacted with at least one first capture antibody (or antibodies) and at least one second detection antibodies either simultaneously or sequentially. In the sandwich assay format, a test sample suspected of containing the marker is first brought into contact with the at least one first capture antibody that specifically binds to a particular epitope under conditions which allow the formation of a first antibody-marker complex. If more than one capture antibody is used, a first multiple capture antibody-marker complex is formed. In a sandwich assay, the antibodies, preferably, the at least one capture antibody, are used in molar excess amounts of the maximum amount of marker expected in the test sample.

Optionally, prior to contacting the test sample with the at least one first capture antibody, the at least one first capture antibody can be bound to a solid support which facilitates the separation the first antibody-marker complex from the test sample. Any solid support known in the art can be used, including but not limited to, solid supports made out of polymeric materials in the forms of wells, tubes or beads. The antibody (or antibodies) can be bound to the solid support by adsorption, by covalent bonding using a chemical coupling agent or by other means known in the art, provided that such binding does not interfere with the ability of the antibody to bind the marker. Moreover, if necessary, the solid support can be derivatized to allow reactivity with various functional groups on the antibody. Such derivatization requires the use of certain coupling agents such as, but not limited to, maleic anhydride, N-hydroxysuccinimide and 1-ethyl-3-(3-dimethylaminopropyl)carbodiimide.

After the test sample suspected of containing the marker is brought into contact with the at least one first capture antibody, the test sample is incubated in order to allow for the formation of a first capture antibody (or multiple antibody)-marker complex. The incubation can be carried out at a pH of from about 4.5 to about 10.0, at a temperature of from about 2°C to about 45°C, and for a period from at least about one (1) minute to about eighteen (18) hours, from about 2-6 minutes, or from about 3-4 minutes.

After formation of the first/multiple capture antibody-marker complex, the complex is then contacted with at least one second detection antibody (under conditions which allow for the formation of a first/multiple antibody-marker second antibody complex). If the first antibody-marker complex is contacted with more than one detection antibody, then a first/multiple capture antibody-marker-multiple antibody detection complex is formed. As with first antibody, when the at least second (and subsequent) antibody is brought into contact with the first antibody-marker complex, a period of incubation under conditions similar to those described above is required for the formation of the first/multiple antibody-marker-second/multiple antibody complex. Preferably, at least one second antibody contains a detectable label. The detectable label can be bound to the at least one second antibody prior to, simultaneously with or after the formation of the first/multiple antibody-marker-second/multiple antibody complex. Any detectable label known in the art can be used.

### 20. Kits for Performing the Methods

Provided herein is a kit, which may be used for performing the methods described above. The kit may provide (1) reagents capable of specifically binding to each of the markers AFP, CEA, ferritin, CYFRA, CA19-9, TIMP-1, Gal3, and/or CRP, to quantify the levels of the markers, AFP, CEA, ferritin, CYFRA, CA19-9, TIMP-1, Gal3, and/or CRP, in a biological sample isolated from a subject and (2) a reference standard indicating reference levels of each of the markers AFP, CEA, ferritin, CYFRA, CA19-9, TIMP-1, Gal3, and/or CRP, wherein at least one reagent comprises at least one antibody capable of specifically binding the appropriate marker. The kit may comprise a reagent that is capable of specifically binding to AFP, a reagent that is capable of specifically binding to CEA, a reagent that is capable of specifically binding to ferritin, a reagent that is capable of specifically binding to CYFRA, a reagent that is capable of specifically binding to CA19-9, a reagent that is capable of specifically binding to TIMP-1, a reagent that is capable of specifically binding to Gal3, and/or a reagent that is capable of specifically binding to CRP, to quantify the concentration of each biomarker in the biological sample and a reference standard indicating the a reference level of each of the biomarker in the biological sample (i.e., AFP, CEA, ferritin, CYFRA, CA19-9, TIMP-1, Gal3, and/or CRP). The kit may further comprise at least one reagent capable of specifically binding (i.e., an antibody) at least one additional biomarker of mS9, HbA1c, C3a, Cat X, suPAR(I), PAI-1, and/or ENO2, or any combinations thereof, and a reference standard indicating a reference level of the at least one additional biomarker of CRC, if present.

The kit may comprise the antibodies and a means for administering the antibodies. The kit can further comprise instructions for using the kit and conducting the analysis, monitoring, or treatment.

The kit may also comprise one or more containers, such as vials or bottles, with each container containing a separate reagent. The kit may further comprise written instructions, which may describe how to perform or interpret an analysis, monitoring, treatment, or method described herein.

For example, the kit can comprise instructions for assaying the test sample for AFP, CEA, ferritin, CYFRA, CA19-9, TIMP-1, Gal3, and/or CRP by immunoassay, e.g., chemiluminescent microparticle immunoassay. The instructions can be in paper form or computer-readable form, such as a disk, CD, DVD, or the like. The antibody can be an AFP, CEA, ferritin, CYFRA, CA19-9, TIMP-1, Gal3, and/or CRP capture antibody and/or AFP, CEA, ferritin, CYFRA, CA19-9, TIMP-1, Gal3, and/or CRP detection antibody (meaning an antibody labeled with a detectable label). For example, the kit can contain at least one capture antibody that specifically binds AFP, at least one capture antibody that specifically binds CEA, at least one capture antibody that specifically binds ferritin, at least one capture antibody that specifically binds CYFRA, at least one capture antibody that specifically binds CA19-9, at least one capture antibody that specifically binds TIMP-1, at least one capture antibody that specifically binds Gal3, and/or at least one capture antibody that specifically binds CRP. The kit can also contain a conjugate antibody (such as an antibody labeled with a detectable label) for each capture antibody (namely, a conjugate antibody for each of the capture antibodies that specifically bind to AFP, CEA, ferritin, CYFRA, CA19-9, TIMP-1, Gal3, and/or CRP, respectively). Alternatively or additionally, the kit can comprise a calibrator or control, e.g., purified, and optionally lyophilized, (e.g., AFP, CEA, ferritin, CYFRA, CA19-9, TIMP-1, Gal3, and/or CRP), and/or at least one container (e.g., tube, microtiter plates or strips, which can be already coated with an anti-AFP, CEA, ferritin, CYFRA, CA19-9, TIMP-1, Gal3, and/or CRP monoclonal antibody) for conducting the assay, and/or a buffer, such as an assay buffer or a wash buffer, either one of which can be provided as a concentrated solution, a substrate solution for the detectable label (e.g., an enzymatic label), or a stop solution. Preferably, the kit comprises all components, i.e., reagents, standards, buffers, diluents, etc., which are necessary to perform the assay. The instructions also can include instructions for generating a standard curve or a reference standard for purposes of quantifying AFP, CEA, ferritin, CYFRA, CA19-9, TIMP-1, Gal3, and/or CRP.

As alluded to above, any antibodies, which are provided in the kit, such as recombinant antibodies specific for AFP, CEA, ferritin, CYFRA, CA19-9, TIMP-1, Gal3, and/or CRP, can incorporate a detectable label, such as a fluorophore, radioactive moiety, enzyme, biotin/avidin label, chromophore, chemiluminescent label, or the like, or the kit can include reagents for labeling the antibodies or reagents for detecting the antibodies (e.g., detection antibodies) and/or for labeling the analytes or reagents for detecting the analyte. The antibodies, calibrators and/or controls can be provided in separate containers or pre-dispensed into an appropriate assay format, for example, into microtiter plates.

Optionally, the kit includes quality control components (for example, sensitivity panels, calibrators, and positive controls). Preparation of quality control reagents is well-known in the art and is described on insert sheets for a variety of immunodiagnostic products. Sensitivity panel members optionally are used to establish assay performance characteristics, and further optionally are useful indicators of the integrity of the immunoassay kit reagents, and the standardization of assays.

The kit can also optionally include other reagents required to conduct a diagnostic assay or facilitate quality control evaluations, such as buffers, salts, enzymes, enzyme cofactors, substrates, detection reagents, and the like. Other components, such as buffers and solutions for the isolation and/or treatment of a test sample (e.g., pretreatment reagents), also can be included in the kit. The kit can additionally include one or more other controls. One or more of the components of the kit can be lyophilized, in which case the kit can further comprise reagents suitable for the reconstitution of the lyophilized components.

The various components of the kit optionally are provided in suitable containers as necessary, e.g., a microtiter plate. The kit can further include containers for holding or storing a sample (e.g., a container or cartridge for a blood sample). Where appropriate, the kit optionally also can contain reaction vessels, mixing vessels, and other components that facilitate the preparation of reagents or the test sample. The kit can also include one or more instrument for assisting with obtaining a test sample, such as a syringe, pipette, forceps, measured spoon, or the like.

If the detectable label is at least one acridinium compound, the kit can comprise at least one acridinium-9-carboxamide, at least one acridinium-9-carboxylate aryl ester, or any combination thereof. If the detectable label is at least one acridinium compound, the kit also can comprise a source of hydrogen peroxide, such as a buffer, solution, and/or at least one basic solution.

If desired, the kit can contain a solid phase, such as a magnetic particle, bead, test tube, microtiter plate, cuvette, membrane, scaffolding molecule, film, filter paper, a quartz crystal, disc or chip. The kit may also include a detectable label that can be or is conjugated to an antibody, such as an antibody functioning as a detection antibody. The detectable label can for example be a direct label, which may be an enzyme, oligonucleotide, nanoparticle, chemiluminophore, fluorophore, fluorescence quencher, chemiluminescence quencher, or biotin. Kits may optionally include any additional reagents needed for detecting the label.

If desired, the kit can further comprise one or more components, alone or in further combination with instructions, for assaying the test sample for another analyte, which can be a biomarker, such as a biomarker of cancer. Examples of analytes include, but are not limited to AFP, CEA, ferritin, CYFRA, CA19-9, TIMP-1, Gal3, and/or CRP, and fragments of AFP, CEA, ferritin, CYFRA, CA19-9, TIMP-1, Gal3, and/or CRP as well other analytes and biomarkers discussed herein, or otherwise known in the art. In some embodiments one or more components for assaying a test sample for AFP, CEA, ferritin, CYFRA, CA19-9, TIMP-1, Gal3, and/or CRP enable the determination of the presence, amount or concentration of AFP, CEA, ferritin, CYFRA, CA19-9, TIMP-1, Gal3, and/or CRP. A sample, such as a serum sample, can also be assayed for AFP, CEA, ferritin, CYFRA, CA19-9, TIMP-1, Gal3, and/or CRP using TOF-MS and an internal standard.

The kit (or components thereof), as well as the method of determining the concentration of AFP, CEA, ferritin, CYFRA, CA19-9, TIMP-1, Gal3, and/or CRP in a test sample by an immunoassay as described herein, can be adapted for use in a variety of automated and semi-automated systems (including those wherein the solid phase comprises a microparticle), as described, e.g., in U.S. Patent Nos. 5,089,424 and 5,006,309, and as commercially marketed, e.g., by Abbott Laboratories (Abbott Park, IL) as ARCHITECT®.

Some of the differences between an automated or semi-automated system as compared to a non-automated system (e.g., ELISA) include the substrate to which the first specific binding partner (e.g., analyte antibody or capture antibody) is attached (which can impact sandwich formation and analyte reactivity), and the length and timing of the capture, detection and/or any optional wash steps. Whereas a non-automated format such as an ELISA may require a relatively longer incubation time with sample and capture reagent (e.g., about 2 hours), an automated or semi-automated format (e.g., ARCHITECT® and any successor platform, Abbott Laboratories) may have a relatively shorter incubation time (e.g., approximately 18 minutes for ARCHITECT®). Similarly, whereas a non-automated format such as an ELISA may incubate a detection antibody such as the conjugate reagent for a relatively longer incubation time (e.g., about 2 hours), an automated or semi-automated format (e.g., ARCHITECT® and any successor platform) may have a relatively shorter incubation time (e.g., approximately 4 minutes for the ARCHITECT® and any successor platform).

Other platforms available from Abbott Laboratories include, but are not limited to, AxSYM®, IMx® (see, e.g., U.S. Pat. No. 5,294,404, which is hereby incorporated by reference in its entirety), PRISM®, EIA (bead), and Quantum™ II, as well as other platforms. Additionally, the assays, kits and kit components can be employed in other formats, for example, on electrochemical or other hand-held or point-of-care assay systems. The present disclosure is, for example, applicable to the commercial Abbott Point of Care (i-STAT®, Abbott Laboratories) electrochemical immunoassay system that performs sandwich immunoassays. Immunosensors and their methods of manufacture and operation in single-use test devices are described, for example in, U.S. Patent No. 5,063,081, U.S. Pat. App. Pub. No. 2003/0170881, U.S. Pat. App. Pub. No. 2004/0018577, U.S. Pat. App. Pub. No. 2005/0054078, and U.S. Pat. App. Pub. No. 2006/0160164, which are incorporated in their entireties by reference for their teachings regarding same.

In particular, with regard to the adaptation of an assay to the I-STAT® system, the following configuration is preferred. A microfabricated silicon chip is manufactured with a pair of gold amperometric working electrodes and a silver-silver chloride reference electrode. On one of the working electrodes, polystyrene beads (0.2 mm diameter) with immobilized capture antibody are adhered to a polymer coating of patterned polyvinyl alcohol over the electrode. This chip is assembled into an I-STAT® cartridge with a fluidics format suitable for immunoassay. On a portion of the wall of the sample-holding chamber of the cartridge there is a layer comprising the detection antibody labeled with alkaline phosphatase (or other label). Within the fluid pouch of the cartridge is an aqueous reagent that includes p-aminophenol phosphate.

In operation, a sample suspected of containing AFP, CEA, ferritin, CYFRA, CA19-9, TIMP-1, Gal3, and/or CRP is added to the holding chamber of the test cartridge and the cartridge is inserted into the I-STAT® reader. After the second antibody (detection antibody) has dissolved into the sample, a pump element within the cartridge forces the sample into a conduit containing the chip. Here it is oscillated to promote formation of the sandwich between the first capture antibody, AFP, CEA, ferritin, CYFRA, CA19-9, TIMP-1, Gal3, and/or CRP, and the labeled second detection antibody. In the penultimate step of the assay, fluid is forced out of the pouch and into the conduit to wash the sample off the chip and into a waste chamber. In the final step of the assay, the alkaline phosphatase label reacts with p-aminophenol phosphate to cleave the phosphate group and permit the liberated p-aminophenol to be electrochemically oxidized at the working electrode. Based on the measured current, the reader is able to calculate the amount of AFP, CEA, ferritin, CYFRA, CA19-9, TIMP-1, Gal3, and/or CRP in the sample by means of an embedded algorithm and factory-determined calibration curve.

It will be readily apparent to those skilled in the art that other suitable modification and adaptations of the methods of the present disclosure described herein are readily applicable and appreciable, and may be made using suitable equivalents without departing from the scope of the present disclosure or the aspects and embodiments disclosed herein. Having now described the present disclosure in detail, the same will be more clearly understood by reference to the following examples which are merely intended only to illustrate some aspects and embodiments of the disclosure, and should not be viewed as limiting to the scope of the disclosure. The disclosures of all journal references, U.S. patents and publications referred to herein are hereby incorporated by reference in their entireties.

The present invention has multiple aspects, illustrated by the following nonlimiting examples. The examples not falling within the scope of the claims are given for reference.

### EXAMPLES

### Example 1

### Methods and Materials

Clinical populations (primary analysis on 4521 of a total 4698 patients) were used from the Hvidovre Hospital Endoscopy II trial patient's plasma specimens. Group 1 (3610): 290 Low Risk adenomas & 3320 other non-cancer population; Group 2 (911): 399 High Risk adenomas, 512 CRC (∼50% early stage) - Staging Information: S1 (107), S2 (163), S3 (139), S4 (108), and 1 missing stage. Group 3 (177): Other non-CRC malignancies: Total 176 (not CRC) (See Table 1), which were removed for primary analyses. Clinical pedigree: Danish national registry & ability to "audit" data. 110 clinical parameters were collected with a focus on objective/trusted parameters.

**Table 1**

| **Cancer** | **Number of Subjects** |
|---|---|
| lung | 33 |
| ovary | 16 |
| breast | 4 |
| kidney | 7 |
| anal canal | 6 |
| unknown primary | 16 |
| B-cell lymphoma | 17 |
| uterine | 1 |
| prostate | 10 |
| gallbladder | 8 |
| larynx | 3 |
| malignant melanoma | 2 |
| pancreas | 22 |
| CLL | 3 |
| malignant myeloma | 5 |
| testes | 1 |
| small intestine | 3 |
| bladder | 1 |
| stomach | 9 |
| mesothelioma | 3 |
| adrenal gland | 1 |
| neuroendocrine | 2 |
| esophagus | 3 |

Individuals with symptoms of CRC that were referred to sigmoidoscopy and/or colonoscopy at one of the six participating Danish Hospitals were included in the study (all received colonoscopy as part of study participation). During recruitment, data was recorded and samples were collected at baseline. Baseline data was summarized (means ± standard deviations or medians and inter-quartile ranges for continuous data and proportions for categorical data). Statistical differences using the unpaired t-test, Mann-Whitney U test, χ², or Fisher exact test, as appropriate, was used to test the statistical difference between groups. Inclusion and exclusion criteria are shown in Table 2.

**Table 2**

| Inclusion criteria: | Exclusion criteria: |
|---|---|
| Age > 18 years old | Previous CRC, adenoma, or member of HNPCC or FAP families |
| Symptoms indicating colorectal cancer | Not able to understand Danish language |
| Never before having had a coloscopy. | Not able to read and/or understand the distributed patient information. |

Screening relevant neoplasias and cancers were defined as these findings in screened patients:
a. Adenomas with high grade dysplasia (Pathologist assessed)-Defined by Danish malignancy conversion grade (high grade only);
b. Adenomas greater than or equal to 1 cm;
c. Number of polyps in the bowel (greater than or equal to 3)
d. CRCs of all stages

54 ml blood was collected from a peripheral, antecubital vein, see Example 2. All blood samples were taken ahead of colonoscopy. The levels of biomarkers were determined in the collected blood samples. The remaining samples were stored at -80°C in a biobank at Hvidovre Hospital, where all -80°C freezers were under an electronic 24 hr/7 days a week surveillance.

### Example 2

### Blood samples-blood collection and handling

All collection devices, collection tubes, pipettes, storage vials, etc. were delivered by the protocol management. All devises and tubes were endotoxin, DNA'se and RNA'se free.

Samples were collected at room temp using light tourniquet, stored before centrifugation at room temp, centrifuged within 2 hours at 3,200xG, 10 min, separated carefully without disturbing the 0.5 cm plasma/serum above the buffy-coat (white band as shown at the study introduction). The samples were separated just after the centrifugation was finalized.
1. 30 ml blood from a peripheral forearm vein in 3 EDTA tubes of 10 ml each (purple). Supernatants from tube 1 were transferred to 4 CM-lab vials with approximately 1 ml in each tube - marked with barcodes 1-4. Supernatants from tubes 2 plus 3 were transferred to CM-lab tubes with approximately 2 ml in each tube - marked with barcodes 5-8.
2. 20 ml blood 2 tubes of 10 ml each-without additives (red). Supernatants from the 2 tubes were transferred to 6 CM-lab vials with approximately 1.5 ml in each - marked with barcodes 9-14.
3. 9 ml blood in 2 tubes with citrate additive - 4.5 ml in each tube (light blue). Supernatants from the 2 tubes were transferred to 4 CM-lab vials with approximately 1.2 ml in each - marked with barcodes 15-18.
4. The buffy-coats from the 3 EDTA tubes. Buffy-coats from each tube were transferred to 1 (one) CM-lab vial each (3 in total). Sterile, isotonic NaCl was added in dilution ratio of 1:1 and turn up and down 8-10 times to mix - marked with barcodes 19-21.

All vials were stored immediately at -80°C in the protocol freezers and using the delivered storage boxes (5.5 x 5.5 x 13 cm). Stored vials were transferred to on a frequent basis to the repository at Hvidovre Hospital for long-term storage at -80°C under 24/7 electronic surveillance.

### Example 3

### Statistical Evaluation

Analyses were performed using SAS v9.2, R v3.0.3, or JMP Pro v11. A multivariable statistical evaluation of the variables was performed using AIM (adaptive index model) (Tian Lu, Tibshirani Robert. Adaptive Index models for marker-based risk stratification. Biostatistics (2011), 12, 1, pp 68-86).

### Example 4

### Biomarker selection

A combination of biomarkers were investigated for new clinical utilities as an aid in the detection of colon and rectum neoplasias (e.g. high grade adenomas) and early stage colorectal cancers (CRCs). The study population included a total of 4698 subjects.

**Algorithm Development.** Adaptive Index Model (AIM) methodology was selected to identify an optimal subset of variables and their cutoffs that when combined as an algorithm ("index") could select persons at high-risk for the outcome under evaluation. Table 7 describes the outcome numbers. In this procedure, a logistic regression model was used to sequentially estimate a sequence of adaptive index models with up to a specified maximum number of binary rules. This procedure employed forward selection of variables and their cutoffs to construct this sequential set of indexes with an increasing number of binary rules. The optimal number of binary rules, or variables, to be included in the index was chosen via k-fold cross-validation. K-fold cross-validation is a sample reuse method that breaks the data into k randomly chosen segments. For each of the iterations, one of the k segments was used as a "test" dataset and the other k-1 segments combined were used as the "training" dataset. As a result, the procedure was repeated k times. For each of the iterations of the procedure, the score test statistic was estimated in the test dataset for testing the association between the outcome and the index constructed using the training data. The optimal number of allowed binary rules, or variables, to be included in the index was determined by the cross-validated (largest mean) score statistic.

The data was randomly split into an internal dataset to be used for algorithm development using the procedure described above and an external dataset that was not used at all in the algorithm development but that acted as an independent evaluation of algorithm performance for each outcome. This method of an internal and external dataset was preferable to mimic a real-world scenario. Using all evaluable data for each outcome from the internal dataset, Model 1-Model 6 were constructed via cross-validation, identifying the optimal number of variables and their cutoffs by gender. Outcome 1, Outcome 5 and Outcome 12 were evaluated. Parameters under evaluation included: markers AFP, CEA, ferritin, Galectin-3, CYFRA 21-1, CA 19-9 XR, TIMP-1, and hs-CRP; age, comorbidity count as a categorical variable (0, 1 or 2+), and comorbidities of CVD and Diabetes as measured by Danish codes. Table 3 shows the criteria for each Model.

**Table 3**

| | |
|---|---|
| Outcome | Outcome 1, Outcome 5 or Outcome 12 |
| Dataset | Training dataset from the internal dataset |
| Gender | Males or Females |
| N | Sample size used to build the model |
| Variables | Variables considered for algorithm |
| Nsteps | Maximum number of allowed binary rules (i.e. the maximum number of variable to be allowed in the model) |
| Kfold | 10-fold cross-validation |
| Kmax | optimal number of variables returned from the AIM procedure |
| Index | the optimal variables and their cutoffs returned from the AIM procedure |

In addition, an AIM score from the identified index was calculated for subjects in both the internal and external datasets. The AIM score is the number of variables from the index in which the cutoff criteria is met. This AIM score takes discrete values from 0 to J (where J is the number of optimal number of variables identified in the index). A score of 0 indicates that the person does not meet the cutoff criteria for any variable in the index. A score of 1 indicates that the person meets the cutoff criteria for only 1 variable in the index. It does not matter which of the J cutoff criterion is met. A score of J indicates that a person meets the cutoff criteria for every variable in the index. A confusion matrix (misclassification rate, accuracy, sensitivity, specificity, negative predictive value, and positive predictive value) can then be calculated for each value of the AIM score from 1 to J, using that score value as a threshold. Subjects with a score greater than or equal to that threshold were considered "positive" for the outcome of interest. A threshold was selected that would be clinically useful in terms of its estimated confusion matrix parameters. By calculating the confusion matrix for the internal and the external datasets, the degree of optimism that results from overfitting was seen in the difference in the measures between the internal and external datasets.

Table 4 shows the AIM methodology, where List: markers AFP, CEA, ferritin, Galectin-3, CYFRA 21-1, CA 19-9 XR, TIMP-1 and hs-CRP; age, CVD comorbidity, Diabetes comorbidity and comorbidity count as a categorical variable (0, 1 or 2+).

**Table 4 AIM Methodology**

| | **Model 1** | **Model 2** | **Model 3** | **Model 4** | **Model 5** | **Model 6** |
|---|---|---|---|---|---|---|
| **Outcome** | 1 | 1 | 5 | 5 | 12 | 12 |
| **Dataset** | Internal | Internal | Internal | Internal | Internal | Internal |
| **Gender** | Males | Females | Males | Females | Males | Females |
| **N** | 1373 | 1526 | 1236 | 1409 | 1101 | 1328 |
| **Variables** | Marker list | Marker list | Marker list | Marker list | Marker list | Marker list |
| **Nsteps** | 12 | 11 | 12 | 11 | 12 | 11 |
| **K-fold** | 10 | 10 | 10 | 10 | 10 | 10 |
| **Kmax** | 5 | 5 | 5 | 5 | 5 | 3 |
| | | | | | | |
| **Index** | cea>6.5 | cyfra>1.72 | cea>6.9 | cea>5.9 | cyfra>2.13 | cyfra>2.06 |
| | age>54 | cea>4.8 | cyfra>2.17 | cyfra>2.01 | crp>15.4 | cea>5.7 |
| | cyfra>1.98 | crp>5.5 | crp>18.3 | CA19-9>24 | TIMP-1>148 | crp>10.4 |
| | crp>1.8 | ferritin<38 | ferritin<97 | ferritin<36 | CA19-9>25.0 | |
| | ferritin<109 | age>63 | CA19-9>26.9 | crp>7.8 | afp>6.7 | |

### Model Performance: Definitions

*Threshold:* potential level of a "score."

% *Misclassified:* the proportion of people that the algorithm classifies as opposite of their true designation.

*Sensitivity (SE):* the proportion of people with the disease who are positive on the test.

*Specificity (SP):* the proportion of people without the disease who are negative on the test.

*Positive Predictive Value (PPV):* the probability that a person with a positive test has the disease.

*Negative Predictive Value (NPV):* the probability that a person with a negative test does not have the disease.

*Accuracy:* proportion of correct diagnosis.

*Likelihood Ratio Positive (LR*+*)*: ratio of the probability of a positive test result in a person with the disease to the probability of a positive test result in a person without the disease.

*Likelihood Ratio Negative (LR*-*)*: ratio of the probability of a negative test result in a person with the disease to the probability of a negative test result in a person without the disease.

*Posttest Probability (Post PR*+*)*: the probability of having the disease, given the prevalence of disease and likelihood of a positive test.

### Example 5

### Results-Males

The confusion matrix for Males is shown in Table 5. Outcome 1 Sample Size: Internal = 1373, External = 769 (178 positives); Outcome 5 Sample Size: Internal = 1236, External = 690 (38 positives, small); Outcome 12 Sample Size: Internal = 1101, External = 618 (33 positives, small). Shaded indicates thresholds having close to 60% specificity and/or 60% sensitivity.

**Confusion Matrix Interpretation.** Using Outcome 1, External Dataset, Threshold ≥ 3 from Table 5 as an example:
% Misclassified: 25% of subjects are misclassified (either predicted to have cancer when they do not or predicted not to have cancer when they do); overall error rate.
Accuracy: 75% overall correct diagnosis.
SE: 55% of people with the disease meet at least 3 of the cutoffs of the variables in this algorithm (a score of at least 3).
SP: 81% of the people without the disease have a score less than 3.
PPV: 47% of the people who have a score of at least 3 actually have the disease.
NPV: 86% of the people who have a score less than 3 do not have the disease.
LR+: 2.96 > 1 signifies that a person with at least a score of 3 has in increased probability of having the disease.
LR-: 0.55 < 1 signifies that a person with a score less than 3 has a decreased probability of having the disease.
Post PR+: have a core of at least 3 increases the probability of having the disease from 20% (the prevalence*) to 43%.
*A prevalence of 20% was used for Outcome 1, 12% for outcome 5 and 5% for Outcome 12, based on the actual rate seen in this study.

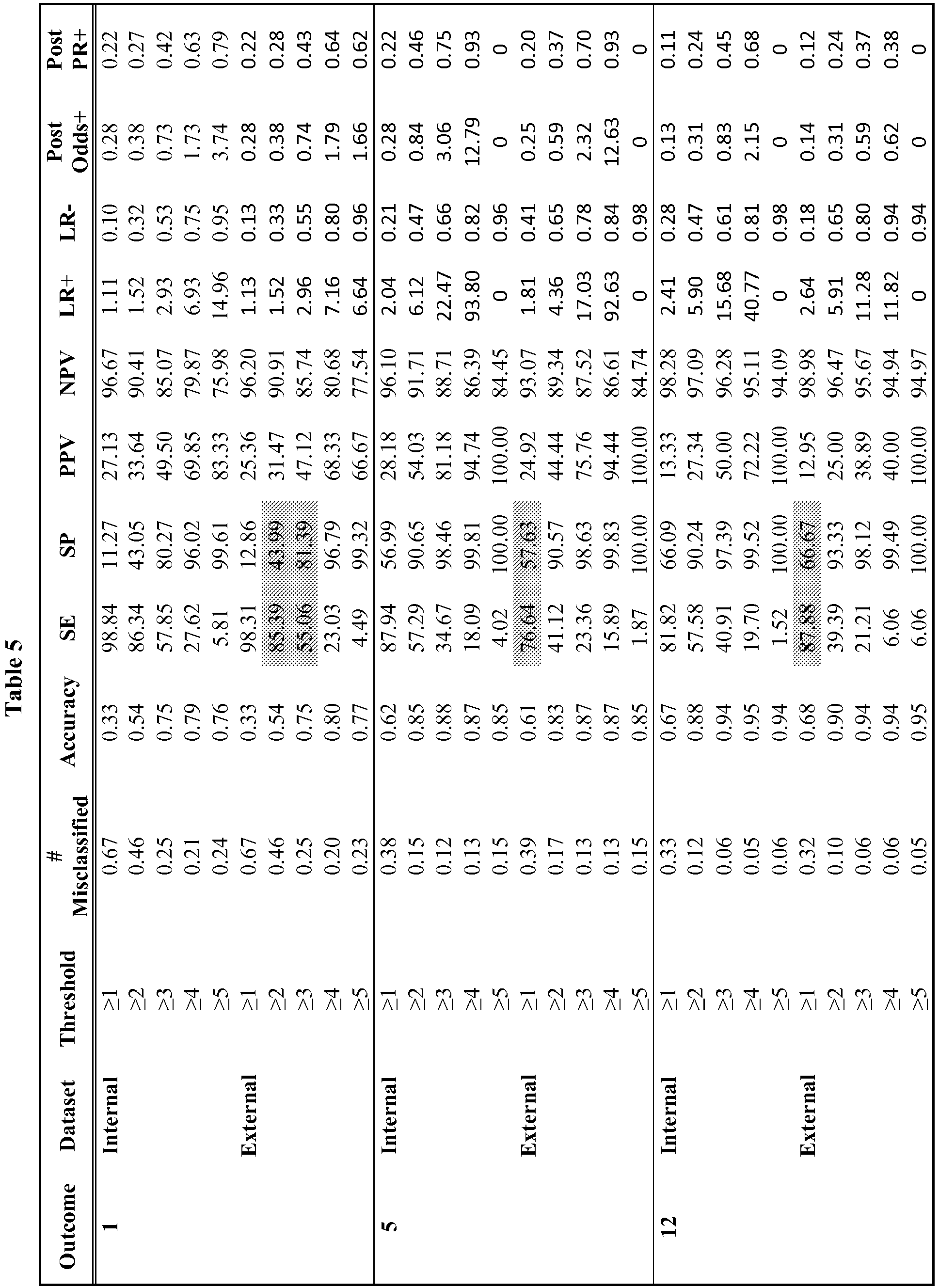

### Example 6

### Results-Females

The confusion matrix for Females is shown in Table 6. Outcome 1 Sample Size: Internal = 1526, External = 851 (133 positives); Outcome 5 Sample Size: Internal = 1409, External = 785 (73 positives); Outcome 12 Sample Size: Internal = 1328, External = 738 (32 positives, small). Shaded indicates thresholds having close to 60% specificity and/or 60% sensitivity.

**Confusion Matrix Interpretation.** Using Outcome 1, External Dataset, Threshold ≥ 3 from Table 6 as an example:
% Misclassified: 20% of subjects are misclassified (either predicted to have cancer when they do not or predicted not to have cancer when they do); overall error rate.
Accuracy: 80% overall correct diagnosis.
SE: 28% of people with the disease meet at least 3 of the cutoffs of the variables in this algorithm (a score of at least 3).
SP: 90% of the people without the disease have a score less than 3.
PPV: 34% of the people who have a score of at least 3 actually have the disease.
NPV: 87% of the people who have a score less than 3 do not have the disease.
LR+: 2.77 > 1 signifies that a person with at least a score of 3 has in increased probability of having the disease.
LR-: 0.80 < 1 signifies that a person with a score less than 3 has a decreased probability of having the disease.
Post PR+: have a core of at least 3 increases the probability of having the disease from 20% (the prevalence*) to 41%.
*A prevalence of 20% was used for Outcome 1, 12% for outcome 5 and 5% for Outcome 12, based on the actual rate seen in this study.

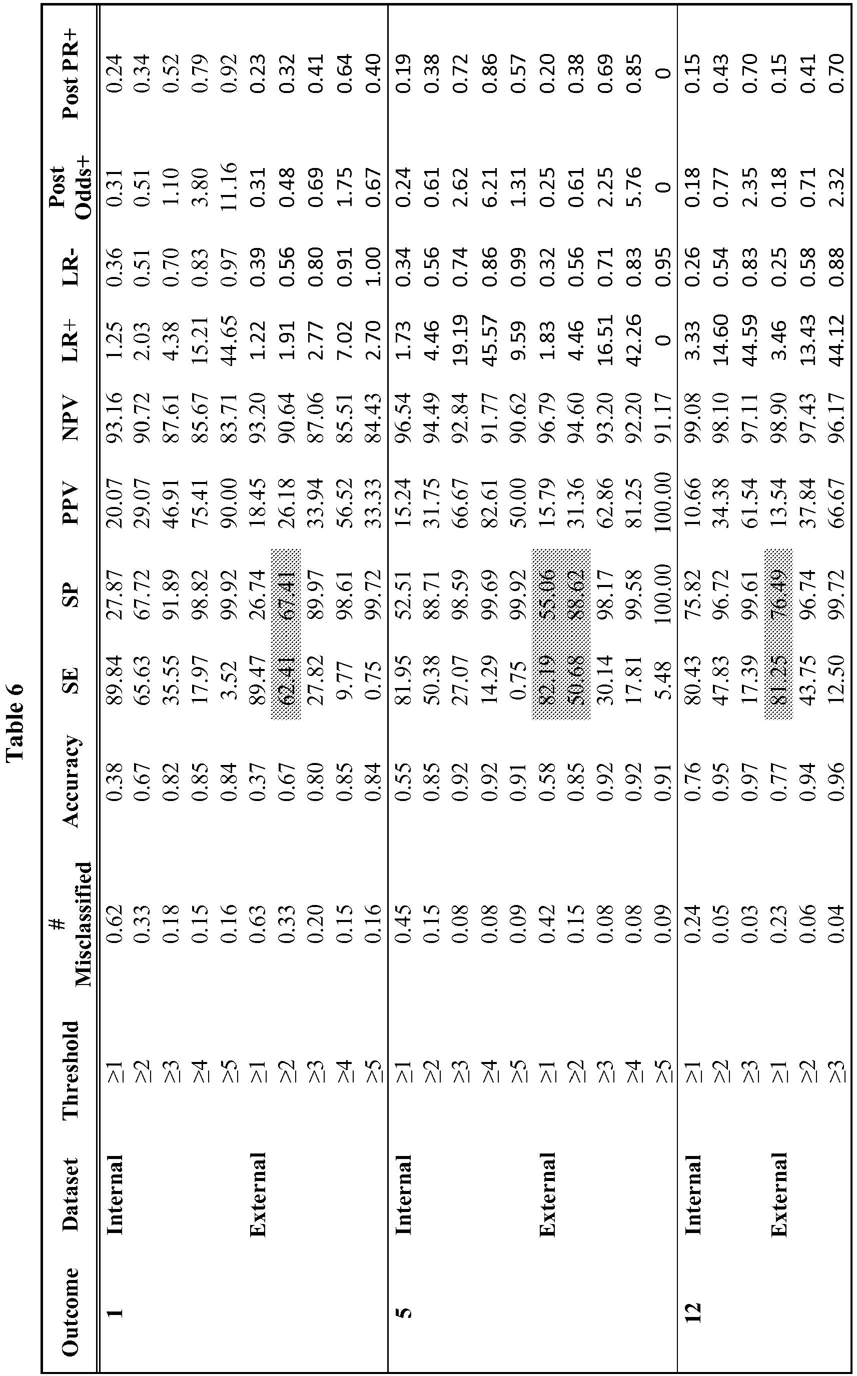

### Example 7

### Stage and Age Assessment

Table 7 describes the Outcome numbers. The Outcome 1 (High risk adenomas + all CRCs vs All non-cancers) Algorithm was used to evaluate and predict a "Disease group" vs a "Control group," as shown in Table 8. Table 8 shows the sub-assessments using the primary outcome (All others without other cancers vs. high risk adenomas + all CRCs) algorithm. Additionally the Outcome 1 Algorithm was also used in the comparison group of All high risk adenomas vs Low risk adenomas.

**Table 7**

| **Outcome number** | **Primary Outcomes:** |
|---|---|
| **1** | ∘ CRC (all stages) and high grade adenomas vs. all others (including low grade adenoma, other findings and no findings, and *excluding* "other non-CRC cancers") |
| **2** | ∘ High grade adenomas vs. all others (including low grade adenoma, other findings and no findings, and *excluding* all CRCs and "other non-CRC cancers") |
| **3** | ∘ CRC (all stages) vs. all others including all adenomas, other findings and no findings, and *excluding* "other non-CRC cancers") |

| | **Secondary Outcomes:** |
|---|---|
| **4** | ∘ All Stages of CRC and all other non-CRC cancers vs. All others (including all adenomas, other findings and no findings) |
| **5** | ∘ All Stages of CRC vs. All others (including low grade adenoma, other findings and no findings) |
| **6** | ∘ Stage I & II CRC vs. All Others (including low grade adenoma, other findings and no findings) |
| **7** | ∘ All Stages of CRC vs. Adenomas |
| **8** | ∘ Stage I & II CRC vs. Adenomas |
| **9** | ∘ All Stages of CRC vs. No findings |
| **10** | ∘ Stage I & II CRC vs. No findings |
| **11** | ∘ High Grade Adenomas vs. Low Grade Adenomas |
| **12** | ∘ Non-colorectal cancers (-CRC and -high grade adenomas) vs. all others |

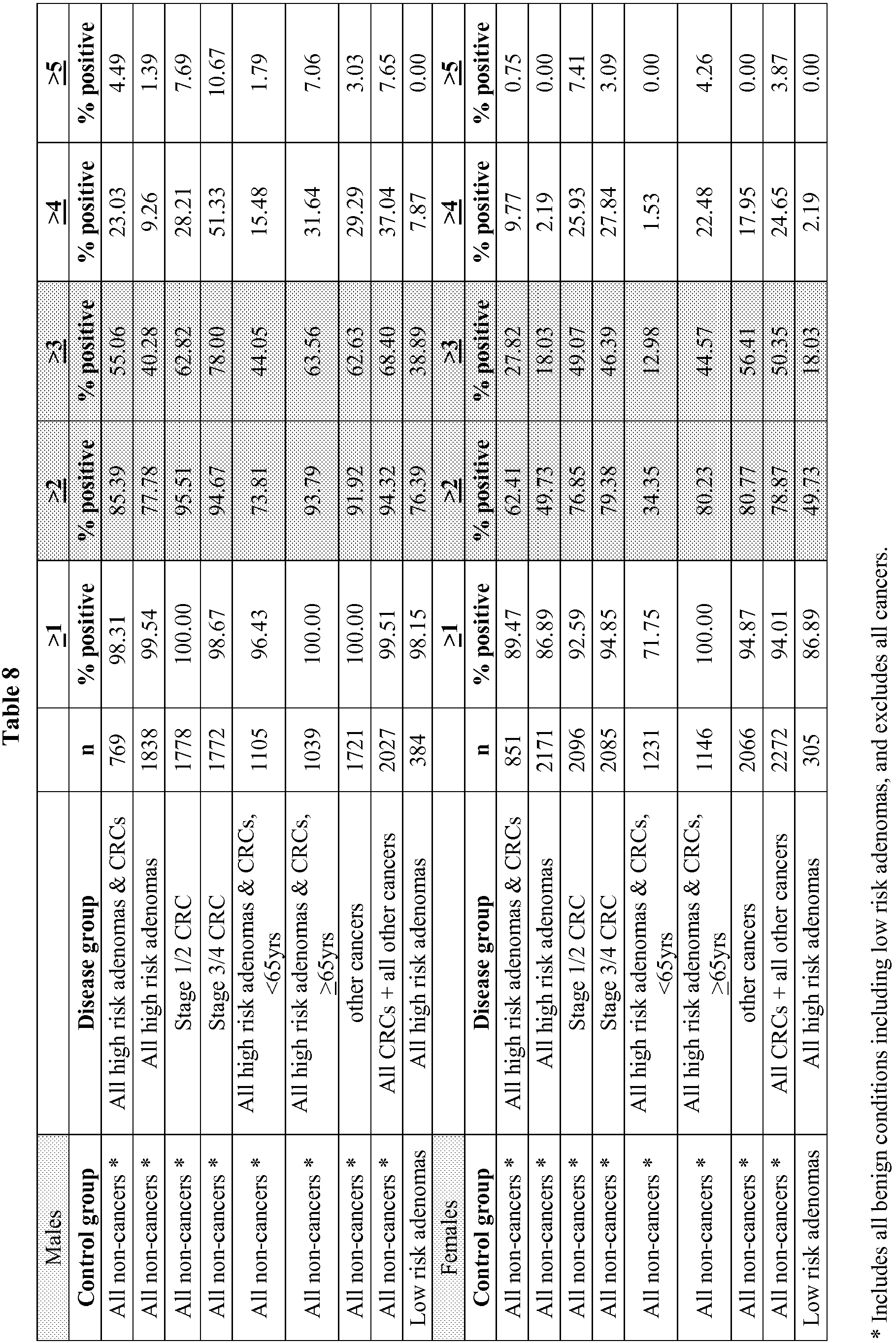

As shown in Table 8, Outcome 1 Algorithm performs well to predict disease outcome when compared to a control group. In particular, Outcome 1 Algorithm used to compare all Stage Cancer group ("All high risk adenomas & CRCs") vs. the non-cancers group (""All non-cancers"") baseline in Males and Females predicted 85.39% and 62.41% positive, respectively, when using a score of more than 2. Outcome 1 Algorithm used to compare Stage I and II CRCs vs. the non-cancers group ("All non-cancers") in Males and Females predicted 95.51% and 76.85% positive, respectively, when using a score of more than 2. Outcome 1 Algorithm used to compare Stage III and IV CRCs vs. the non-cancers group ("All non-cancers") in Males and Females predicted 94.67% and 79.38% positive, respectively, when using a score of more than 2. Outcome 1 Algorithm used to compare other cancers (including cancers listed in Table 1) vs. the non-cancers group ("All non-cancers") in Males and Females predicted 91.92% and 80.77% positive, respectively, when using a score of more than 2, indicating that the Outcome 1 Algorithm can be used to detect other cancers besides colorectal cancers.

It is understood that the foregoing detailed description and accompanying examples are merely illustrative and are not to be taken as limitations upon the scope of the invention, which is defined solely by the appended claims and their equivalents.

Various changes and modifications to the disclosed embodiments will be apparent to those skilled in the art. Such changes and modifications, including without limitation those relating to the chemical structures, substituents, derivatives, intermediates, syntheses, compositions, formulations, or methods of use of the invention, may be made without departing from the scope of the claims.

## Claims

1. An in vitro method of determining whether a subject is suffering from, or at risk of suffering from, high risk adenomas or colorectal cancer (CRC), the method comprising the steps of:
(a) providing a biological sample previously obtained from a subject;
(b) determining the levels of carcinoembryonic antigen (CEA), Cytokeratin 19 Fragment (CYFRA), C-reactive protein (CRP), and ferritin in the biological sample from the subject;
(c) comparing the levels of CEA, CYFRA, CRP, and ferritin in the biological sample to reference levels of CEA, CYFRA, CRP, and ferritin;
(i) providing a CEA score for the subject wherein the subject gets a score of 1 if the level of CEA in the biological sample is greater than the reference level of CEA and a score of 0 if the level of CEA in the biological sample is equal or less than the reference level of CEA;
(ii) providing a CYFRA score for the subject wherein the subject gets a score of 1 if the level of CYFRA in the biological sample is greater than the reference level of CYFRA and a score of 0 if the level of CYFRA in the biological sample is equal or less than the reference level of CYFRA;
(iii) providing a CRP score for the subject wherein the subject gets a score of 1 if the level of CRP in the biological sample is greater than the reference level of CRP and a score of 0 if the level of CRP in the biological sample is equal or less than the reference level of CRP; and
(iv) providing a ferritin score for the subject wherein the subject gets a score of 1 if the level of ferritin in the biological sample is less than the reference level of ferritin and a score of 0 if the level of ferritin in the biological sample is equal or greater than the reference level of ferritin;
(d) adding the scores from step (c) to generate a total score; and
(e) providing a diagnosis of a subject as suffering from, or at risk of suffering from, high risk adenomas or CRC if the total score is greater than a reference score, or a diagnosis of a subject as not suffering from, or not at risk of suffering from, high risk adenomas or CRC if the total score is equal to or less than the reference score.

2. The method of claim 1, further comprising: determining the age of the subject in step (b); comparing the age of the subject to a reference age in step (c); and
(v) providing an age score for the subject wherein the subject gets a score of 1 if the age of the subject is greater than or equal to the reference age and a score of 0 if the age of the subject is less than the reference age.

3. The method of claim 1 or 2, wherein the subject is male or female.

4. The method of claim 3, wherein:
(a) in a male subject at least 54 years old, the reference level of CEA is at least 6.5 ng/mL, the reference level of CYFRA is at least 1.98 ng/mL, the reference level of CRP is at least 1.8 mg/mL, and the reference level of ferritin is less than 109 ng/mL; and
(b) in a female subject at least 63 years old, the reference level of CEA is at least 4.8 ng/mL, the reference level of CYFRA is at least 1.72 ng/mL, the reference level of CRP is at least 5.5 mg/mL, and the reference level of ferritin is less than 38 ng/mL.

5. The method of claim 2, wherein the reference age for a male subject is 54, and the reference age for a female subject is 63.

6. The method of any one of claims 1-5, wherein the reference score is 0, 1, 2, 3, 4, or 5.

7. The method of claim 6, wherein the reference score is 2 or 3 for a male subject, and the reference score is 2 for a female subject.

8. The method of claim 6 or 7, wherein a total score greater than 2 for a male or female subject indicates that the subject is suffering from, or at risk of suffering from, high risk adenomas or CRC.

9. The method of any one of the preceding claims, further comprising a CRC structural screening regimen, or a CRC monitoring regimen to the subject diagnosed as suffering from, or at risk of suffering from, high risk adenomas or CRC; wherein:
the CRC structural screening regimen is a colonoscopy or sigmoidoscopy; and
the CRC monitoring regimen comprises determining levels of CEA, CYFRA, CRP, and ferritin at periodic intervals.

10. The method of any one of claims 1-9, wherein determining the levels of CEA, CYFRA, CRP, and ferritin comprises an immunological method with at least one antibody capable of specifically binding to CEA, CYFRA, CRP, ferritin, or combinations thereof.

11. The method of claim 10, wherein the immunological method comprises:
(a) measuring the levels of CEA by:
(i) contacting the test sample with at least one capture antibody, wherein the capture antibody binds to an epitope on CEA or a fragment of CEA to form a capture antibody-CEA antigen complex;
(ii) contacting the capture antibody-CEA antigen complex with at least one detection antibody comprising a detectable label, wherein the detection antibody binds to an epitope on CEA that is not bound by the capture antibody and forms a capture antibody-CEA antigen-detection antibody complex; and
(iii) determining the CEA levels in the test sample based on the signal generated by the detectable label in the capture antibody-CEA antigen-detection antibody complex formed in (a)(ii);
(b) measuring the levels of CYFRA by:
(i) contacting the test sample with at least one capture antibody, wherein the capture antibody binds to an epitope on CYFRA or a fragment of CYFRA to form a capture antibody-CYFRA antigen complex;
(ii) contacting the capture antibody-CYFRA antigen complex with at least one detection antibody comprising a detectable label, wherein the detection antibody binds to an epitope on CYFRA that is not bound by the capture antibody and forms a capture antibody-CYFRA antigen-detection antibody complex; and
(iii) determining the CYFRA levels in the test sample based on the signal generated by the detectable label in the capture antibody-CYFRA antigen-detection antibody complex formed in (b)(ii);
(c) measuring the levels of CRP by:
(i) contacting the test sample with at least one capture antibody, wherein the capture antibody binds to an epitope on CRP or a fragment of CRP to form a capture antibody-CRP antigen complex;
(ii) contacting the capture antibody-CRP antigen complex with at least one detection antibody comprising a detectable label, wherein the detection antibody binds to an epitope on CRP that is not bound by the capture antibody and forms a capture antibody-CRP antigen-detection antibody complex; and
(iii) determining the CRP levels in the test sample based on the signal generated by the detectable label in the capture antibody-CRP antigen-detection antibody complex formed in (c)(ii); and
(d) measuring the levels of ferritin by:
(i) contacting the test sample with at least one capture antibody, wherein the capture antibody binds to an epitope on ferritin or a fragment of ferritin to form a capture antibody-ferritin antigen complex;
(ii) contacting the capture antibody-ferritin antigen complex with at least one detection antibody comprising a detectable label, wherein the detection antibody binds to an epitope on ferritin that is not bound by the capture antibody and forms a capture antibody-ferritin antigen-detection antibody complex; and
(iii) determining the ferritin levels in the test sample based on the signal generated by the detectable label in the capture antibody-ferritin antigen-detection antibody complex formed in (d)(ii).

12. The method of any one of claims 1-11, further comprising determining the level of at least one additional biomarker of CRC in the biological sample selected from: Methyl Septin 9 (mS9), Galectin-3 (Gal3), Glycated hemoglobin (HbA1c), complement component 3 (C3a), Cathepsin X (Cat X), soluble urokinase receptor (suPAR(I)), Plasminogen activator inhibitor-1 (PAI-1), Enolase 2 (ENO2), or combinations thereof, and comparing the level of the at least one additional biomarker of CRC to a reference level for the at least one additional biomarker of CRC.

13. The method of any one of the preceding claims, wherein the biological sample is a tissue sample, whole blood, plasma, serum, urine, bronchoalveolar lavage fluid, or a cell culture suspension or fraction thereof.

14. The method of claim 10, wherein the antibody is selected from a polyclonal antibody, a monoclonal antibody, a human antibody, a disulfide linked Fv, an affinity matured antibody, a scFv, a chimeric antibody, a single domain antibody, a CDR-grafted antibody, a diabody, a humanized antibody, a multispecific antibody, a Fab, a dual-variable-domain (DVD) immunoglobulin, a Fab', a bispecific antibody, a F(ab')2, and a Fv.

15. Use of a kit for performing the method of any one of claims 1-14, the kit comprising:
(a) a reagent capable of specifically binding to CEA, a reagent capable of specifically binding to CYFRA, a reagent capable of specifically binding to CRP, and a reagent capable of specifically binding to ferritin, to quantify the levels of CEA, CYFRA, CRP, and ferritin in the biological sample of a subject;
(b) a reference standard indicating reference levels of CEA, CYFRA, CRP, and ferritin; and optionally
(c) at least one additional reagent capable of specifically binding at least one additional biomarker selected from Methyl Septin 9 (mS9), Galectin-3 (Gal3), Glycated hemoglobin (HbA1c), complement component 3 (C3a), Cathepsin X (Cat X), soluble urokinase receptor (suPAR(I)), Plasminogen activator inhibitor-1 (PAI-1), Enolase 2 (ENO2), or combinations thereof, and
(d) a reference standard indicating a reference level of the at least one additional biomarker of Methyl Septin 9 (mS9), Galectin-3 (Gal3), Glycated hemoglobin (HbA1c), complement component 3 (C3a), Cathepsin X (Cat X), soluble urokinase receptor (suPAR(I)), Plasminogen activator inhibitor-1 (PAI-1), Enolase 2 (ENO2), or combinations thereof.

## Patentansprüche

1. Ein In-vitro-Verfahren zur Bestimmung, ob eine Testperson an Adenomen mit hohem Risiko oder Kolorektalkrebs (CRC) leidet oder gefährdet ist daran zu leiden, wobei das Verfahren folgende Schritte umfasst:
(a) Bereitstellen einer biologische Probe, die zuvor von einer Testperson erhalten wurde;
(b) Bestimmen der Spiegel von karzinoembryonalem Antigen (CEA), Cytokeratin 19 Fragment (CYFRA), C-reaktivem Protein (CRP) und Ferritin in der biologischen Probe von der Testperson;
(c) Vergleichen der Spiegel von CEA, CYFRA, CRP und Ferritin in der biologischen Probe mit Referenzspiegeln von CEA, CYFRA, CRP und Ferritin;
(i) Bereitstellen eines CEA-Werts für die Testperson, worin die Testperson einen Wert von 1 bekommt, wenn der Spiegel von CEA in der biologischen Probe größer ist als der Referenzspiegel von CEA, und einen Wert von 0, wenn der Spiegel von CEA in der biologischen Probe gleich oder geringer ist als der Referenzspiegel von CEA;
(ii) Bereitstellen eines CYFRA-Werts für die Testperson, worin die Testperson einen Wert von 1 bekommt, wenn der Spiegel von CYFRA in der biologischen Probe größer ist als der Referenzspiegel von CYFRA, und einen Wert von 0, wenn der Spiegel von CYFRA in der biologischen Probe gleich oder geringer ist als der Referenzspiegel von CYFRA;
(iii) Bereitstellen eines CRP-Werts für die Testperson, worin die Testperson einen Wert von 1 bekommt, wenn der Spiegel von CRP in der biologischen Probe größer ist als der Referenzspiegel von CRP, und einem Wert von 0, wenn der Spiegel von CRP in der biologischen Probe gleich oder geringer ist als der Referenzspiegel von CRP; und
(iv) Bereitstellen eines Ferritin-Werts für die Testperson, worin die Testperson einen Wert von 1 bekommt, wenn der Spiegel von Ferritin in der biologischen Probe geringer ist als der Referenzspiegel von Ferritin und einen Wert von 0, wenn der Spiegel von Ferritin in der biologischen Probe gleich oder größer ist als der Referenzspiegel von Ferritin;
(d) Addieren der Werte aus Schritt (c), um einen Gesamtwert zu erzeugen; und
(e) Bereitstellen einer Diagnose für eine Testperson, dass sie an Adenomen mit hohem Risiko oder CRC leidet oder gefährdet ist daran zu leiden, wenn der Gesamtwert größer ist als ein Referenzwert, oder eine Diagnose für eine Testperson, dass sie nicht an Adenomen mit hohem Risiko oder CRC leidet oder gefährdet ist daran zu leiden, wenn der Gesamtwert gleich oder geringer ist als der Referenzwert.

2. Das Verfahren gemäß Anspruch 1, weiter Folgendes umfassend: Bestimmen des Alters der Testperson in Schritt (b); Vergleichen des Alters der Testperson mit einem Referenzalter in Schritt (c); und
(v) Bereitstellen eines Alterswerts für die Testperson, worin die Testperson einen Wert von 1 bekommt, wenn das Alter der Testperson höher oder gleich dem Referenzalter ist, und einen Wert von 0, wenn das Alter der Testperson geringer ist als das Referenzalter.

3. Das Verfahren gemäß Anspruch 1 oder 2, worin die Testperson männlich oder weiblich ist.

4. Das Verfahren gemäß Anspruch 3, worin:
(a) in einer männlichen Testperson von mindestens 54 Jahren der Referenzspiegel von CEA mindestens 6,5 ng/ml ist, der Referenzspiegel von CYFRA mindestens 1,98 ng/ml ist, der Referenzspiegel von CRP mindestens 1,8 mg/ml ist, und der Referenzspiegel von Ferritin geringer als 109 ng/ml ist; und
(b) in einer weiblichen Testperson von mindestens 63 Jahren der Referenzspiegel von CEA mindestens 4,8 ng/ml ist, der Referenzspiegel von CYFRA mindestens 1,72 ng/ml ist, der Referenzspiegel von CRP mindestens von 5,5 mg/ml ist, und der Referenzspiegel von Ferritin geringer als 38 ng/ml ist.

5. Das Verfahren gemäß Anspruch 2, worin das Referenzalter für eine männliche Testperson 54 ist, und das Referenzalter für eine weibliche Testperson 63 ist.

6. Das Verfahren gemäß irgendeinem der Ansprüche 1-5, worin der Referenzwert 0, 1, 2, 3, 4 oder 5 ist.

7. Das Verfahren gemäß Anspruch 6, worin der Referenzwert für eine männliche Testperson 2 oder 3 ist, und der Referenzwert für eine weibliche Testperson 2 ist.

8. Das Verfahren gemäß Anspruch 6 oder 7, worin ein Gesamtwert von größer als 2 für eine männliche oder weibliche Testperson anzeigt, dass die Testperson an Adenomen mit hohem Risiko oder CRC leidet oder gefährdet ist daran zu leiden.

9. Das Verfahren gemäß irgendeinem der vorhergehenden Ansprüche, weiter umfassend ein CRC strukturelles Screening-Regime, oder ein CRC Überwachungs-Regime für die Testperson, bei der diagnostiziert wurde, dass sie an Adenomen mit hohem Risiko oder CRC leidet oder gefährdet ist daran zu leiden;
worin
das CRC strukturelle Screening-Regime eine Darmspiegelung oder Sigmoidoskopie ist; und
das CRC Überwachungs-Regime das Bestimmen der Spiegel von CEA, CYFRA, CRP und Ferretin in periodischen Intervallen umfasst.

10. Das Verfahren gemäß irgendeinem der Ansprüche 1-9, worin das Bestimmen der Spiegel von CEA, CYFRA, CRP, und Ferritin ein immunologisches Verfahren mit mindestens einem Antikörper, der in der Lage ist spezifisch an CEA, CYFRA, CRP, Ferritin oder Kombinationen daraus zu binden, umfasst.

11. Das Verfahren gemäß Anspruch 10, worin das immunologische Verfahren Folgendes umfasst:
(a) Messen der Spiegel von CEA durch:
(i) In-Kontakt-bringen der Testprobe mit mindestens einem Einfangantikörper, worin der Einfangantikörper an ein Epitop auf CEA oder ein Fragment von CEA bindet, um einen Einfangantikörper-CEA Antigen-Komplex zu bilden;
(ii) In-Kontakt-bringen des Einfangantikörper-CEA Antigen-Komplexes mit mindestens einem Detektionsantikörper umfassend einen detektierbaren Marker, worin der Detektionsantikörper an ein Epitop auf CEA bindet, das nicht durch den Einfangantikörper gebunden ist, und einen Einfangantikörper-CEA Antigen-Detektionsantikörper-Komplex bildet; und
(iii) Bestimmen der CEA-Splege1 in der Testprobe basierend auf dem Signal, das durch den detektierbaren Marker in dem in (a)(ii) gebildeten Einfangantikörper-CEA Antigen-Detektionsantikörper-Komplex erzeugt wurde;
(b) Messen der Spiegel von CYFRA durch:
(i) In-Kontakt-bringen der Testprobe mit mindestens einem Einfangantikörper, worin der Einfangantikörper an ein Epitop auf CYFRA oder ein Fragment von CYFRA bindet, um einen Einfangantikörper-CYFRA Antigen-Komplex zu bilden;
(ii) In-Kontakt-bringen des Einfangantikörper-CYFRA Antigen-Komplexes mit mindestens einem Detektionsantikörper umfassend einen detektierbaren Marker, worin der Detektionsantikörper an ein Epitop auf CYFRA bindet, das nicht durch den Einfangantikörper gebunden ist, und einen Einfangantikörper-CYFRA Antigen-Detektionsantikörper-Komplex bildet; und
(iii) Bestimmen der CYFRA-Spiegel in der Testprobe basierend auf dem Signal, das durch den detektierbaren Marker in dem in (b)(ii) gebildeten Einfangantikörper-CYFRA Antigen-Detektionsantikörper-Komplex erzeugt wurde;
(c) Messen der Spiegel von CRP durch:
(i) In-Kontakt-bringen der Testprobe mit mindestens einem Einfangantikörper, worin der Einfangantikörper an ein Epitop auf CRP oder ein Fragment von CRP bindet, um einen Einfangantikörper-CRP Antigen-Komplex zu bilden;
(ii) In-Kontakt-bringen des Einfangantikörper-CRP Antigen-Komplexes mit mindestens einem Detektionsantikörper umfassend einen detektierbaren Marker, worin der Detektionsantikörper an ein Epitop auf CRP bindet, das nicht durch den Einfangantikörper gebunden ist, und einen Einfangantikörper-CRP Antigen-Detektionsantikörper-Komplex bildet; und
(iii) Bestimmen der CRP-Spiegel in der Testprobe, basierend auf dem Signal, das durch den detektierbaren Marker in dem in (c)(ii) gebildeten Einfangantikörper-CRP Antigen-Detektionsantikörper-Komplex erzeugt wurde; und
(d) Messen der Spiegel von Ferritin durch:
(i) In-Kontakt-bringen der Testprobe mit mindestens einem Einfangantikörper, worin der Einfangantikörper an ein Epitop auf Ferritin oder ein Fragment von Ferritin bindet, um einen Einfangantikörper-Ferritin Antigen-Komplex zu bilden;
(ii) In-Kontakt-bringen des Einfangantikörper-Ferritin Antigen-Komplexes mit mindestens einem Detektionsantikörper umfassend einen detektierbaren Marker, worin der Detektionsantikörper an ein Epitop von Ferritin bindet, das nicht durch den Einfangantikörper gebunden ist, und einen Einfangantikörper-Ferritin Antigen-Detektionsantikörper-Komplex bildet; und
(iii) Bestimmen der Ferritinspiegel in der Testprobe basierend auf dem Signal, das durch den detektierbaren Marker in dem in (d)(ii) gebildeten Einfangantikörper-Ferritin Antigen-Detektionsantikörper-Komplex erzeugt wurde.

12. Das Verfahren gemäß irgendeinem der Ansprüche 1-11, weiter umfassend das Bestimmen der Spiegel von mindestens einem zusätzlichen Biomarker von CRC in der biologischen Probe gewählt aus: Methyl Septin 9 (mS9), Galectin-3 (Gal3), glykiertem Hämoglobin (HbA1c), Komplementkomponente 3 (C3a), Cathepsin X (Cat X), löslichem Urokinaserezeptor (suPAR(I)), Plasminogenaktivator Inhibitor-1 (PAI-1), Enolase 2 (ENO2), oder Kombinationen daraus, und Vergleichen des Spiegels des mindestens einen zusätzlichen Biomarkers von CRC mit einem Referenzspiegel für den mindestens einen zusätzlichen Biomarker von CRC.

13. Das Verfahren gemäß irgendeinem der vorhergehenden Ansprüche, worin die biologische Probe eine Gewebeprobe, Vollblut, Plasma, Serum, Urin, bronchoalveoläre Lavage-Flüssigkeit oder eine Zellkultursuspension oder Fraktion davon ist.

14. Das Verfahren gemäß Anspruch 10, worin der Antikörper gewählt ist aus einem polyklonalen Antikörper, einem monoklonalen Antikörper, einem menschlichen Antikörper, einem Disulfid verknüpften Fv, einem affinitätsgereiften Antikörper, einem scFv, einem chimären Antikörper, einem Einzeldomain-Antikörper, einem durch CDR-grafting erzeugten Antikörper, einem Diabody, einem humanisierten Antikörper, einem multispezifischen Antikörper, einem Fab, einem dual-variable-Domain (DVD) Immunglobulin, einem Fab', einem bispezifischen Antikörper, einem F(ab')2, und einem Fv.

15. Verwendung eines Kits zur Durchführung des Verfahrens gemäß irgendeinem der Ansprüche 1-14, wobei der Kit Folgendes umfasst:
(a) ein Reagens, das in der Lage ist spezifisch an CEA zu binden, ein Reagens, das in der Lage ist spezifisch an CYFRA zu binden, ein Reagens, das in der Lage ist spezifisch an CRP zu binden, und ein Reagens, das in der Lage ist spezifisch an Ferritin zu binden, um die Spiegel von CEA, CYFRA, CRP, und Ferritin in der biologischen Probe einer Testperson zu quantifizieren;
(b) einen Referenzstandard, der Referenzspiegel von CEA, CYFRA, CRP und Ferritin anzeigt; und wahlweise
(c) mindestens ein zusätzliches Reagens, das in der Lage ist spezifisch mindestens einen zusätzlichen Biomarker gewählt aus Methyl Septin 9 (mS9), Galectin-3 (Gal3), glykiertem Hämoglobin (HbA1c), Komplementkomponente 3 (C3a), Cathepsin X (Cat X), löslichem Urokinaserezeptor (suPAR(I)), Plasminogenaktivator Inhibitor-1 (PAI-1), Enolase 2 (ENO2), oder Kombinationen daraus zu binden, und
(d) einen Referenzstandard, der einen Referenzspiegel des mindestens einen zusätzlichen Biomarkers von Methyl Septin 9 (mS9), Galectin-3 (Gal3), glykiertem Hämoglobin (HbA1c), Komplementkomponente 3 (C3a), Cathepsin X (Cat X), löslichem Urokinaserezeptor (suPAR(I)), Plasminogenaktivator Inhibitor-1 (PAI-1), Enolase 2 (ENO2), oder Kombinationen daraus anzeigt.

## Revendications

1. Procédé in vitro pour déterminer si un sujet souffre, ou risque de souffrir, d'un cancer colorectal (CRC) ou d'adénomes à haut risque, le procédé comprenant les étapes suivantes :
(a) disposition d'un échantillon biologique préalablement obtenu auprès d'un sujet ;
(b) détermination des niveaux d'antigène carcino-embryonnaire (CEA), de fragment de cytokératine 19 (CYFRA), de protéine C réactive (CRP), et de ferritine dans l'échantillon biologique provenant du sujet ;
(c) comparaison des niveaux de CEA, CYFRA, CRP et ferritine dans l'échantillon biologique avec des niveaux de référence de CEA, CYFRA, CRP et ferritine ;
(i) obtention d'une note de CEA pour le sujet, lequel sujet obtient une note de 1 si le niveau de CEA dans l'échantillon biologique est supérieur au niveau de référence de CEA et une note de 0 si le niveau de CEA dans l'échantillon biologique est égal ou inférieur au niveau de référence de CEA ;
(ii) obtention d'une note de CYFRA pour le sujet, lequel sujet obtient une note de 1 si le niveau de CYFRA dans l'échantillon biologique est supérieur au niveau de référence de CYFRA et une note de 0 si le niveau de CYFRA dans l'échantillon biologique est égal ou inférieur au niveau de référence de CYFRA ;
(iii) obtention d'une note de CRP pour le sujet, lequel sujet obtient une note de 1 si le niveau de CRP dans l'échantillon biologique est supérieur au niveau de référence de CRP et une note de 0 si le niveau de CRP dans l'échantillon biologique est égal ou inférieur au niveau de référence de CRP ; et
(iv) obtention d'une note de ferritine pour le sujet, lequel sujet obtient une note de 1 si le niveau de ferritine dans l'échantillon biologique est inférieur au niveau de référence de ferritine et une note de 0 si le niveau de ferritine dans l'échantillon biologique est égal ou supérieur au niveau de référence de ferritine ;
(d) addition des notes de l'étape (c) pour générer une note totale ; et
(e) fourniture d'un diagnostic d'un sujet comme souffrant, ou risquant de souffrir, de CRC ou d'adénomes à haut risque si la note totale est supérieure à une note de référence, ou d'un diagnostic d'un sujet comme ne souffrant pas, ou ne risquant pas de souffrir, de CRC ou d'adénomes à haut risque si la note totale est égale ou inférieure à la note de référence.

2. Procédé selon la revendication 1, comprenant en outre : la détermination de l'âge du sujet dans l'étape (b) ; la comparaison de l'âge du sujet à un âge de référence dans l'étape (c) ; et
(v) fourniture d'une note d'âge pour le sujet, lequel sujet obtient une note de 1 si l'âge du sujet est supérieur ou égal à l'âge de référence et une note de 0 si l'âge du sujet est inférieur ou égal à l'âge de référence.

3. Procédé selon la revendication 1 ou 2, dans lequel le sujet est mâle ou femelle.

4. Procédé selon la revendication 3, dans lequel :
(a) chez un sujet masculin âgé d'au moins 54 ans, le niveau de référence de CEA est d'au moins 6,5 ng/mL, le niveau de référence de CYFRA est d'au moins 1,98 ng/mL, le niveau de référence de CRP est d'au moins 1,8 mg/mL, et le niveau de référence de ferritine est inférieur à 109 ng/mL ; et
(b) chez un sujet féminin âgé d'au moins 63 ans, le niveau de référence de CEA est d'au moins 4,8 ng/mL, le niveau de référence de CYFRA est d'au moins 1,72 ng/mL, le niveau de référence de CRP est d'au moins 5,5 mg/mL, et le niveau de référence de ferritine est inférieur à 38 ng/mL.

5. Procédé selon la revendication 2, dans lequel l'âge de référence pour un sujet masculin est 54, et l'âge de référence pour un sujet féminin est 63.

6. Procédé selon l'une quelconque des revendications 1 à 5, dans lequel la note de référence est 0, 1, 2, 3, 4 ou 5.

7. Procédé selon la revendication 6, dans lequel la note de référence est 2 ou 3 pour un sujet masculin, et la note de référence est 2 pour un sujet féminin.

8. Procédé selon la revendication 6 ou 7, dans lequel une note totale supérieure à 2 pour un sujet masculin ou féminin indique que le sujet souffre, ou risque de souffrir, d'un CRC ou d'adénomes à haut risque.

9. Procédé selon l'une quelconque des revendications précédentes, comprenant en outre un régime de dépistage structurel de CRC, ou un régime de surveillance de CRC au sujet diagnostiqué comme souffrant, ou risquant de souffrir, d'un CRC ou d'adénomes à haut risque ; dans lequel :
le régime de dépistage structurel de CRC est une colonoscopie ou une sigmoïdoscopie ; et
le régime de surveillance de CRC comprend la détermination des niveaux de CEA, CYFRA, CRP et ferritine à intervalles périodiques.

10. Procédé selon l'une quelconque des revendications 1 à 9, dans lequel la détermination des niveaux de CEA, CYFRA, CRP et ferritine comprennent un procédé immunologique avec au moins un anticorps capable de se lier spécifiquement au CEA, CYFRA, CRP, ferritine, ou leurs combinaisons.

11. Procédé selon la revendication 10, dans lequel le procédé immunologique comprend :
(a) la mesure des niveaux de CEA par :
(i) mise en contact de l'échantillon de test avec au moins un anticorps de capture, lequel anticorps de capture se lie à un épitope sur le CEA ou un fragment de CEA pour former un complexe d'anticorps de capture-antigène de CEA ;
(ii) mise en contact du complexe d'anticorps de capture-antigène de CEA avec au moins un anticorps de détection comprenant un marqueur détectable, lequel anticorps de détection se lie à un épitope sur le CEA qui n'est pas lié par l'anticorps de capture et forme un complexe d'anticorps de capture-antigène de CEA-anticorps de détection ; et
(iii) détermination des niveaux de CEA dans l'échantillon de test sur la base du signal généré par le marqueur détectable dans le complexe d'anticorps de capture-antigène de CEA-anticorps de détection formé en (a)(ii) ;
(b) la mesure des niveaux de CYFRA par :
(i) mise en contact de l'échantillon de test avec au moins un anticorps de capture, lequel anticorps de capture se lie à un épitope sur le CYFRA ou un fragment de CYFRA pour former un complexe d'anticorps de capture-antigène de CYFRA ;
(ii) mise en contact du complexe d'anticorps de capture-antigène de CYFRA avec au moins un anticorps de détection comprenant un marqueur détectable, lequel anticorps de détection se lie à un épitope sur le CYFRA qui n'est pas lié par l'anticorps de capture et forme un complexe d'anticorps de capture-antigène de CYFRA -anticorps de détection ; et
(iii) détermination des niveaux de CYFRA dans l'échantillon de test sur la base du signal généré par le marqueur détectable dans le complexe d'anticorps de capture-antigène de CYFRA-anticorps de détection formé en (b)(ii) ;
(c) la mesure des niveaux de CRP par :
(i) mise en contact de l'échantillon de test avec au moins un anticorps de capture, lequel anticorps de capture se lie à un épitope sur la CRP ou un fragment de CRP pour former un complexe d'anticorps de capture-antigène de CRP ;
(ii) mise en contact du complexe d'anticorps de capture-antigène de CRP avec au moins un anticorps de détection comprenant un marqueur détectable, lequel anticorps de détection se lie à un épitope sur la CRP qui n'est pas lié par l'anticorps de capture et forme un complexe d'anticorps de capture-antigène de CRP-anticorps de détection ; et
(iii) détermination des niveaux de CRP dans l'échantillon de test sur la base du signal généré par le marqueur détectable dans le complexe d'anticorps de capture-antigène de CRP-anticorps de détection formé en (c)(ii) ; et
(d) la mesure des niveaux de ferritine par :
(i) mise en contact de l'échantillon de test avec au moins un anticorps de capture, lequel anticorps de capture se lie à un épitope sur la ferritine ou un fragment de ferritine pour former un complexe d'anticorps de capture-antigène de ferritine ;
(ii) mise en contact du complexe d'anticorps de capture-antigène de ferritine avec au moins un anticorps de détection comprenant un marqueur détectable, lequel anticorps de détection se lie à un épitope sur la ferritine qui n'est pas lié par l'anticorps de capture et forme un complexe d'anticorps de capture-antigène de ferritine-anticorps de détection ; et
(iii) détermination des niveaux de ferritine dans l'échantillon de test sur la base du signal généré par le marqueur détectable dans le complexe d'anticorps de capture-antigène de ferritine-anticorps de détection formé en (d)(ii).

12. Procédé selon l'une quelconque des revendications 1 à 11, comprenant en outre la détermination du niveau d'au moins un marqueur biologique additionnel de CRC dans l'échantillon biologique, choisi parmi : la méthyl-septine 9 (mS9), la galectine-3 (Gal3), l'hémoglobine glyquée (HbA1c), le composant de complément 3 (C3a), la cathepsine X (Cat X), le récepteur d'urokinase soluble (suPAR(I)), l'inhibiteur 1 d'activateur du plasminogène (PAI-1), l'énolase 2 (ENO2), ou leurs combinaisons, et la comparaison du niveau de l'au moins un marqueur biologique additionnel de CRC avec un niveau de référence pour l'au moins un marqueur biologique additionnel de CRC.

13. Procédé selon l'une quelconque des revendications précédentes, dans lequel l'échantillon biologique est un échantillon de tissu, du sang entier, du plasma, du sérum, de l'urine, du fluide de lavage broncho-alvéolaire, ou une suspension de culture cellulaire ou une fraction de celle-ci.

14. Procédé selon la revendication 10, dans lequel l'anticorps est choisi parmi un anticorps polyclonal, un anticorps monoclonal, un anticorps humain, un Fv à liaison disulfure, un anticorps maturé par affinité, un scFv, un anticorps chimérique, un anticorps à un seul domaine, un anticorps greffé de CDR, un diacorps, un anticorps humanisé, un anticorps multispécifique, un Fab, une immunoglobuline à domaine variable double (DVD), un Fab', un anticorps bispécifique, un F(ab')2, et un Fv.

15. Utilisation d'une trousse pour effectuer le procédé de l'une quelconque des revendications 1 à 14, la trousse comprenant :
(a) un réactif capable de se lier spécifiquement au CEA, un réactif capable de se lier spécifiquement au CYFRA, un réactif capable de se lier spécifiquement à la CRP, et un réactif capable de se lier spécifiquement à la ferritine, pour quantifier les niveaux de CEA, CYFRA, CRP et ferritine dans l'échantillon biologique d'un sujet ;
(b) un étalon de référence indiquant des niveaux de référence de CEA, CYFRA, CRP et ferritine ; et éventuellement
(c) au moins un réactif additionnel capable de se lier spécifiquement à au moins un marqueur biologique additionnel choisi parmi la méthyl-septine 9 (mS9), la galectine-3 (Gal3), l'hémoglobine glyquée (HbA1c), le composant de complément 3 (C3a), la cathepsine X (Cat X), le récepteur d'urokinase soluble (suPAR(I)), l'inhibiteur 1 d'activateur du plasminogène (PAI-1), l'énolase 2 (ENO2), ou leurs combinaisons, et
(d) un étalon de référence indiquant un niveau de référence de l'au moins un marqueur biologique additionnel parmi la méthyl-septine 9 (mS9), la galectine-3 (Gal3), l'hémoglobine glyquée (HbA1c), le composant de complément 3 (C3a), la cathepsine X (Cat X), le récepteur d'urokinase soluble (suPAR(I)), l'inhibiteur 1 d'activateur du plasminogène (PAI-1), l'énolase 2 (ENO2), ou leurs combinaisons.
